# EUROPEAN PATENT APPLICATION

(11) **EP 1 547 996 A1**
(43) Date of publication of application: **29.06.2005**
(21) Application number: 03808871.2
(22) Date of filing: 29.08.2003
(51) Int. Cl.: C07C 211/48, A61K 49/00, A61K 31/4184, A61K 51/00, A61P 31/00, A61P 43/00, C07D 235/14, A61K 31/381, C07D 413/04, C07D 215/12, A61K 31/47, A61K 31/4709, C07C 215/74, A61K 31/428, C07D 417/04, C07D 213/38, C07D 405/04, C07D 277/64, A61K 31/4245, C07D 277/66, A61K 31/433

(54) **DIAGNOSTIC PROBES AND REMEDIES FOR DISEASES WITH ACCUMULATION OF PRION PROTEIN, AND STAINS FOR PRION PROTEIN**

(30) Priority: 30.08.2002 JP 2002255013; 30.08.2002 JP 2002255014; 30.08.2002 JP 2002255015; 18.03.2003 JP 2003073344
(71) Applicant: BF Research Institute, Inc., Osaka-shi, Osaka 532-8686 (JP); TOHOKU UNIVERSITY, Sendai City Miyagi Prefecture (JP)
(72) Inventor: KUDO, Yukitsuka, Suita-shi, Osaka 565-0842 (JP); SAWADA, Tohru, Ibaraki-shi, Osaka 567-0064 (JP); DOH-URA, Katsumi, Sendai-shi, Miyagi 982-0826 (JP)
(74) Representative: Schnappauf, Georg Dr.
(86) International application number: PCT/JP2003/011056
(87) International publication number: WO 2004/035522

(57) **Abstract**

A compound, which is used for the diagnosis and the prophylaxis/treatment of diseases in which prion protein is accumulated, or specific staining of abnormal prion protein in samples, represented by the formula (I) or (II): 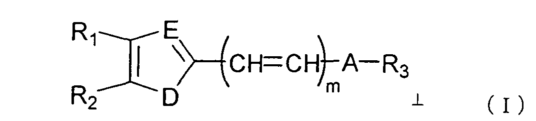 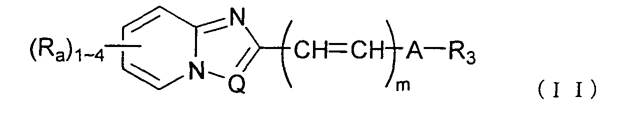 or a salt or solvate thereof.

## Description

### TECHNICAL FIELD

The present invention relates to probes for the imaging diagnosis of diseases in which prion protein is accumulated, in particular, probes labeled with positron-emitting radionuclides, as well as to diagnostic compositions comprising such probes. Also the present invention relates to compositions for the prophylaxis/treatment of diseases in which prion protein is accumulated and to agents for specifically staining abnormal prion protein in samples. Furthermore, the present invention relates to methods for the diagnosis and prophylaxis/treatment of diseases in which prion protein is accumulated in the brain, and methods for the detection of abnormal prion protein in samples.

### BACKGROUND ART

There are known diseases in which the so-called prion protein is accumulated in the brain, including in humans, Creutzfeldt-Jacob disease (CJD), Gerstmann-Sträussler-Scheinker disease (GSS), variant Creutzfeldt-Jacob disease (VCJD), fatal familial insomnia (FFI), kuru, and in non-human animals, sheep scrapie, bovine spongiform encephalopathy (BSE), transmissible mink encephalopathy, feline spongiform encephalopathy, and the like, each of which is a fatal, infectious incurable neurological disease.

In the case of these diseases, administration of ground tips of the brain of an affected individual to a healthy individual can result in the transmission of the illness, and additionally the affected brain leads to the occurrence of spongiform lesions. Thus, these diseases are referred to as transmissible spongiform encephalopathy (TSE).

The concept of prion was first proposed by Stanley Prusiner in 1982 as the causative agent of transmissible spongiform encephalopathy, proteinaceous infectious particles, that is to say, prion. Prion is a generic term for etiologies whose body indispensable for the transmission of the illness is suggested to be composed of only a protein and which themselves do not contain nucleic acids necessary for self-replication. In recent years, an abnormal prion protein has been found as the etiology responsible for transmissible spongiform encephalopathy and in addition, prion protein is accumulated in transmissible spongiform encephalopathy, which has been commonly referred to as prion disease.

Human prion protein is a basic protein of 253 amino acids and encoded in the short arm of chromosome 20. It is known that for normal prion protein, only 3 % or less of β-sheet structures are contained therein, whereas abnormal prion proteins have more than 40 % of β-sheet structures. This means that the etiology responsible for transmissible spongiform encephalopathy or prion diseases, i.e., the main body of abnormal prion proteins, is a protein that has acquired infectivity by possessing changes in conformational structures of the protein, i.e., an abundance of β-sheet structures. It is also known that abnormal prion proteins themselves act as templates to convert normal prion protein to proteins of the same types as themselves.

Normal prion protein can be broken down readily with proteases and dissolved with detergents, whereas abnormal prion proteins are resistant to proteases and insoluble in detergents and do not lose their infectivity by means of usual sterilization.

Creutzfeldt-Jacob disease (CJD) is a prion disease which is typical in humans, and includes sporadic type whose cause is unknown, familial type based on genetic mutations, and iatrogenic type resulting from medical practice (dural graft, corneal graft, and the like).

Gerstmann-Sträussler-Scheinker disease (GSS) is a hereditary disease based on mutations of the prion protein gene.

Variant Creutzfeldt-Jacob disease (v-CJD) is presumed to be due to the ingestion of nerve tissues of bovines affected with bovine spongiform encephalopathy (BSE).

For Kuru, it is known that it was transmitted by cannibalistic rites among the Fore tribe in Papua New Guinea.

With regard to the number of patients with prion diseases, 110-120 new patients are caused annually in Japan. Of these patients, about 90 % represent sporadic Creutzfeldt-Jacob disease, about 5 % are hereditary, and the remaining 5 % are of acquired types, including iatrogenic and mutated Creutzfeldt-Jacob diseases.

Some of the prion diseases have recently come to fore as serious social problems. A first problem is of iatrogenic Creutzfeldt-Jacob disease of patients who had received the graft of human dried dura mater in Japan. In Japan, more than ten thousand grafts of human dried dura mater have been used every year since 1973, for dural filling during the brain surgery. Unfortunately, many patients have been emerged who are likely to be due to the use of dura mater contaminated with prion, and it is estimated to reach as many as 200, 000 of patients who are suspected to have received dura mater of potential risk and have the history of grafting in which the use of such dura mater cannot be denied completely.

A second problem is of variant Creutzfeldt-Jacob disease. As described above, variant Creutzfeldt-Jacob disease is presumed to be due to the ingestion of nerve tissues of bovines affected with bovine spongiform encephalopathy (BSE). It is reported that in Europe, particularly, the United Kingdom, there are more than 180,000 occurrences of bovine spongiform encephalopathy (BSE) (Office International des Epizooties, data dated on May 8, 2003). The mortality of patients with definite or probable variant Creutzfeldt-Jacob disease reaches 132 persons and will increase up to 136, when living patients are included (UK Department of Health, data dated on July 11, 2003). Thus, according to a conjecture about the future, one is afraid that thousands to tens of thousands of patients will be caused.

At the present, the diagnosis of prion diseases has commonly utilized methods of using and evaluating the following indicators:
1) indicating of progressive dementia, 2) indicating of periodic synchronous discharges (PSD) by electroencephalography, 3) progressing of brain atrophy by CT or MRI, 4) increasing of 14-3-3 protein in the cerebrospinal fluid, and the like. However, these diagnostic methods are insufficient to confirm these diseases. For definite diagnosis, the most reliable method is to detect abnormal prion proteins in the central nervous system.

As results of many studies, it have been revealed that neuronal degeneration characteristic to prion diseases has already taken place much earlier than when their initial clinical symptoms appear. It is also known in the diseases that the pathology in the central nervous system has already progressed to an irretrievable state, when families or clinicians relating to the patients become aware of their initial clinical symptoms.

Pathologies of prion diseases are representative by two main signs, i.e., the accumulation of prion protein in the central nervous system and spongiform degeneration. Abnormal prion proteins are characteristic to prion diseases and the detection of such proteins in the brain as a maker can be an important method for the diagnosis of the diseases.

For the purpose of diagnosing prion diseases, attempts have been made to search low-molecular weight organic compounds that specifically bind to intracerebral abnormal prion proteins. Until now, however, low-molecular weight organic compounds have not been found yet which specifically bind to abnormal prion proteins, cross easily the blood-brain barrier, and do not pose problems regarding toxicity and others.

For the treatment of prion diseases, at present, no methods for specific treatment are provided, and symptomatic treatments are mainly employed. Recently, quinacrine, chloroquine, chlorprozine, and others attract attention as drugs for treating prion diseases (Doh-ura et. al., Journal of Virology, vol. 74, 4894-4897, 2000, and Korth et. al., Proceedings of the National Academy of Sciences, USA, vol. 98, 9836-9841, 2001). Quinacrine, which is considered to be most promising among such compounds, also does not always give clinical effects as expected.

In light of the circumstances mentioned above, an object of the present invention is to provide compounds that have high specificity for abnormal prion proteins and enhanced blood-brain barrier permeability, and can be used as probes for the diagnosis of diseases in which prion protein is accumulated, as well as diagnostic compositions and kits comprising such compounds. The present invention also provide those compounds which have been labeled and can be used as probes for the imaging diagnosis of diseases in which prion protein is accumulated, as well as compositions and kits for imaging diagnosis, comprising such probes. Such compounds, compositions, and kits are for clear staining of abnormal prion protein. Another object of the present invention is to provide compositions comprising such compounds, for the prophylaxis and/or treatment of diseases in which prion protein is accumulated in the brain, such as, in humans, Creutzfeldt-Jacob disease (CJD), Gerstmann-Sträussler-Scheinker disease (GSS), variant Creutzfeldt-Jacob disease (vCJD), fatal familial insomnia (FFI), kuru, and in non-human animals, sheep scrapie, bovine spongiform encephalopathy (BSE), transmissible mink encephalopathy, feline spongiform encephalopathy and the like. Still another object of the present invention is to provide methods for the diagnosis (including imaging diagnosis) of and treatment and/or prophylaxis of diseases in which prion protein is accumulated, as well as for staining abnormal prion protein, the methods utilizing the above-described compounds.

### DISCLOSURE OF THE INVENTION

The present inventors have intensively studied to achieve the above objects and found that compounds represented by the formula (I) or (II), or salts or solvates thereof, have remarkably high specificity of binding to abnormal prion proteins and furthermore enhanced blood-brain barrier permeability, leading to the completion of the present invention. Therefore, it can be said that the compounds of the present invention are compounds capable of correct and early diagnosis/discovery of diseases in which prion protein is accumulated. In addition, the compounds of the present invention, which have enhanced blood-brain barrier permeability, allow noninvasive diagnosis while in life. Further, the compounds of the present invention have been found to suppress the production of abnormal prion proteins by cells producing prion protein, and shown to be useful for the prophylaxis and/or treatment of diseases in which prion protein is accumulated. Accordingly, the present invention provides probes for diagnosing diseases in which prion protein is accumulated, compositions and kits therefor comprising such probes, and compositions for the prophylaxis and/or treatment of diseases in which prion protein is accumulated, as well as methods for the diagnosis (including imaging diagnosis) of, and treatment and/or prophylaxis of diseases in which prion protein is accumulated, and methods for staining abnormal prion protein.

Thus, the present invention provides the followings:
(1) a compound, which is used as a probe for diagnosing diseases in which prion protein is accumulated, represented by the formula (I) or (II): 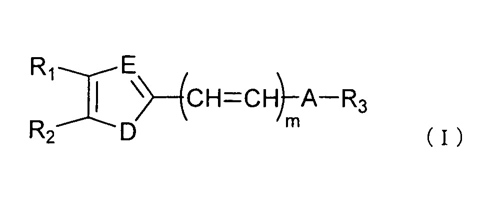 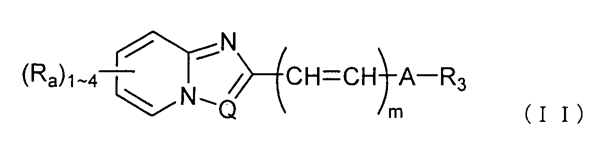 wherein D is NR', S, O, CH=CH, or CH₂,
   R' is H, alkyl having 1 to 4 carbons (hereinafter, referred to as C₁₋₄ alkyl), or phenyl, wherein the C₁₋₄ alkyl is optionally substituted with halogen(s),
   E is N or CH,
   Rₐ is, each independently, selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O- C₁₋₄ alkyl, COOH, and SO₃H, wherein the C₁₋₄ alkyl is optionally substituted with halogen(s),
   Q is N or CR_{b},
   R_{b} is selected from the group consisting of H, C₁₋₄ alkyl, halogen, OH, C₁₋₄ alkyl-OH, C₁₋₄ alkyl-O-C₁₋₄ alkyl, NH(C₁₋₄ alkyl), NH₂, N(C₁₋₄ alkyl)₂, NO₂, O-C₁₋₄ alkyl, COOH, and SO₃H, wherein the C₁₋₄ alkyl is optionally substituted with halogen(s),
   m is an integer of 0 to 4,
   R₁ and R₂ are independently selected from the group consisting of H, C₁₋₄ alkyl, halogen, OH, C₁₋₄ alkyl-OH, C₁₋₄ alkyl-O-C₁₋₄ alkyl, NH(C₁₋₄ alkyl), NH₂, N(C₁₋₄ alkyl)₂, NO₂, O-C₁₋₄ alkyl, COOH, and SO₃H, wherein the C₁₋₄ alkyl is optionally substituted with halogen (s),
   or alternatively, R₁ and R₂, together, form a benzene or naphthalene ring which is optionally substituted with one to four R₄,
   R₃ is selected from the group consisting of H, C₁₋₄ alkyl, halogen, OH, C₁₋₄ alkyl-OH, C₁₋₄ alkyl-O-C₁₋₄ alkyl, NH₂, NH (C₁₋₄ alkyl) , N(C₁₋₄ alkyl)₂, NO₂, O-C₁₋₄ alkyl, COOH, SO₃H, wherein the C₁₋₄ alkyl is optionally substituted with halogen(s), and any one of the moieties represented by (a) to (e): 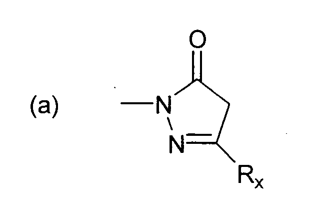 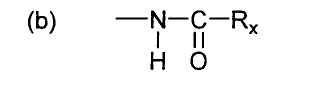 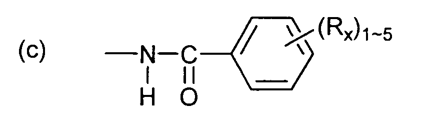 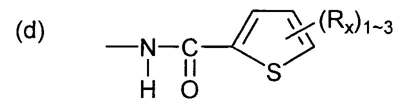 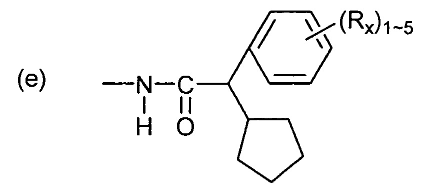 wherein each Rₓ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, N=CH-allyl, NO₂, O-C₁₋₄ alkyl, COOH, and SO₃H, wherein the C₁₋₄ alkyl is optionally substituted
   with halogen(s),
   each R₄ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, SO₃H,
   wherein the C₁₋₄ alkyl is optionally substituted with halogen (s), and any one of the moieties represented by (f) to (l): 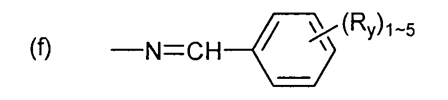 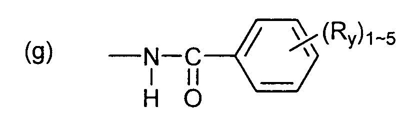 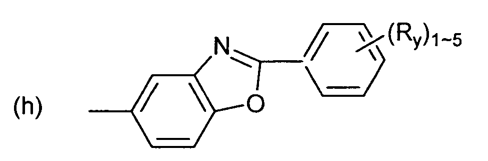 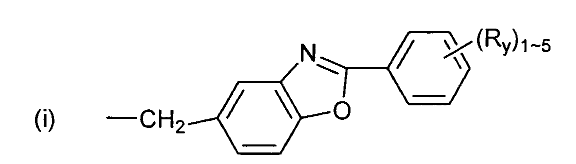 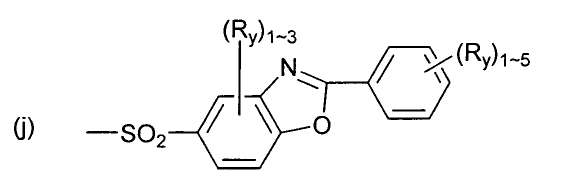 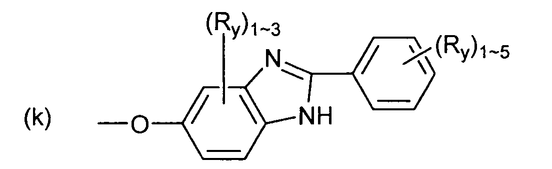 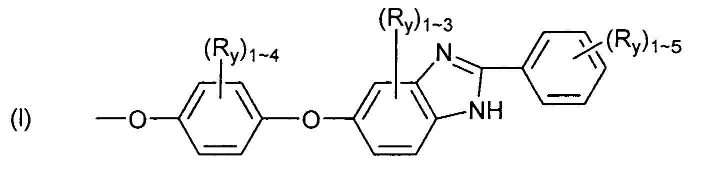 wherein two R₄s attached on adjacent carbons may form a methylenedioxy group, and wherein the C₁₋₄ alkyl is optionally substituted with halogen(s),
   each R_{y} is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, and SO₃H,
   wherein the C₁₋₄ alkyl is optionally substituted with halogen (s),
   A is any one of the rings represented by (i) to (ix): 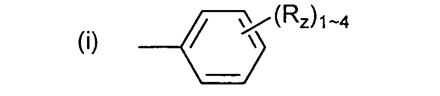 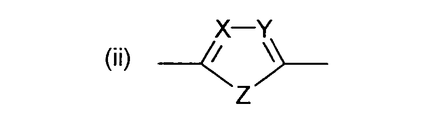 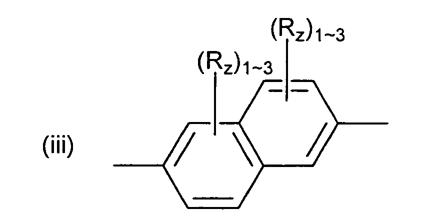 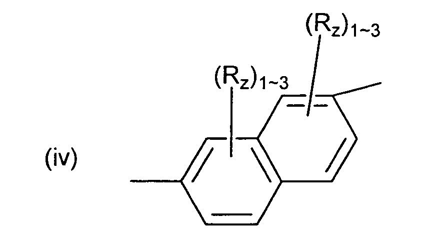 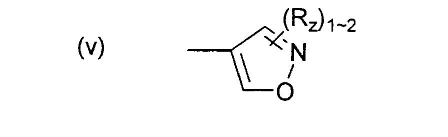 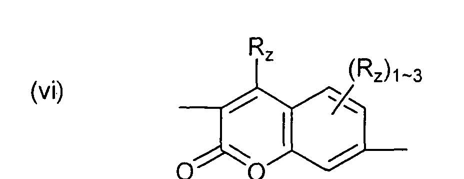 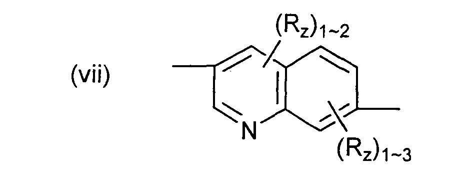 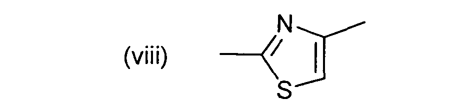 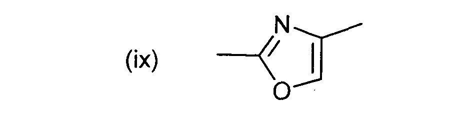 wherein each R_{z} is independently selected from the group consisting of H, C₁₋₄ alkyl, halogen, OH, C₁₋₄ alkyl-OH, C₁₋₄ alkyl -O-C₁₋₄ alkyl, NH₂, NH(C₁₋₄ alkyl), N(C₁₋₄ alkyl)₂, NO₂, O-C₁₋₄ alkyl, phenyl, COOH, and SO₃H, wherein the C₁₋₄ alkyl is optionally substituted with halogen(s),
   X is N or CH,
   Y is N or CH,
   Z is O, S, CH₂, N-CₚH₂ₚ₊₁, and
   p is an integer of 0 to 4,
   or a salt or solvates thereof;
(2) the compound according to claim (1), wherein the compound is selected from the group consisting of BF-124, BF-125, BF-126, BF-133, BF-136, BF-142, BF-143, BF-147, BF-148, BF-150, BF-151, BF-154, BF-160, BF-162, BF-165, BF-168, BF-172, BF-180, BF-191, BF-192, BF-196, BF-197, BF-198, BF-200, BF-201, BF-203, BF-206, BF-208, BF-225, BF-227, BF-228, N-227, N-228, N-276, N-282, N-283, and N-407;
(3) the compound according to (1), wherein the compound is selected from the group consisting of BF-124, BF-148, BF-165, BF-168, BF-191, BF-192, BF-196, BF-197, BF-198, BF-200, BF-201, BF-203, BF-206, BF-208, BF-227, BF-228, N-276, N-277, and N-313;
(4) the compound according to any one of (1) to (3), wherein the compound is labeled, or a salt or solvate thereof;
(5) the compound according to any one of (1) to (3), wherein the compound is labeled with a radionuclide, or a salt or solvate thereof;
(6) the compound according to any one of (1) to (3), wherein the compound is labeled with a γ-ray emitting nuclide, or a salt or solvate thereof;
(7) the compound according to any one of (1) to (3), wherein the compound is labeled with a γ-ray emitting nuclide selected from the group consisting of ^{99m}Tc, ¹¹¹In, ⁶⁷Ga, ²⁰¹Tl, ¹²³I, and ¹³³Xe, or a salt or solvate thereof;
(8) the compound according to any one of (1) to (3), wherein the compound is labeled with a γ-ray emitting nuclide selected from the group consisting of ^{99m}Tc and ¹²³I, or a salt or solvate thereof;
(9) the compound according to any one of (1) to (3), wherein the compound is labeled with a positron emitting nuclide, or a salt or solvate thereof;
(10) the compound according to any one of (1) to (3), wherein the compound is labeled with a positron emitting nuclide selected from the group consisting of ¹¹C, ¹³_{N}, ¹⁵O, and ¹⁸F, or a salt or solvate thereof;
(11) the compound according to any one of (1) to (3), wherein the compound is labeled with ¹⁸F, or a salt or solvate thereof;
(12) a composition for the diagnosis of diseases in which prion protein is accumulated, comprising a compound according to any one of (1) to (11), or a salt or solvate thereof and a pharmaceutically acceptable carrier;
(13) a kit for the diagnosis of diseases in which prion protein is accumulated, comprising a compound according to any one of (1) to (11), or a salt or solvate thereof as the essential ingredient;
(14) a method for the diagnosis of diseases in which prion protein is accumulated, which comprises employing a compound according to any one of (1) to (11), or a salt or solvate thereof;
(15) the composition according to (12), the kit according to (13), or the method according to (14), wherein the compound is a compound according to (2);
(16) a composition for the imaging diagnosis of diseases in which prion protein is accumulated, comprising a compound according to any one of (5) to (11), or a pharmaceutically acceptable salt or solvate thereof and a pharmaceutically acceptable carrier;
(17) the composition according to (16), comprising a compound according to (8), or a pharmaceutically acceptable salt or solvate thereof;
(18) the composition according to (16), comprising a compound according to (11), or a pharmaceutically acceptable salt or solvate thereof;
(19) a kit for the imaging diagnosis of diseases in which prion protein is accumulated, comprising a compound according to any one of (5) to (11), or a pharmaceutically acceptable salt or solvate thereof as the essential ingredient;
(20) the kit according to (19), comprising a compound according to (8), or a pharmaceutically acceptable salt or solvate thereof as the essential ingredient;
(21) the kit according to (19), comprising a compound according to (11), or a pharmaceutically acceptable salt or solvate thereof as the essential ingredient;
(22) a method for the imaging diagnosis of diseases in which prion proteinisaccumulated,characterized by employing a compound accordingtoanyoneof (5) to (11), or a pharmaceutically acceptable salt or solvate thereof;
(23) the composition according to any one of (16) to (18), the kit according to any one of (19) to (21), or the method according to (22), wherein the compound is a compound according to (3) labeled with a γ-ray or positron emitting nuclide, and the imaging diagnosis is carried out by PET or SPECT;
(24) a composition for staining abnormal prion protein in samples, comprising a compound according to any one of (1) to (11), or a salt or solvate thereof;
(25) a kit for staining abnormal prion protein in samples, comprising a compound according to any one of (1) to (11), or a salt or solvate thereof as the essential ingredient;
(26) a method for staining abnormal prion protein in samples, characterized by employing a compound according to any one of (1) to (11), or a salt or solvate thereof;
(27) the composition according to (24), the kit according to (25), or the method according to (26), wherein the compound is a compound according to (2);
(28) a composition for the in vitro diagnosis of an individual with a disease having accumulated prion protein in the living body, comprising a compound according to any one of (1) to (11), or a salt or solvate thereof;
(29) a kit for the in vitro diagnosis of an individual with a disease having accumulated prion protein in the living body, comprising a compound according to any one of (1) to (11), or a salt or solvate thereof as the essential ingredient;
(30) a method for the in vitro diagnosis of an individual with a disease having accumulated prion protein in the living body, which comprises obtaining samples from a subject animal, and contacting to said samples a compound according to any one of (1) to (11), or a salt or solvate thereof;
(31) the composition according to (28), the kit according to (29), or the method according to (30), wherein the compound is selected from the group consisting of BF-168, BF-191, BF-192, BF-196, BF-197, BF-198, BF-200, BF-201, BF-203, BF-206, BF-208, BF-227, BF-228, and N-278;,
(32) a pharmaceutical composition f or the prophylaxis and/or treatment of a disease in which the accumulation of prion protein in the body constitutes or partially constitutes the etiology, comprising a compound according to any one of (1), or a pharmaceutically acceptable salt or solvate thereof and a pharmaceutically acceptable carrier;
(33) the pharmaceutical composition according to (32), wherein the disease is selected from the group consisting of transmissible spongiform encephalopathy and prion diseases;
(34) a method for the treatment of a disease in which the accumulation of prion protein in the body constitutes or partially constitutes the etiology, characterized by administrating a compound according to any one of (1), or a pharmaceutically acceptable salt or solvate thereof;
(35) the method according to (34), wherein the disease is selected from the group consisting of transmissible spongiform encephalopathy and prion diseases;
(36) use of a compound according to (1), or a pharmaceutically acceptable salt or solvate thereof for prophylaxis and/or treatment of a disease in which the accumulation of prion protein in the body constitutes or partially constitutes the etiology;
(37) the use according to (36), wherein the disease is selected from the group consisting of transmissible spongiform encephalopathy and prion diseases;
(38) use of a compound of the present invention for manufacturing a medicament for the prophylaxis and/or treatment of a disease in which the accumulation of prion protein in the body constitutes or partially constitutes the etiology;
(39) the use according to (38), wherein the disease is selected from the group consisting of transmissible spongiform encephalopathy and prion diseases;
(40) the composition according to (32) or (33), the kit according to (34) or (35), the method according to (36) or (37), or the method according to (38) or (39), wherein the compound is selected from the group consisting of BF-130, F-135, BF-136, BF-141, BF-146, BF-148, BF-150, BF-153, BF-168, N-220, N-221, N-223, N-224, N-232, N-243, N-246, N-407, N-437, N-441, N-453, N-457, BF-192, BF-193, BF-198, BF-199, BF-201, BF-203, BF-204, BF-206, BF-208, BF-211, BF-213, BF-227, and BF-231;
(41) the composition according to (32) or (33), the kit according to (34) or (35), the method according to (36) or (37), or the method according to (38) or (39), wherein the compound is selected from the group consisting of BF-130, BF-135, BF-146, N-407, N-437, N-441, N-453, N-457, BF-208, BF-227, BF-231, BF-192, BF-193, BF-198, BF-199, BF-201, BF-203, BF-204, BF-206, BF-208, BF-211, BF-213, N-220, N-221, N-223, and N-224;
(42) a labeled precursor of a compound according to (1) or (11); and
(43) a labeled precursor of BF-168, BF-224, or N-227, wherein the precursor is a tosylate derivative.

### BRIEF DESRIPTION OF THE DRAWINGS

Fig. 1 shows the detection of spotted depositions of abnormal prion proteins (kuru plaques, indicated by arrowheads in the figure) in brain sections of a GSS patient with compounds of the present invention (BF-124, N-276, N-277, BF-283, andBF-162) (scale bar: 100 µm).
Fig. 2 shows the detection of spotted depositions of abnormal prion proteins (kuru plaques, indicated by arrowheads in the figure) in brain sections of a GSS patient with compounds of the present invention (BF-125, N-282, BF-133, BF-145, BF-148, and BF-165) (scale bar: 100 µm).
Fig. 3 shows the detection of spotted depositions of abnormal prion proteins (kuru plaques, indicated by arrowheads in the figure) in brain sections of a GSS patient with compounds of the present invention (BF-168 and BF-169) (scale bar: 100 µm).
Fig. 4 shows the detection of spotted depositions of abnormal prion proteins (kuru plaques, indicated by arrowheads in the figure) in brain sections of a GSS patient with compounds of the present invention (BF-126, BF-166, and N-398) (scale bar: 100 µm).
Fig. 5 shows the detection of spotted depositions of abnormal prion proteins (kuru plaques, indicated by arrowheads in the figure) in brain sections of a GSS patient with a compound of the present invention (BF-136) (scale bar: 100 µm).
Fig. 6 shows the detection of spotted depositions of abnormal prion proteins (kuru plaques, indicated by arrowheads in the figure) in brain sections of a GSS patient with a compound of the present invention (BF-142) (scale bar: 100 µm).
Fig. 7 shows the detection of spotted depositions of abnormal prion proteins (kuru plaques, indicated by arrowheads in the figure) in brain sections of a GSS patient with a compound of the present invention (BF-151) (scale bar: 100 µm).
Fig. 8 shows the detection of spotted depositions of abnormal prion proteins (kuru plaques, indicated by arrowheads in the figure) in brain sections of a GSS patient with a compound of the present invention (BF-154) (scale bar: 100 µm).
Fig. 9 shows the detection of spotted depositions of abnormal prion proteins (kuru plaques, indicated by arrowheads in the figure) in brain sections of a GSS patient with compounds of the present invention (N-310 and N-313) (scale bar: 100 µm).
Fig. 10 shows the detection of spotted depositions of abnormal prion proteins (kuru plaques, indicated by arrowheads in the figure) in brain sections of a GSS patient with a compound of the present invention (BF-227) (scale bar: 100 µm).
Fig. 11 shows the detection of spotted depositions of abnormal prion proteins (kuru plaques, indicated by arrowheads in the figure) in brain sections of a GSS patient with a compound of the present invention (N-227) (scale bar: 100 µm).
Fig. 12 shows the detection of spotted depositions of abnormal prion proteins (kuru plaques, indicated by arrowheads in the figure) in brain sections of a GSS patient with a compound of the present invention (N-407) (scale bar: 100 µm).
Fig. 13 shows the detection of spotted depositions of abnormal prion proteins (kuru plaques, indicated by arrowheads in the figure) in brain sections of a GSS patient with compounds of the present invention (N-408, N-438, N-440, N-441, and N-454) (scale bar: 100 µm).
Fig. 14 shows the detection of spotted depositions of abnormal prion proteins (kuru plaques, indicated by arrowheads in the figure) in brain sections of a GSS patient with a compound of the present invention (SA-271) (scale bar: 100 µm).
Fig. 15 shows the detection of spotted depositions of abnormal prion proteins (kuru plaques, indicated by arrowheads in the figure) in brain sections of a GSS patient with a compound of the present invention (BF-179) (scale bar: 100 µm).
Fig. 16 shows immunostaining of abnormal prion proteins in brain sections of a GSS patient (PrP GSS).
Fig. 17 shows inhibitory effects of compounds of the present invention (BF-124, N-276, N-277, BF-283, and BF-162) on producing abnormal prion proteins (indicated by three arrowheads in the figure) in ScNa2 cells with persistent infection of prion.
Fig. 18 shows inhibitory effects of compounds of the present invention (BF-125, N-282, BF-133, andBF-135) on producing abnormal prion proteins (indicated by three arrowheads in the figure) in ScNa2 cells with persistent infection of prion.
Fig. 19 shows inhibitory effects of compounds of the present invention (BF-140, BF-145, BF-146, and BF-148) on producing abnormal prion proteins (indicated by three arrowheads in the figure) in ScNa2 cells with persistent infection of prion.
Fig. 20 shows inhibitory effects of compounds of the present invention (BF-165, BF-168, BF-169, BF-173, and BF-180) on producing abnormal prion proteins (indicated by three arrowheads in the figure) in ScNa2 cells with persistent infection of prion.
Fig. 21 shows inhibitory effects of compounds of the present invention (BF-126, BF-166, N-398, N-404, and N-442) on producing abnormal prion proteins (indicated by three arrowheads in the figure) in ScNa2 cells with persistent infection of prion.
Fig. 22 shows inhibitory effects of a compound of the present invention (BF-136) on producing abnormal prion proteins (indicated by three arrowheads in the figure) in ScNa2 cells with persistent infection of prion.
Fig. 23 shows inhibitory effects of compounds of the present invention (BF-137, BF-138, BF-139, BF-141, and BF-142) onproducing abnormal prion proteins (indicated by three arrowheads in the figure) in ScNa2 cells with persistent infection of prion.
Fig. 24 shows inhibitory effects of compounds of the present invention (BF-151 and BF-161) on producing abnormal prion proteins (indicated by three arrowheads in the figure) in ScNa2 cells with persistent infection of prion.
Fig. 25 shows inhibitory effects of compounds of the present invention (BF-153 and SA-272) on producing abnormal prion proteins (indicated by three arrowheads in the figure) in ScNa2 cells with persistent infection of prion.
Fig. 26 shows inhibitory effects of a compound of the present invention (N-411) on producing abnormal prion proteins (indicated by three arrowheads in the figure) in ScNa2 cells with persistent infection of prion.
Fig. 27 shows inhibitory effects of compounds of the present invention (BF-158, BF-170, N-310, andN-313) on producing abnormal prion proteins (indicated by three arrowheads in the figure) in ScNa2 cells with persistent infection of prion.
Fig. 28 shows inhibitory effects of compounds of the present invention (BF-187 and BF-189) on producing abnormal prion proteins (indicated by three arrowheads in the figure) in ScNa2 cells with persistent infection of prion.
Fig. 29 shows inhibitory effects of compounds of the present invention (N-402, N-457, and N-491) on producing abnormal prion proteins (indicated by three arrowheads in the figure) in ScNa2 cells with persistent infection of prion.
Fig. 30 shows inhibitory effects of a compound of the present invention (N-407) on producing abnormal prion proteins (indicated by three arrowheads in the figure) in ScNa2 cells with persistent infection of prion.
Fig. 31 shows inhibitory effects of compounds of the present invention (N-408, N-438, N-439, N-440, and N-411) on producing abnormal prion proteins (indicated by three arrowheads in the figure) in ScNa2 cells with persistent infection of prion.
Fig. 32 shows inhibitory effects of compounds of the present invention (N-452, N-453, N-454, and N-455) on producing abnormal prion proteins (indicated by three arrowheads in the figure) in ScNa2 cells with persistent infection of prion.
Fig. 33 shows inhibitory effects of compounds of the present invention (N-437, N-463, N-464, N-465, N-467, and N-468) on producing abnormal prion proteins (indicated by three arrowheads in the figure) in ScNa2 cells with persistent infection of prion.
Fig. 34 shows inhibitory effects of compounds of the present invention (N-469, N-471, N-472, N-473, and N-475) on producing abnormal prion proteins (indicated by three arrowheads in the figure) in ScNa2 cells with persistent infection of prion.
Fig. 35 shows inhibitory effects of a compound of the present invention (SA-271) on producing abnormal prion proteins (indicated by three arrowheads in the figure) in ScNa2 cells with persistent infection of prion.
Fig. 36 shows inhibitory effects of compounds of the present invention (BF-178 and BF-179) on producing abnormal prion proteins (indicated by three arrowheads in the figure) in ScNa2 cells with persistent infection of prion.

### DETAILED DESCRIPTION OF THE INVENTION

The compounds of the present invention are represented by the general formula (I) or (II): 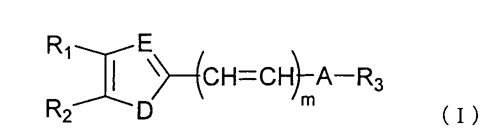 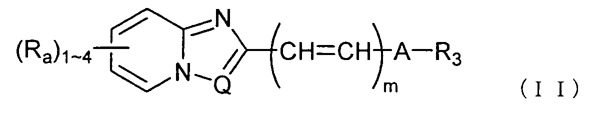 and have high specificity for abnormal prion protein. Thus, these compounds, including their salts or solvates, can be used for the diagnosis, prophylaxis, and/or treatment of diseases in which prion protein is accumulated. The compounds of the formula (I) or (II) also can be used as agents for staining abnormal prion protein. The compounds of the formula (I) or (II) may be labeled. In particular, radioactively labeled compounds of the formula (I) or (II) are suitable for the imaging diagnosis of diseases in which prion protein is accumulated.

Substances which can be used as diagnostic probes of the present invention are compounds represented by the general formula (I) or (II), or salts or solvates thereof. Diagnosis, as referred to herein, is intended to include imaging diagnosis, unless otherwise specified.

The following explanation is given of the structure and substituents of the compounds of the formula (I) or (II).

As referred to herein, "C₁₋₄ alkyl" (alkyl having one to four carbons) is intended to include methyl, ethyl, propyl, butyl, and structural isomers thereof.

"Halogen" means fluorine, chlorine, bromine, or iodine.

D is NR' , S, O, CH=CH, or CH₂. R' is H, C₁₋₄ alkyl, or phenyl, wherein the C₁₋₄ alkyl is optionally substituted with halogen(s). Preferably, D is S, O, or CH, with R' being preferably H.

E is N or CH.

Each Rₐ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, and SO₃H, wherein the C₁₋₄ alkyl is optionally substituted with halogen(s). Preferred Rₐ is H, C₁₋₄ alkyl, and halogen.

Q is N or CR_{b}.

R_{b} is selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, and SO₃H, wherein the C₁₋₄ alkyl is optionally substituted with halogen(s).

Preferred R_{b} is H and C₁₋₄ alkyl.

m is an integer of 0 to 4, and preferably, m is 0 or 1. When m is 1 or more, a compound of the present invention may have its cis and trans isomers, both of which are contemplated in the present invention.

R₁ and R₂ are independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, and SO₃H, wherein the C₁₋₄ alkyl is optionally substituted with halogen (s). Alternatively, R₁ and R₂, together, form a benzene or naphthalene ring which is optionally substituted with one to four R₄s. Preferred R₁ and R₂ are hydrogen and methyl. It is also preferable that R₁ and R₂, together, may form an optionally substituted benzene ring.

R₃ is selected from the group consisting of H, C₁₋₄ alkyl, halogen, OH, C₁₋₄alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, SO₃H, wherein the C₁₋₄ alkyl is optionally substituted with halogen(s), and any one of the moieties represented by (a) to (e): 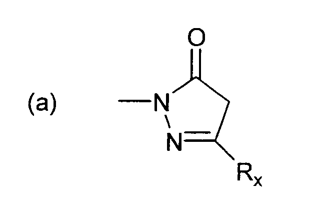 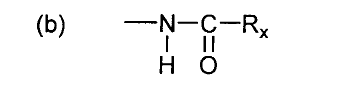 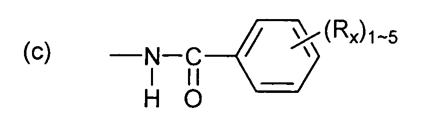 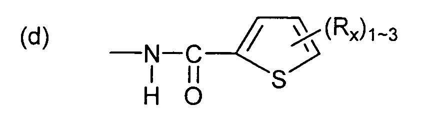 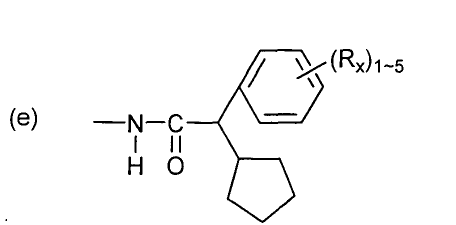 wherein each Rₓ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, N=CH-allyl, NO₂, O-C₁₋₄ alkyl, COOH, and SO₃H, wherein the C₁₋₄ alkyl is optionally substituted with halogen(s).

Preferred R₃ is H, NH₂, NH(C₁₋₄ alkyl), and N(C₁₋₄ alkyl)₂. Preferred Rₓ is H, halogen, NH₂, NH(C₁₋₄ alkyl), and N(C₁₋₄ alkyl)₂, or Rₓ may be any one of (a) to (e).

Each R₄ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, SO₃H, wherein the C₁₋₄ alkyl is optionally substituted with halogen (s), and any one of the moieties represented by (f) to (1): 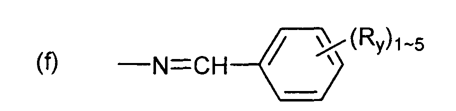 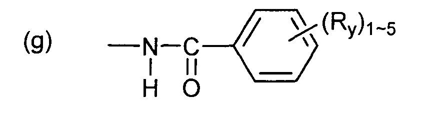 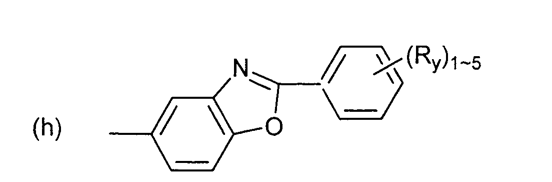 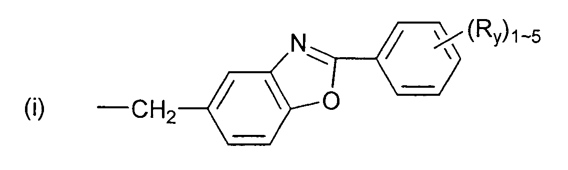 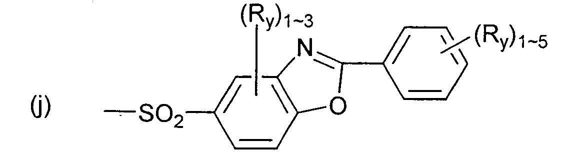 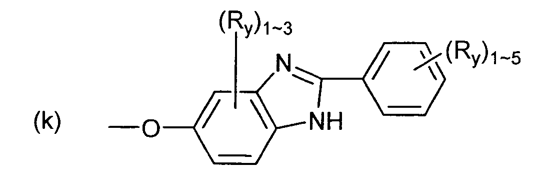 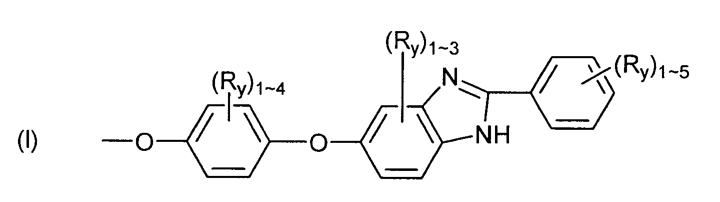 wherein two R₄s attached on adjacent carbons may form a methylenedioxy group, and wherein the C₁₋₄ alkyl is optionally substituted with halogen(s), and
wherein each R_{y} is independently selected from the group consisting of H, C₁₋₄ alkyl, halogen, OH, C₁₋₄ alkyl-OH, C₁₋₄ alkyl-O- C₁₋₄ alkyl, NH₂, NH(C₁₋₄ alkyl), N(C₁₋₄ alkyl)₂, NO₂, O-C₁₋₄ alkyl, COOH, and SO₃H,
wherein the C₁₋₄ alkyl is optionally substituted with halogen (s).

In the case that R₁ and R₂, together, form a benzene or naphthalene ring, preferred R₄ substituent on the ring is H, halogen, OH, NO₂, NH₂, and optionally substituted C₁₋₄ alkyl, and may be any one of (f) to (1) described above. Preferred R_{y} is H, halogen, and NH₂. A is any one of the rings represented by (i) to (ix) described below: 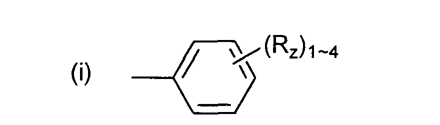 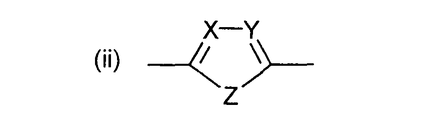 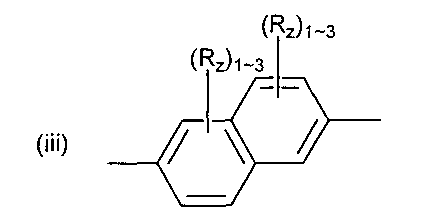 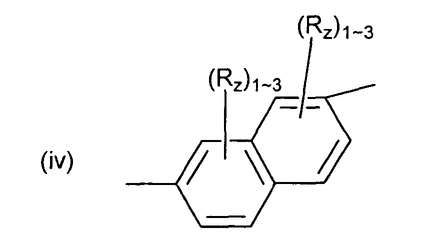 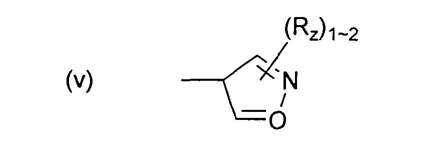 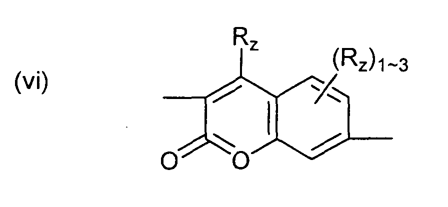 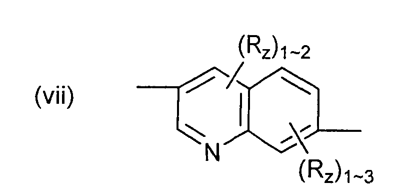 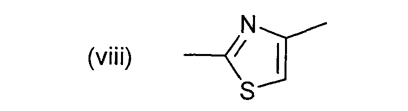 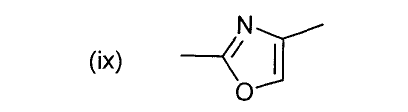 wherein each R_{z} is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, phenyl, COOH, and SO₃H, wherein the C₁₋₄ alkyl is optionally substituted with halogen(s).

Preferred ring A is a benzene and naphthalene rings. Preferred R_{z} is H, C₁₋₄ alkyl, halogen, and OH.

X is N or CH. Y is N or CH. Z is O, S, CH₂, or N-CₚH₂ₚ₊₁, and p is an integer of 0 to 4. Preferably, Z is O, S, CH₂, or N-CH₃.

Also included in the present invention are salts of the compounds of the present invention of the formula (I) or (II). Salts may be formed with a nitrogen atom or atoms, or any functional group of the compounds of the formula (I) or (II). For example, in the case of a compound possessing a carboxyl or sulfonic acid group, salts may be formed between the group and metals. Examples of such salts include salts with alkali metals such as lithium, sodium, and potassium, and with alkaline earth metals such as magnesium, calcium, and barium, and others. In the case of compounds of the formula (I) or (II) possessing one or more hydroxyl groups, compounds in which the hydrogen of a hydroxyl group is substituted with a metal such as sodium, potassium, or the like are also encompassed in the present invention. In addition, complexes which are formed from compounds of the formula (I) or (II) and metal salts (for example, complexes formed with metal salts such as magnesium chloride and iron chloride) are herein intended to be included in salts of the compounds of the formula (I) or (II). When salts of the compounds of the present invention are used in compositions, kits, or methods applicable to the body of subjects, they are preferably salts that are pharmaceutically acceptable. Also, a compound of the present invention (I) may form onium salts with anions, depending on types of its substituents. Such anions include halide, organic acid, sulfonate, perchlorate ions, and others. It is preferable that such onium salts also are pharmaceutically acceptable. Pharmaceutically acceptable salts of the compounds of the formula (I) or (II) include, for example, salts with halide ions, such as, of chlorine, bromine, and iodine, or alternatively, salts with metals such as sodium, potassium, and calcium. Such salts are encompassed in the present invention. Further, some of the compounds of the present invention can be complexed with metal salts such iron chloride and cobalt chloride, and such salts are also encompassed in the present invention. Additionally, solvates of the compounds of the formula (I) or (II) are also encompassed in the present invention. Solvates include hydrates, methanolates, ethanolates, ammoniates, and others. When solvates of the compounds of the present invention are used in compositions, kits, or methods applicable to the body of subjects, they are preferably solvates that are pharmaceutically acceptable. Pharmaceutically acceptable solvates include hydrate, ethanolates, and others.

When herein referred to an "compounds of the present invention (s)" or "compound (s) of the present invention", reference is made to a compound (s) represented by the formula (I) or (II), which may be unlabeled or labeled. Additionally, its/their salts and solvates, if any, are intended to included. For example, when "N-437" is referred to, it is intended to include the compound N-437 which is unlabeled or labeled, and further its salts (for example, hybrobromide) or solvates (if any).

Examples of the compounds of the present invention are listed in Table 1. As mentioned above, these compounds have high specificity for abnormal prion protein, and thus will find applications as diagnostic probes for diseases in which prion protein is accumulated, agents for specifically staining abnormal prion protein, therapeutics against diseases in which prion protein is accumulated, or others.

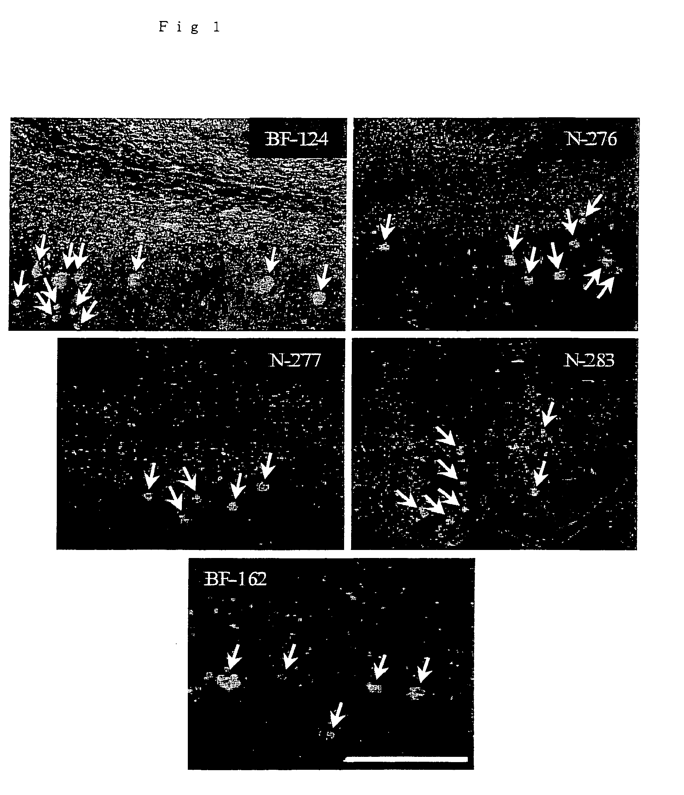

From the viewpoint of the clearness of staining of abnormal prion protein, preferable compounds of the present invention include BF-124, BF-125, BF-126, BF-133, BF-136, BF-142, BF-143, BF-147, BF-148, BF-150, BF-151, BF-154, BF-160, BF-162, BF-165, BF-168, BF-172, BF-180, BF-191, BF-192, BF-196, BF-197, BF-198, BF-200, BF-201, BF-203, BF-206, BF-208, BF-225, BF-227, BF-228, N-227, N-228, N-276, N-282, N-283, N-407, and other (see, Example 3, Figs. 1 to 4).

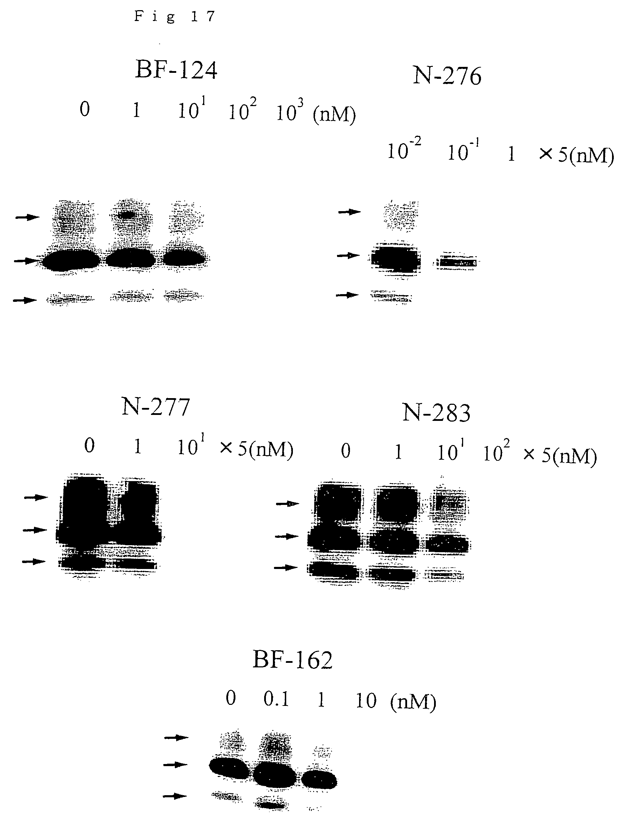

From the viewpoint of anti-prion effects (see, Example 4, Figs. 17 to 36), preferable compounds of the present invention include BF-130, BF-135, BF-136, BF-141, BF-146, BF-148, BF-150, BF-153, BF-168, N-220, N-221, N-223, N-224, N-232, N-243, N-246, N-407, N-437, N-441, N-453, N-457, BF-192, BF-193, BF-198, BF-199, BF-201, BF-203, BF-204, BF-206, BF-208, BF-211, BF-213, BF-227, BF-231, and others. Further, of compounds of the present invention which have anti-prion effects, the following compounds are more preferable: BF-130, BF-135, BF-146, N-407, N-437, N-441, N-453, N-457, BF-208, BF-227, BF-231, BF-192, BF-193, BF-198, BF-199, BF-201, BF-203, BF-204, BF-206, BF-208, BF-211, BF-213, N-220, N-221, N-223, and N-224, taking into account data regarding the property of permeability into the brain, acute toxicity, and the like (see, the Example section). These compounds are expected to be useful also for staining abnormal prion protein as described above, since it is likely that they are highly specific for abnormal prion protein due to having high anti-prion effects. Examples of such compounds include BF-135, BF-146, BF-148, and BF-168 (see, Example 3, Figs. 1 to 15, Example 4, Table 4, Figs. 17 to 36).

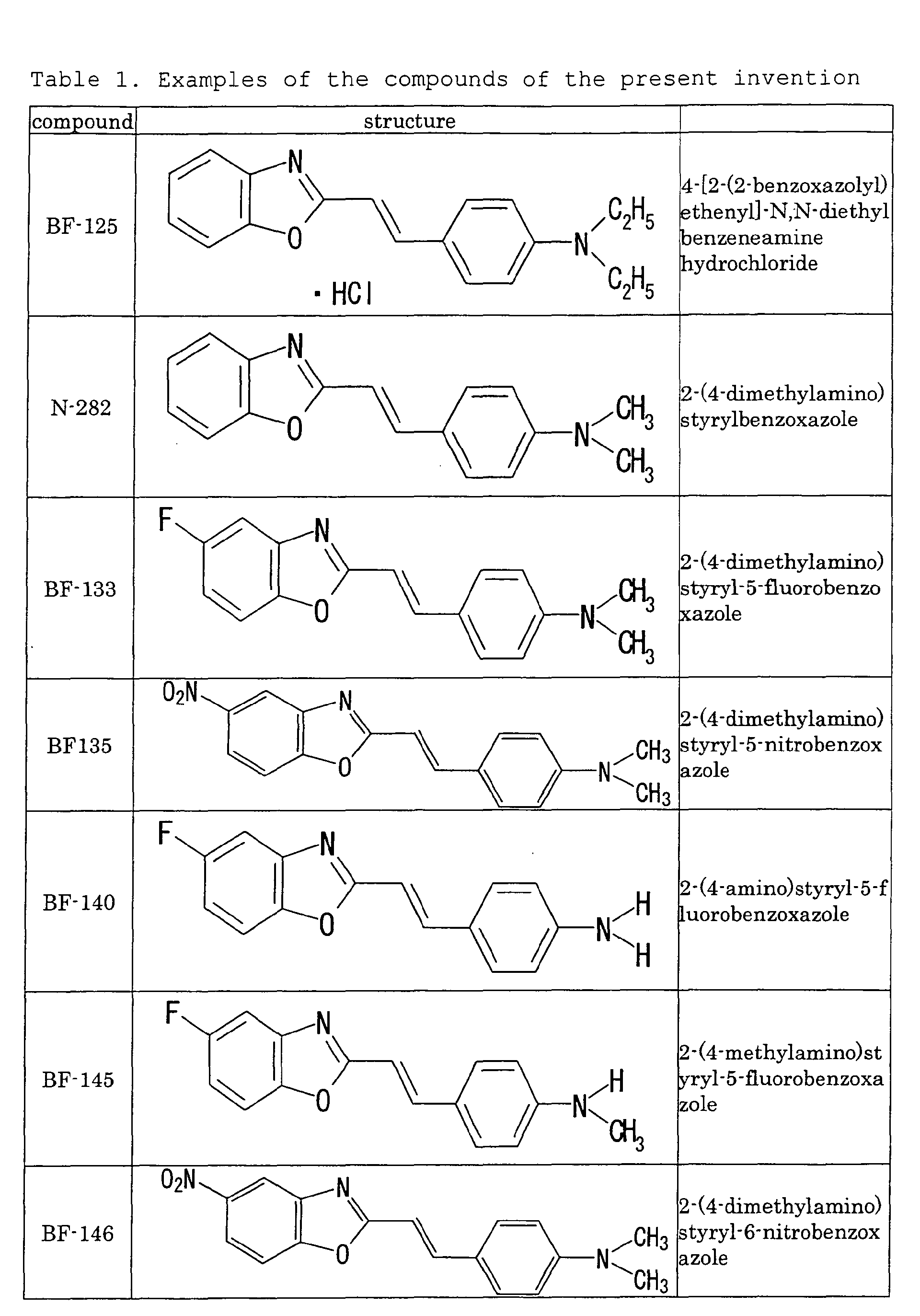

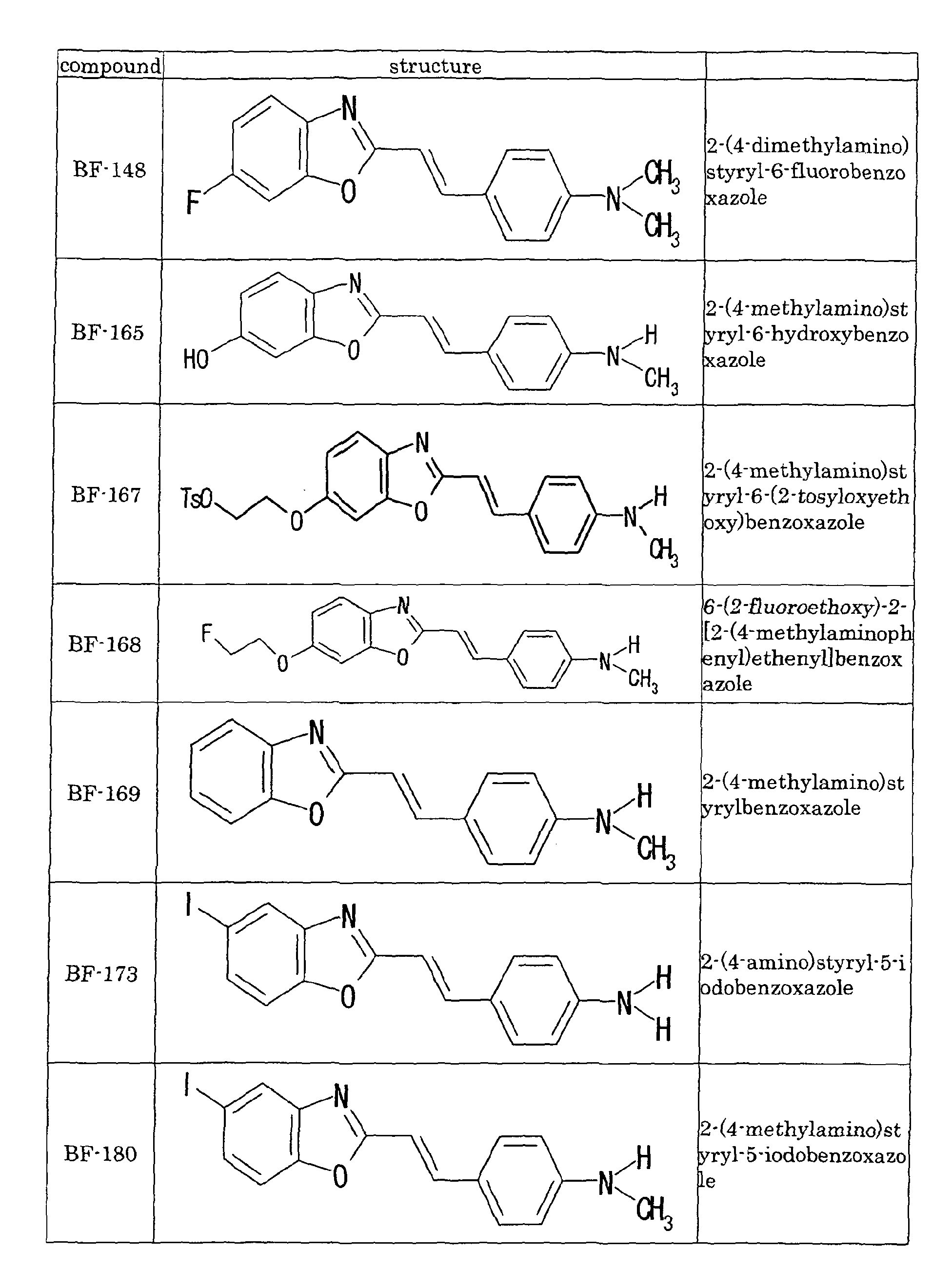

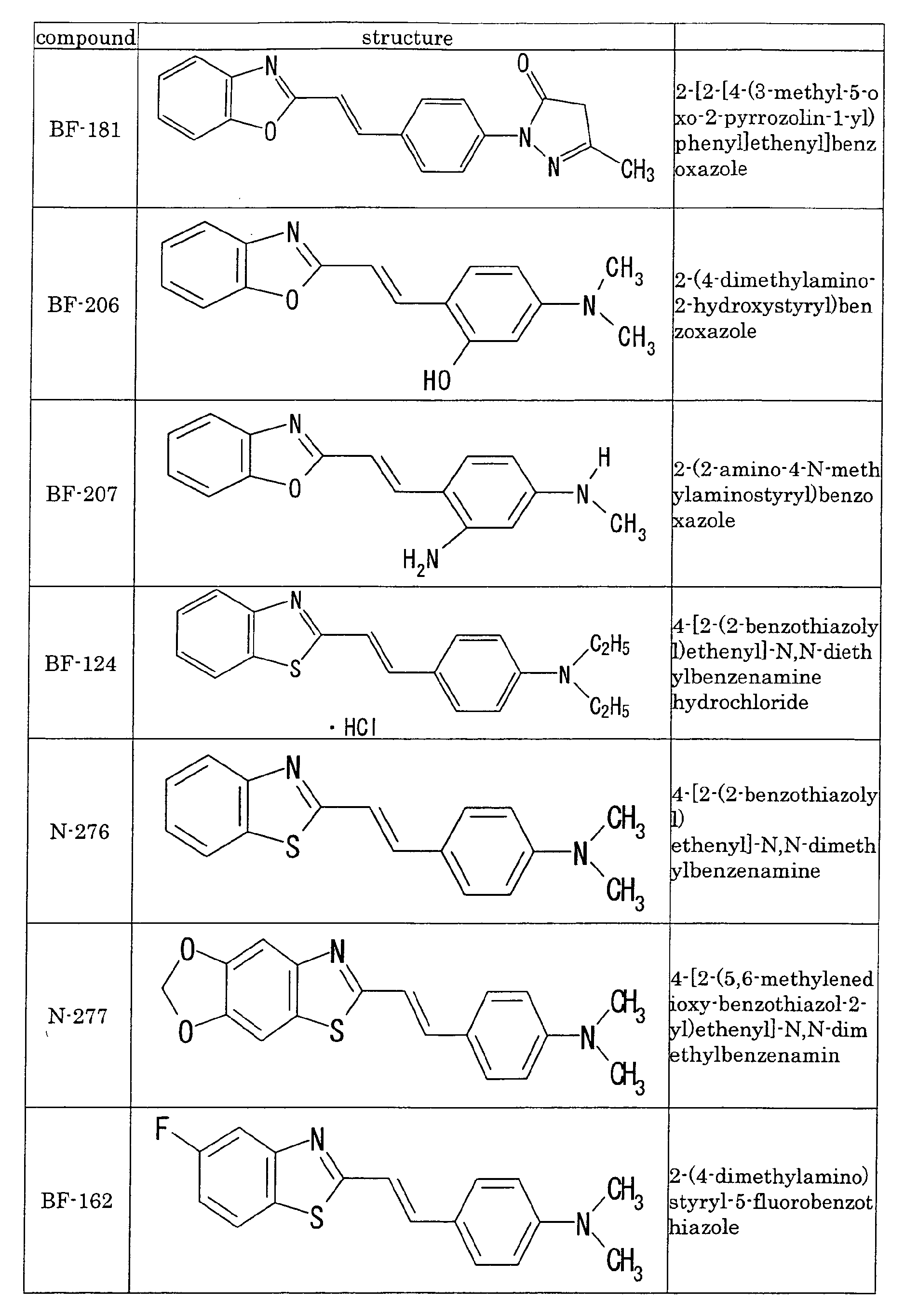

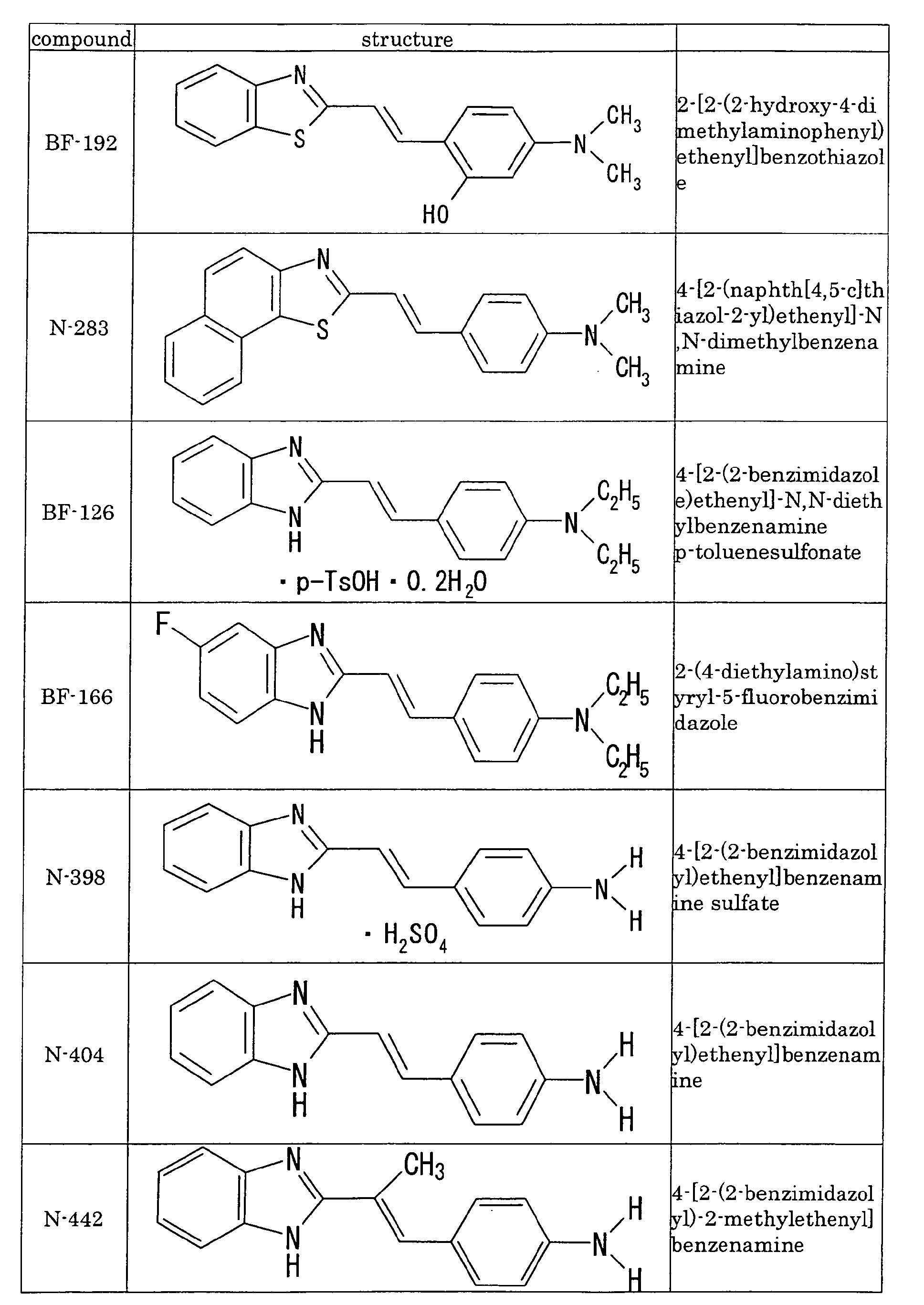

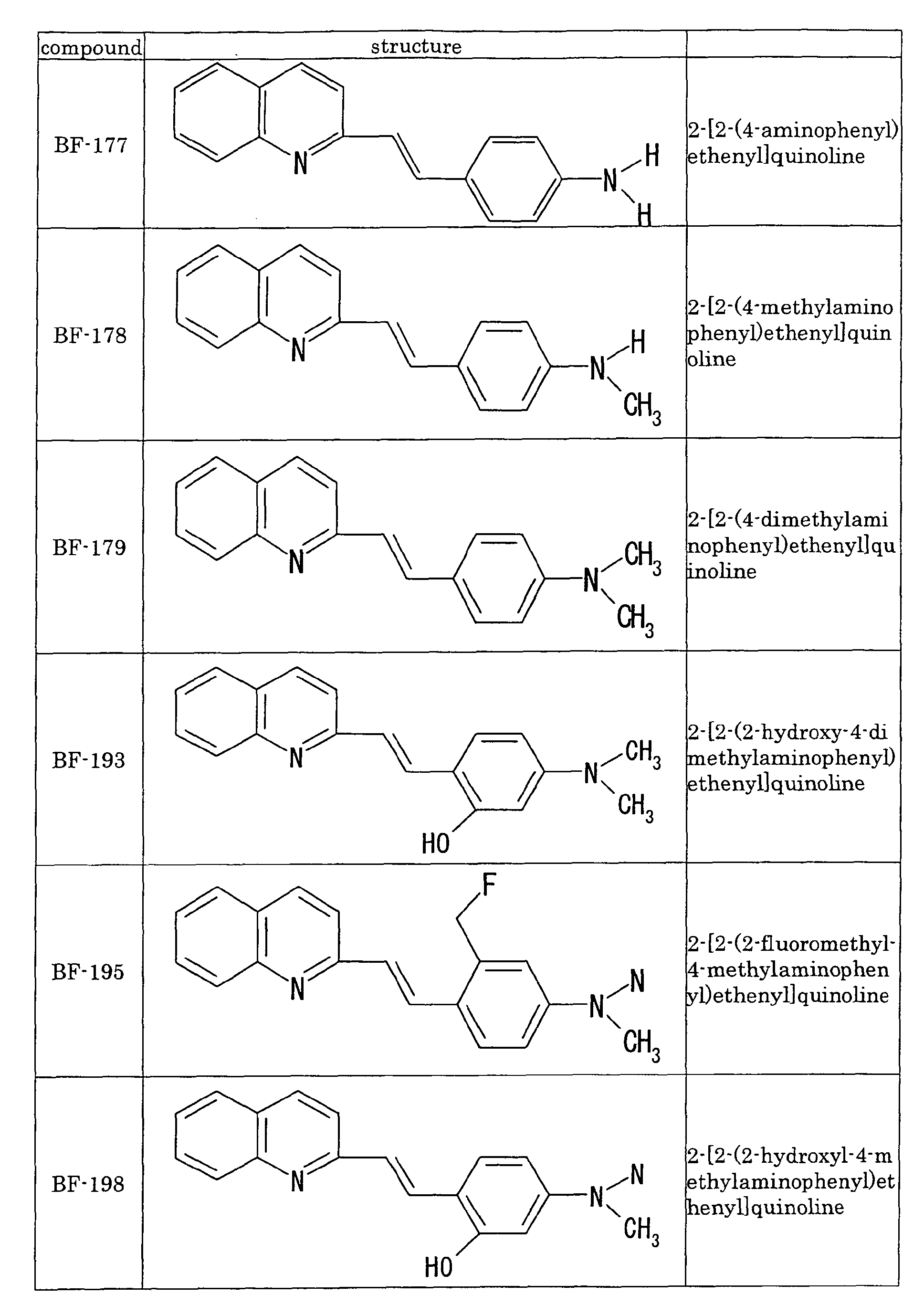

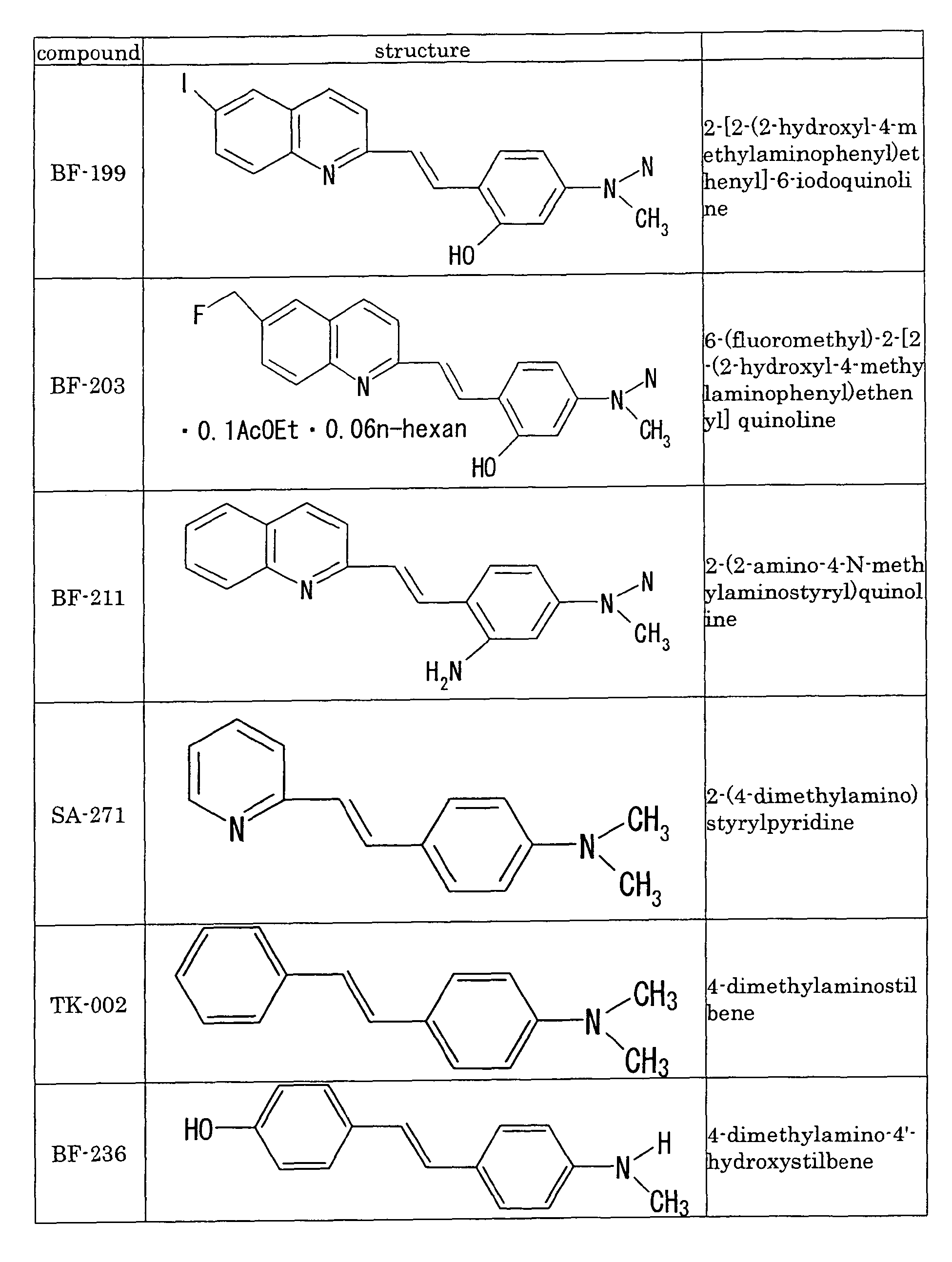

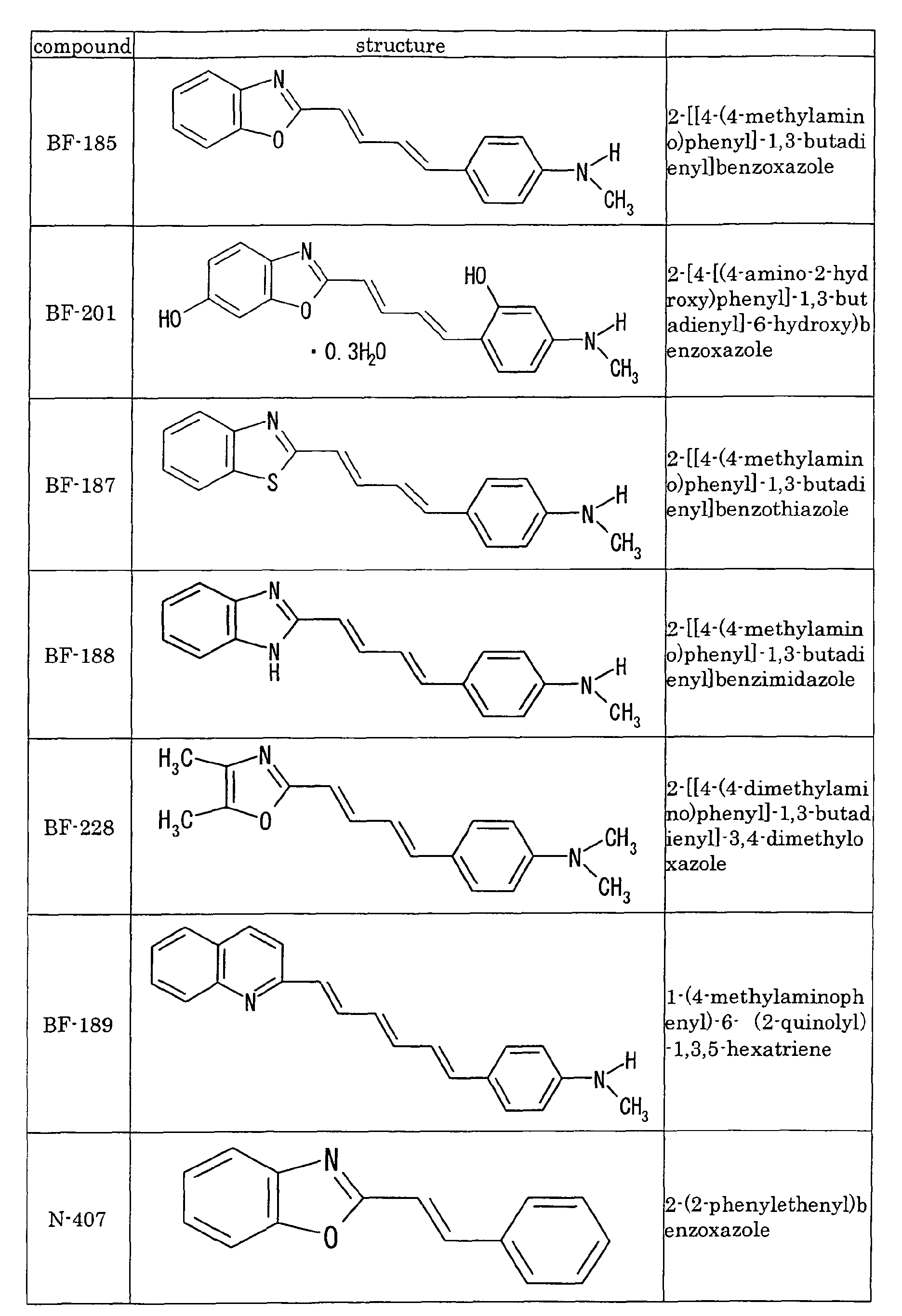

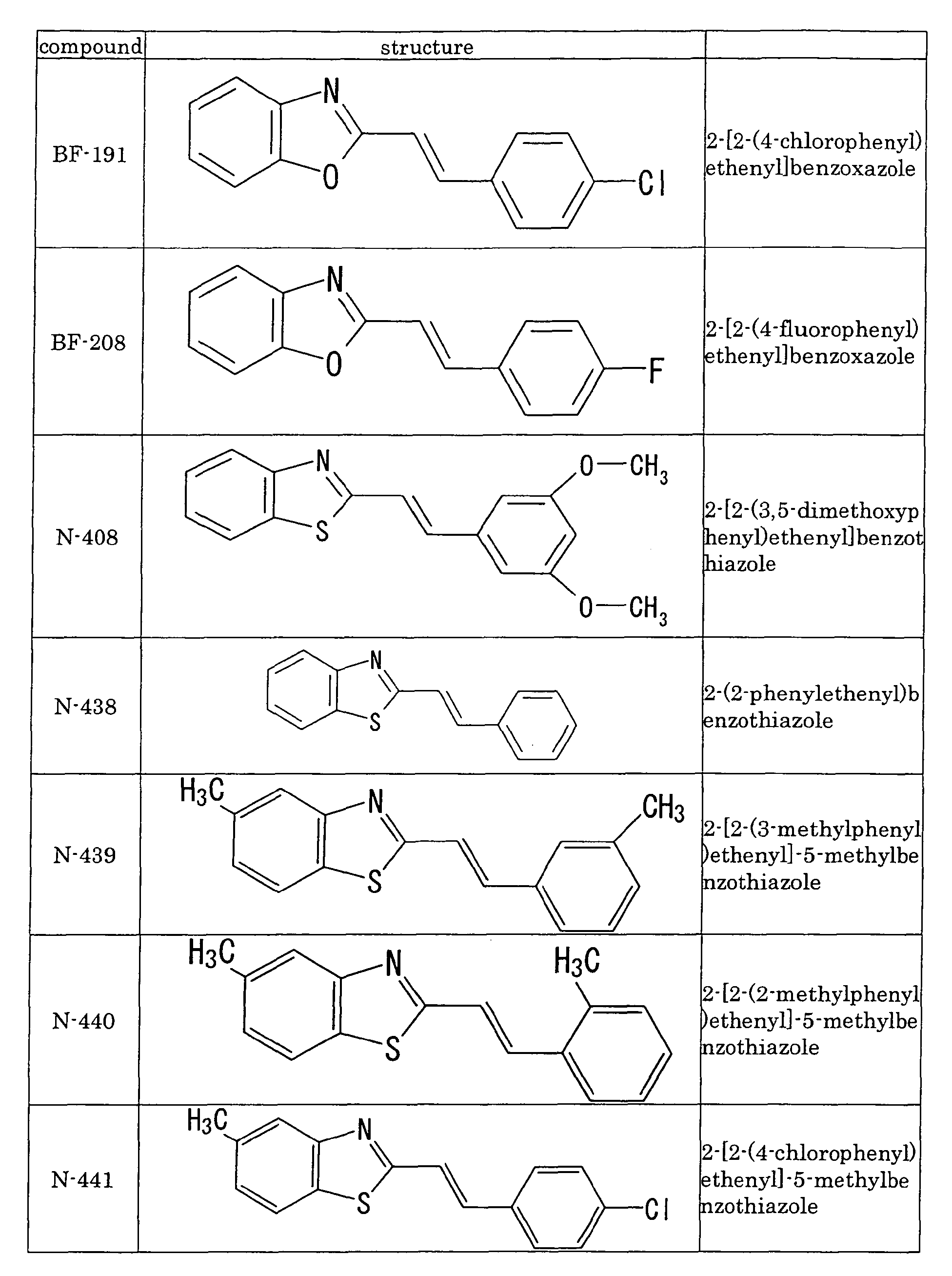

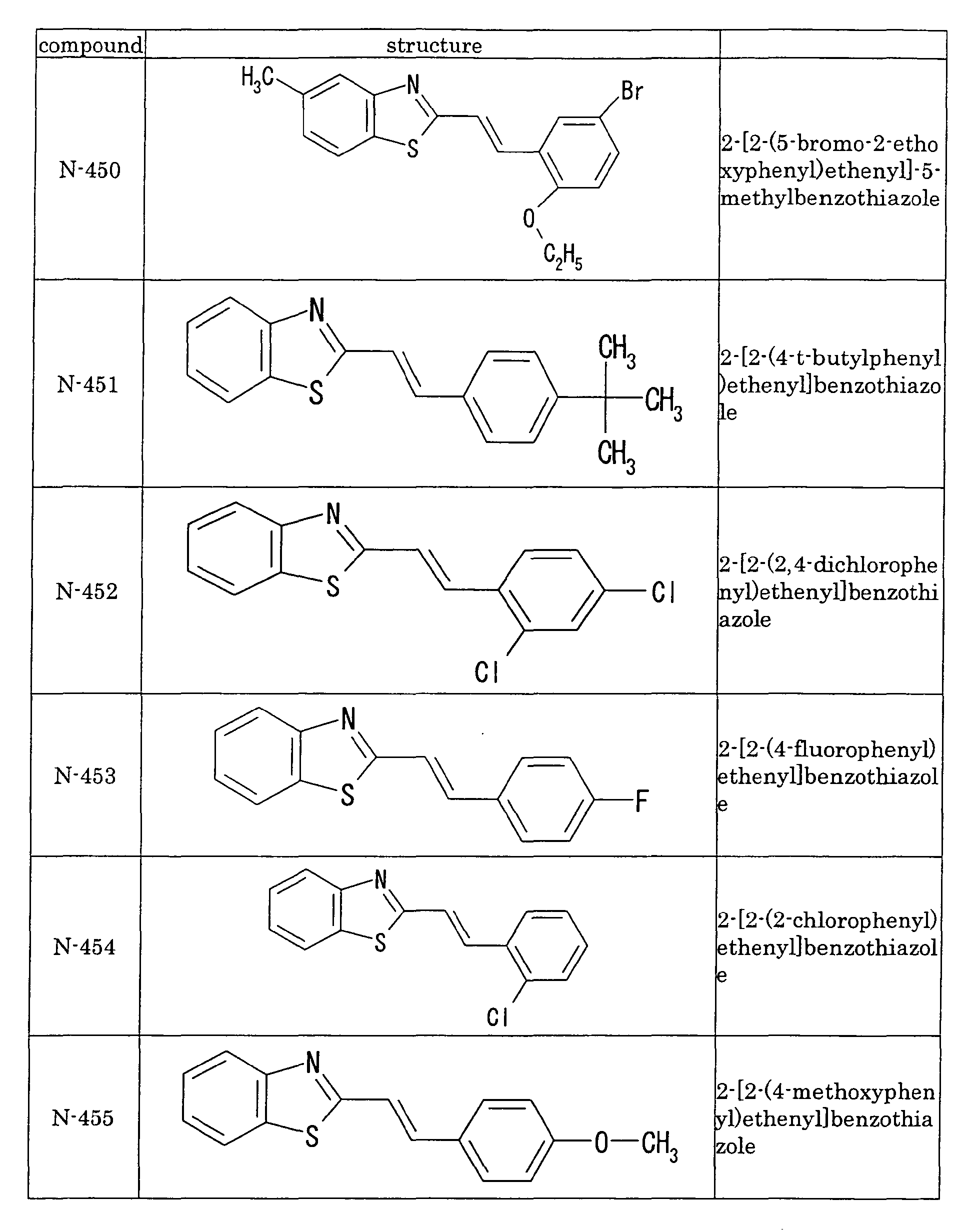

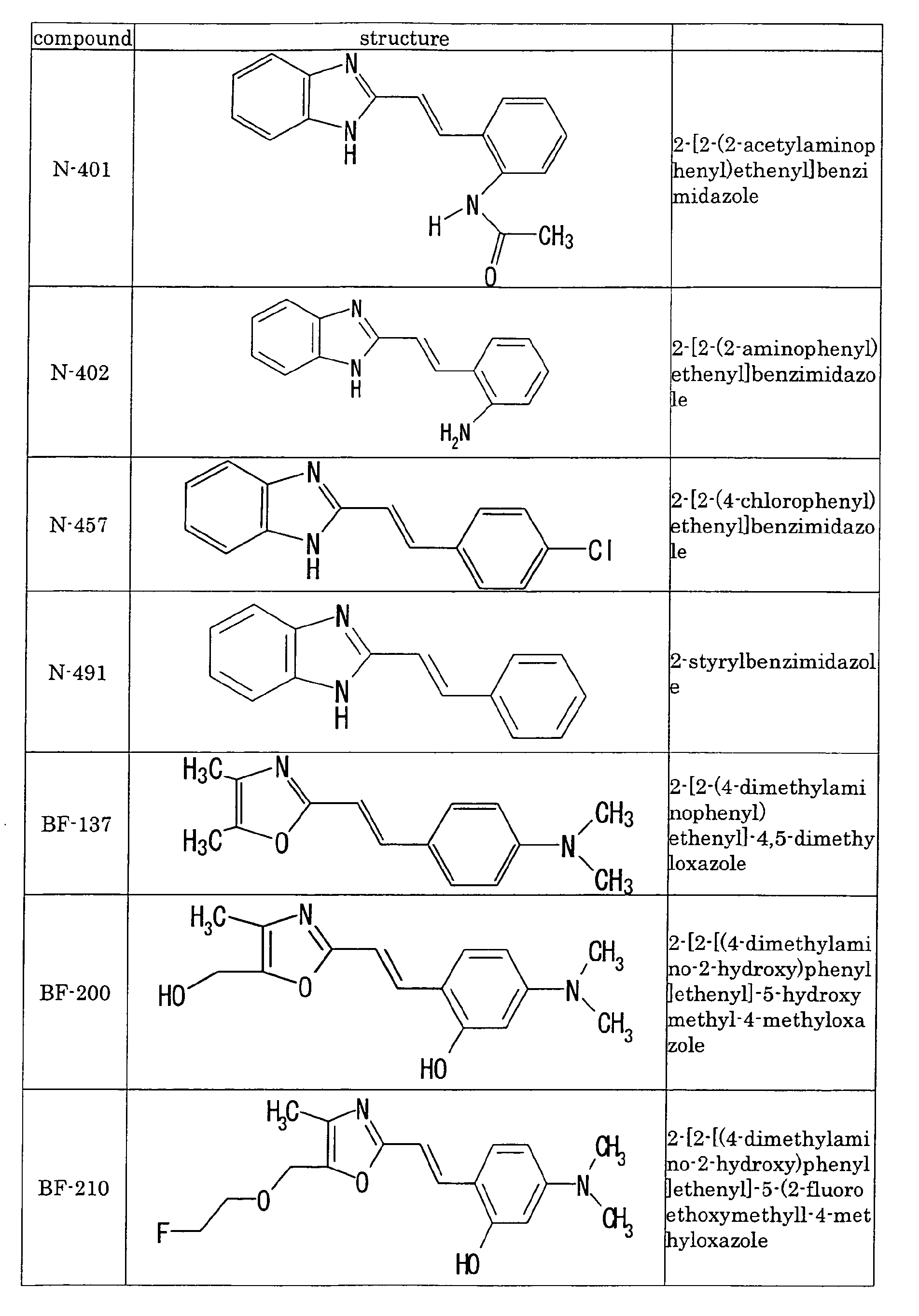

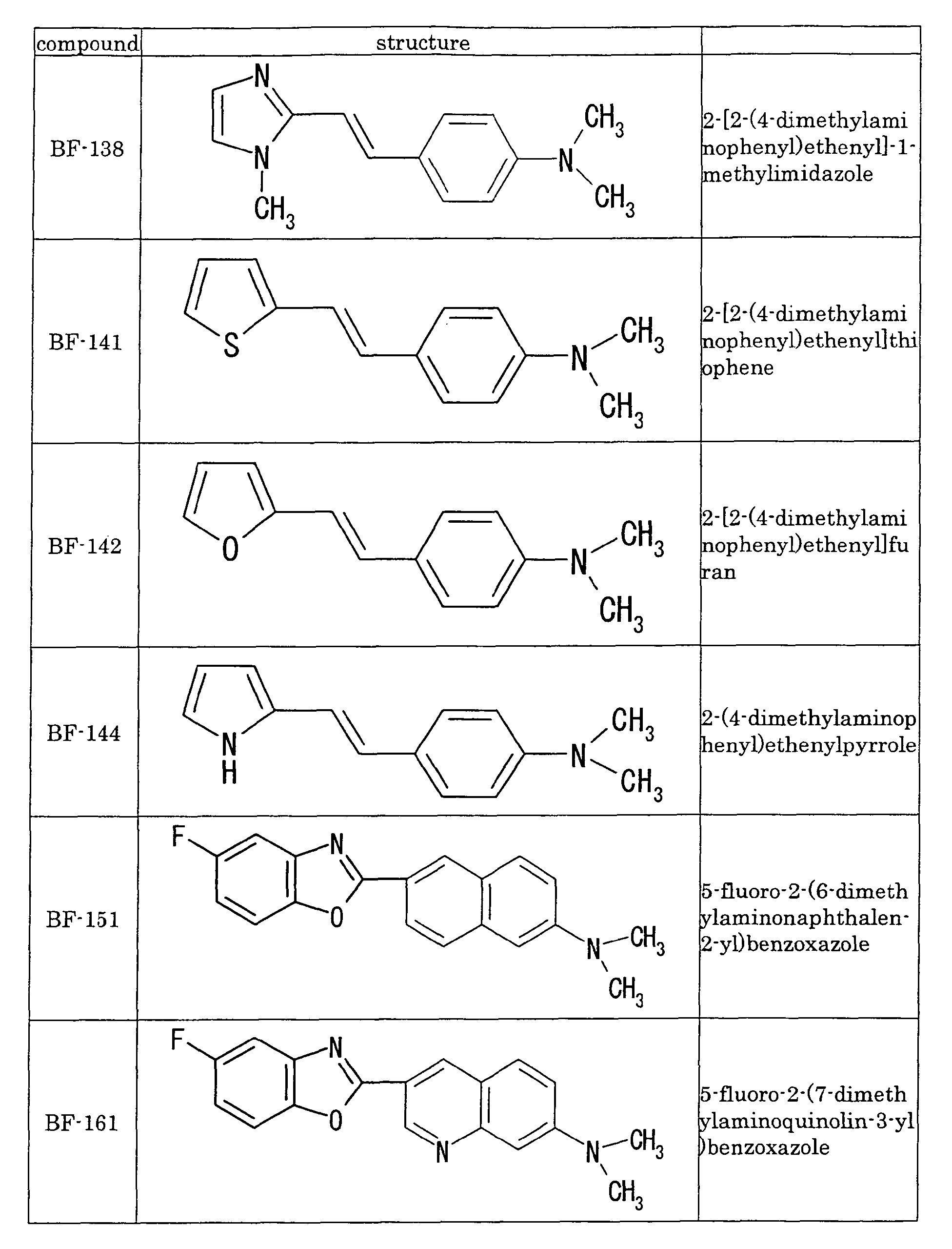

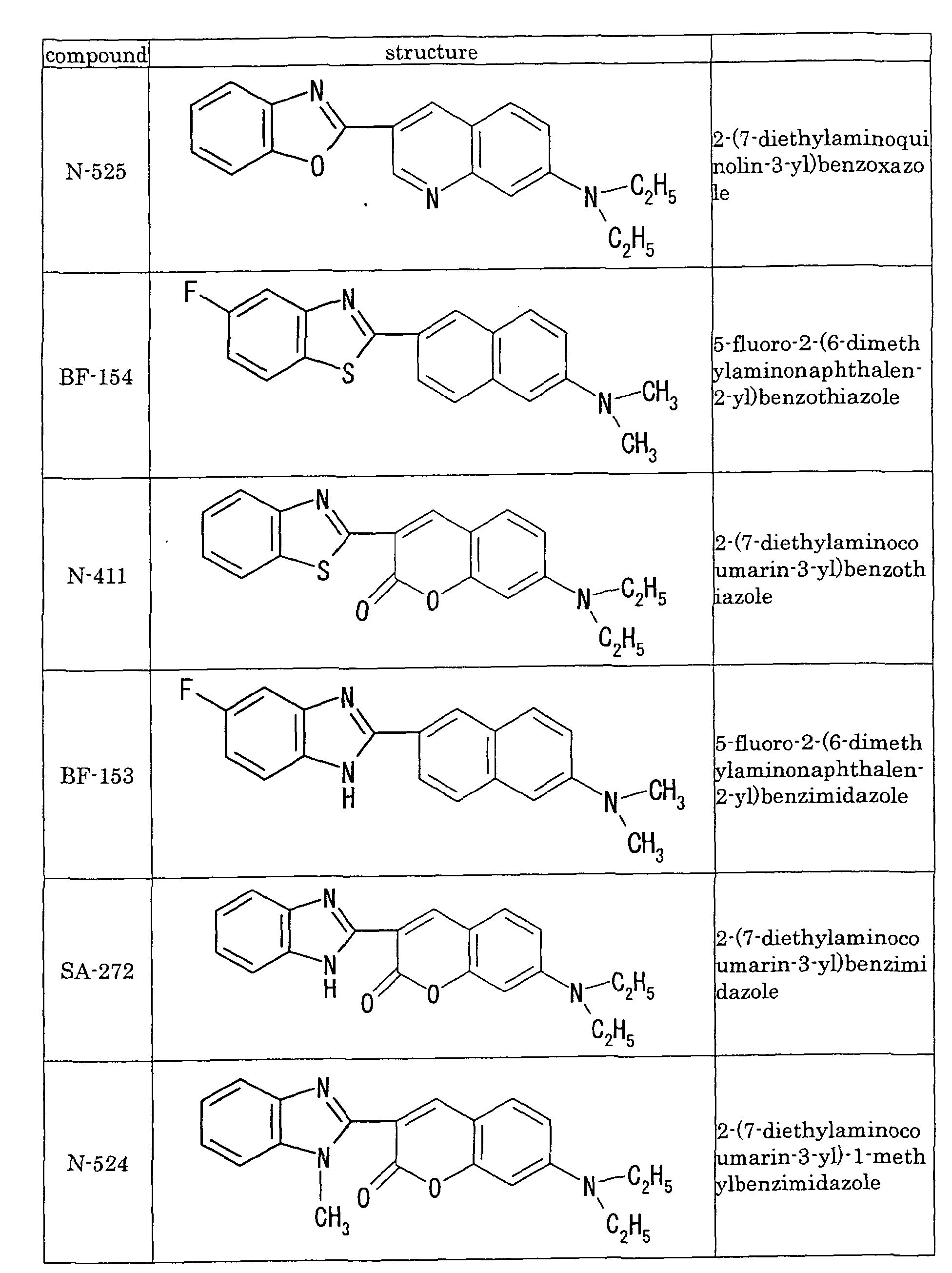

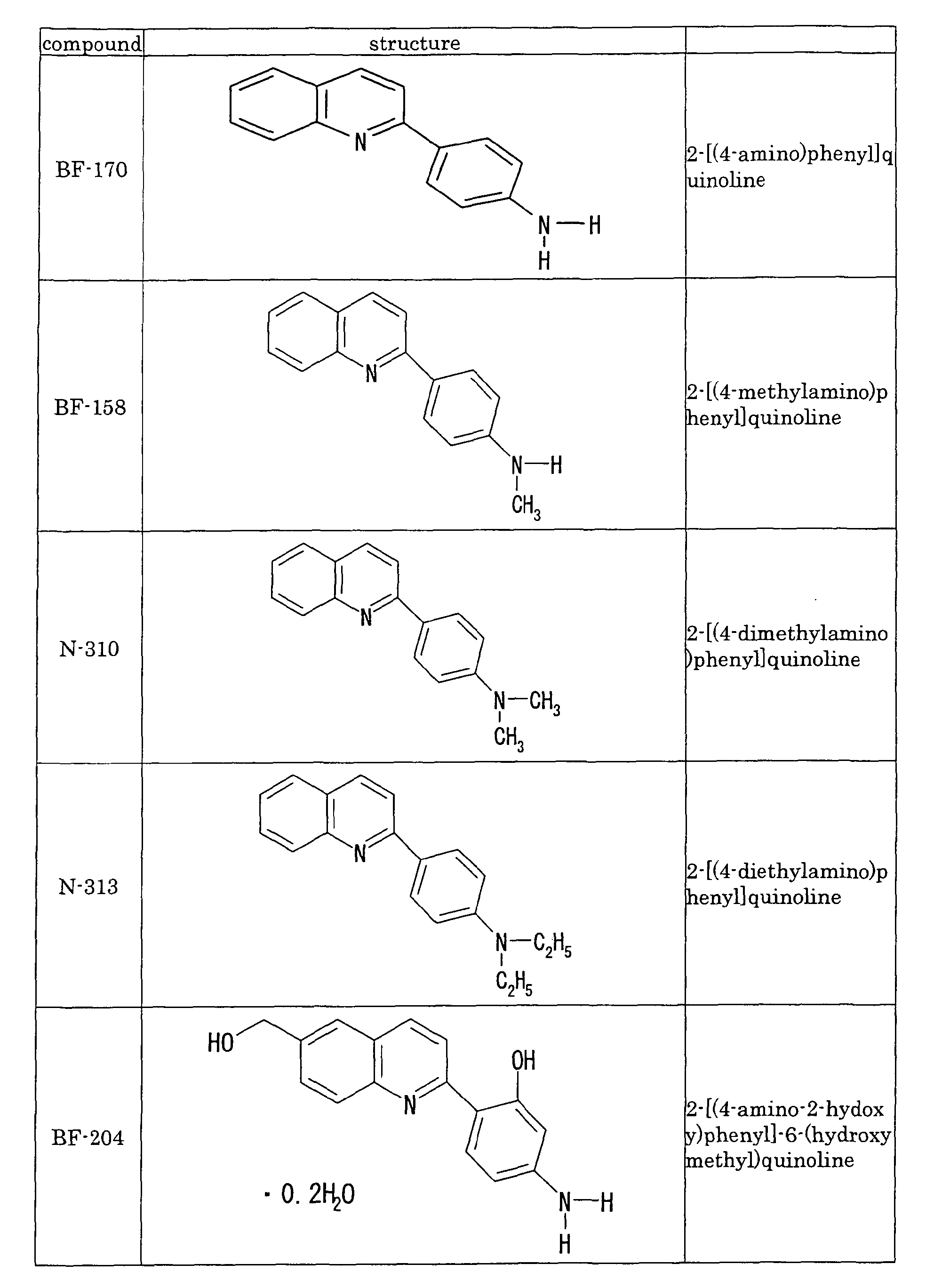

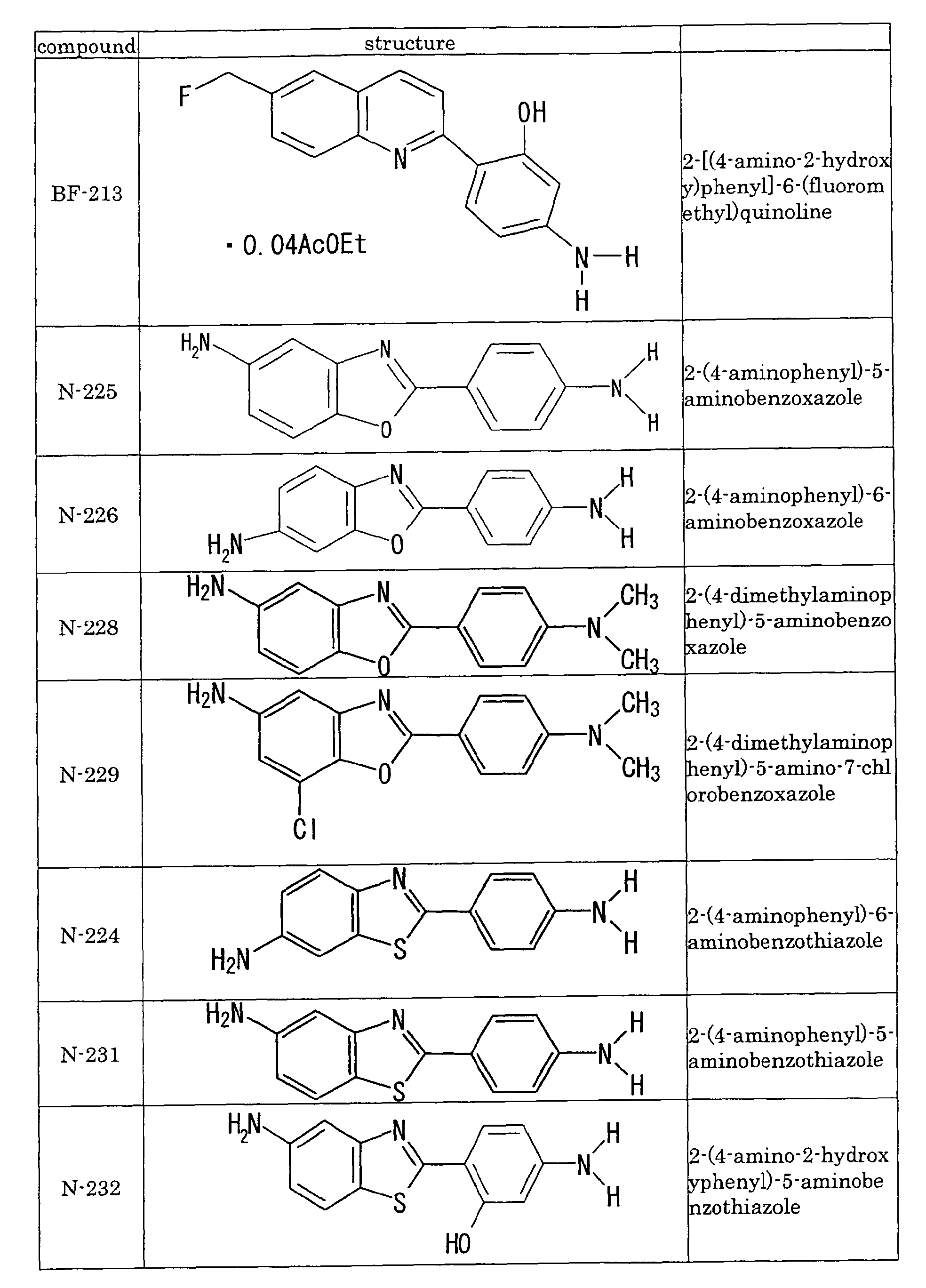

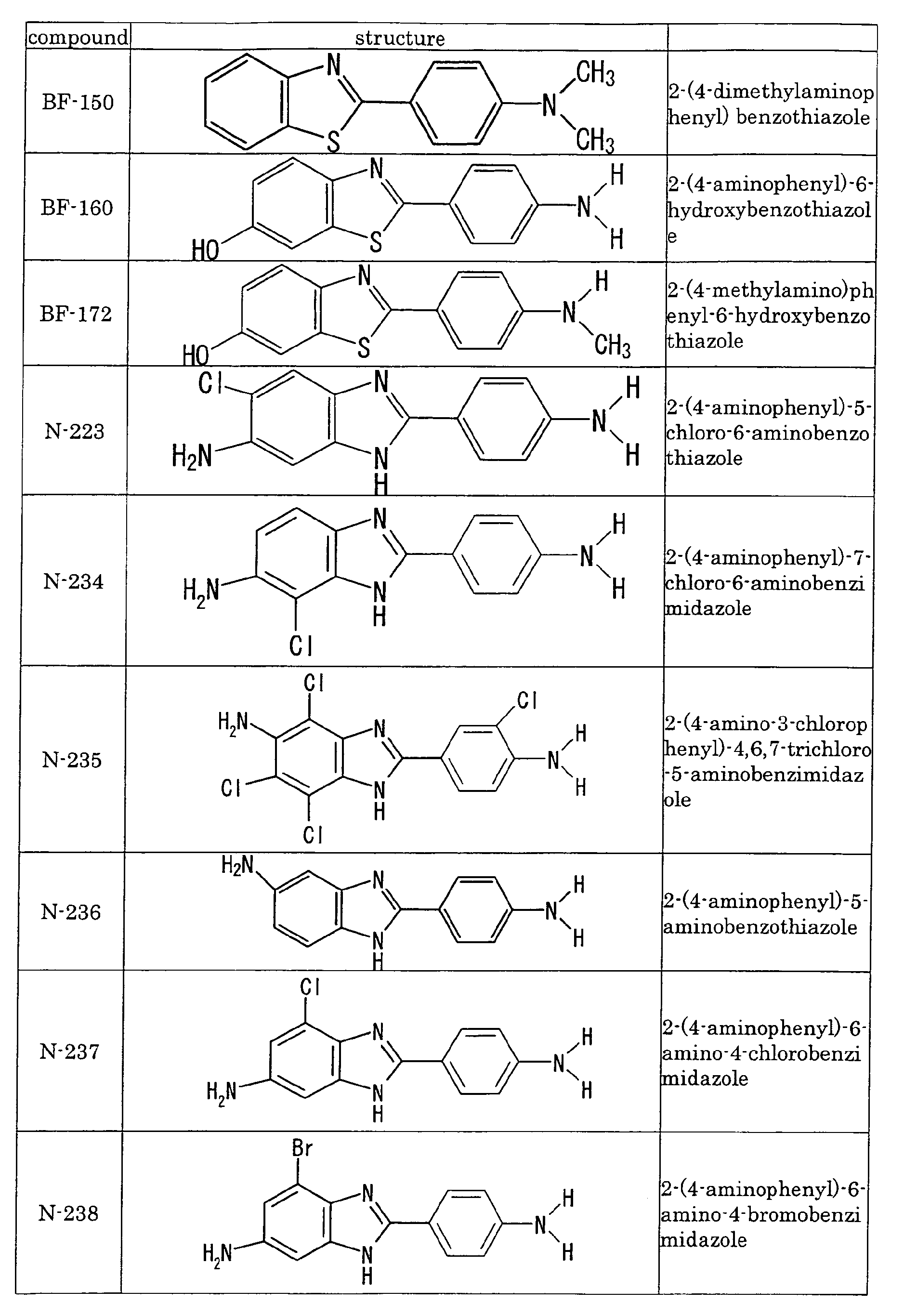

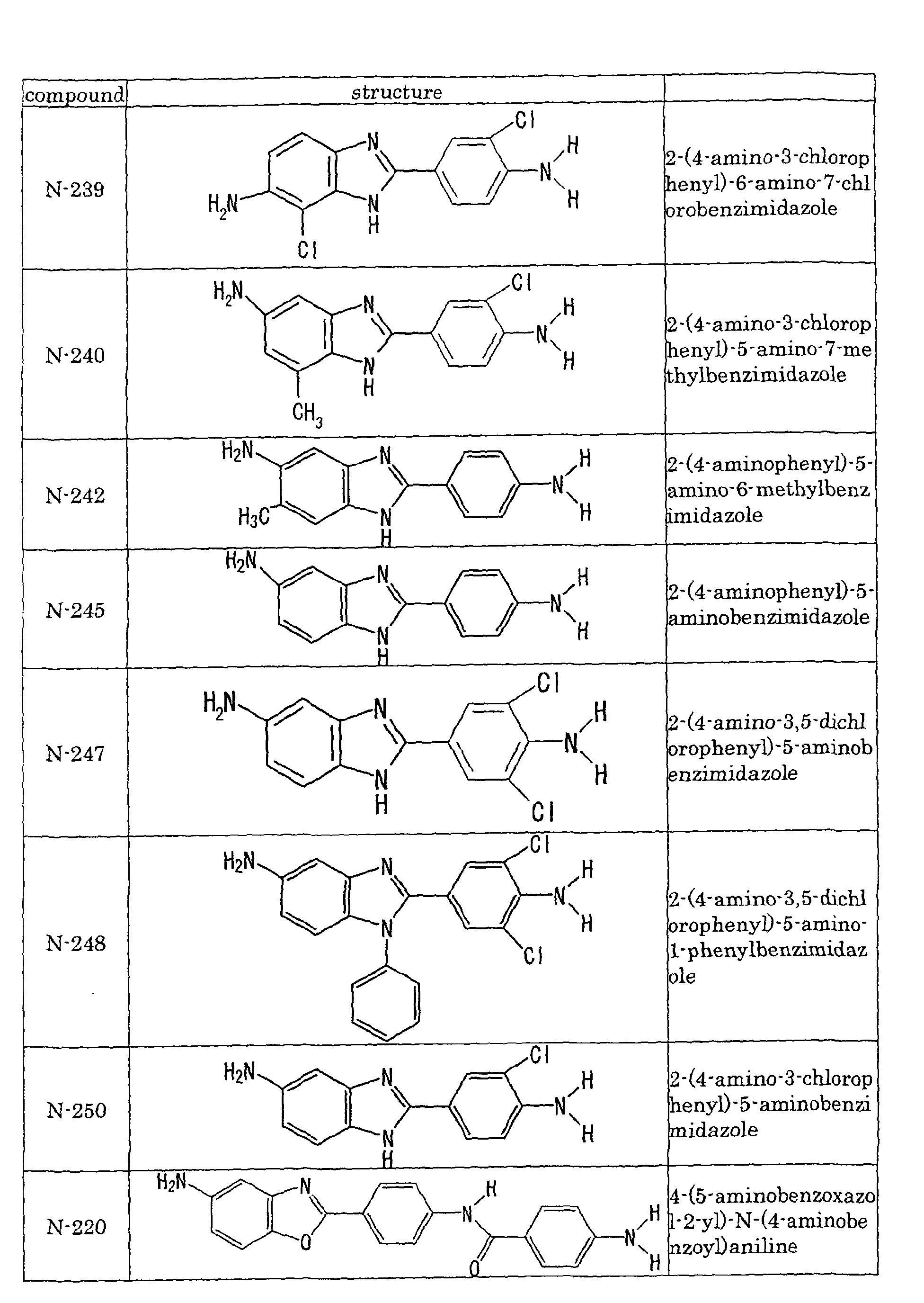

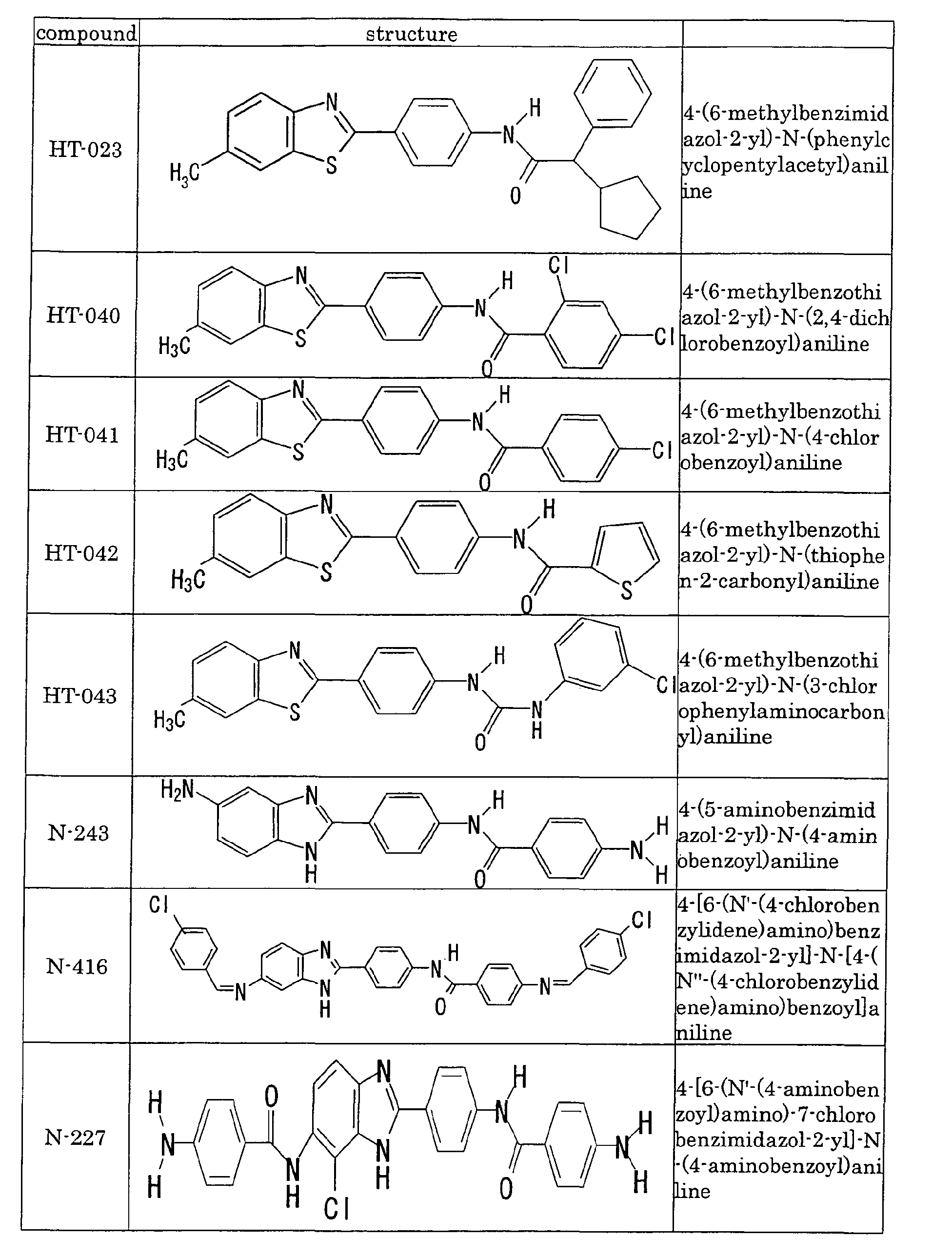

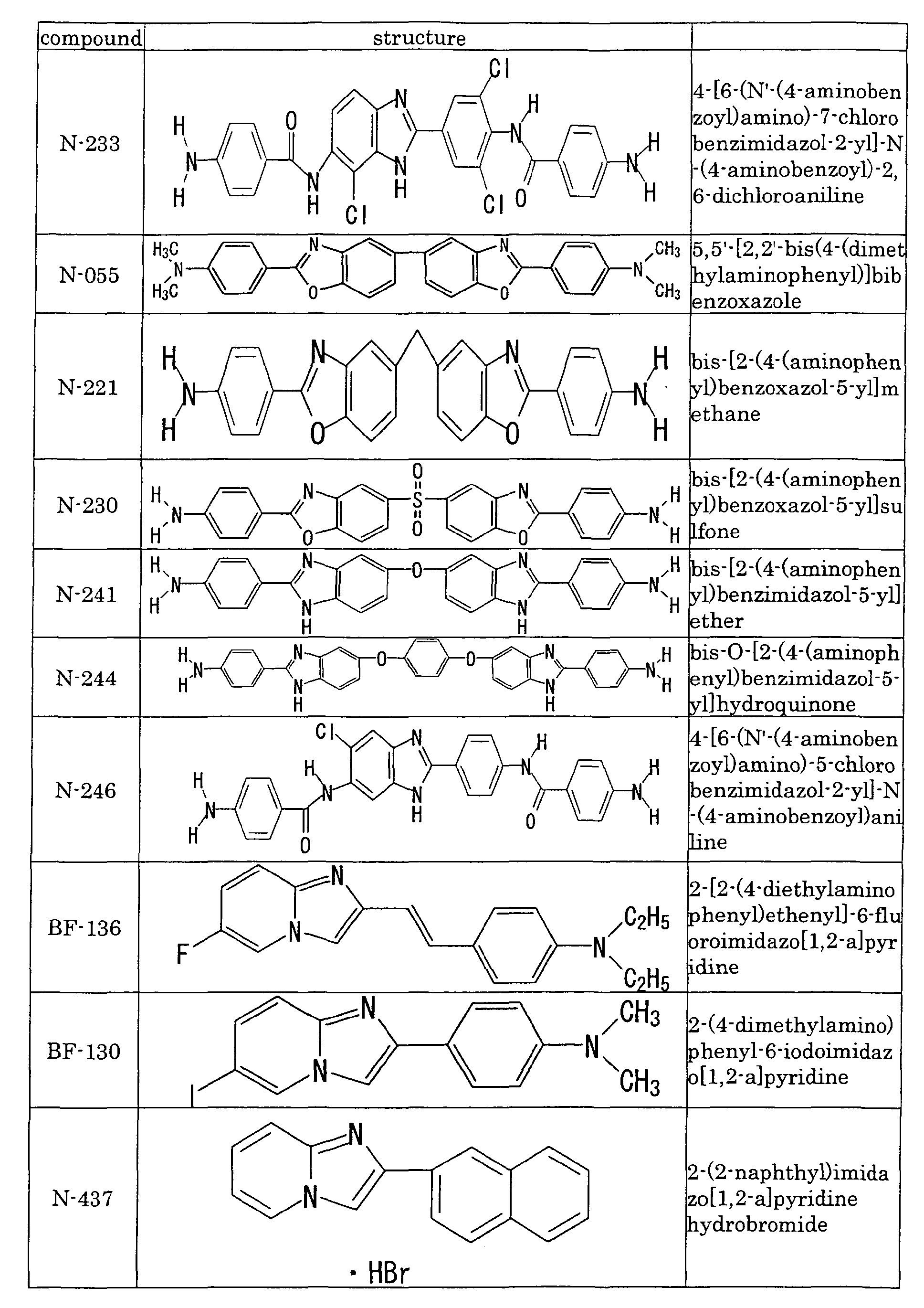

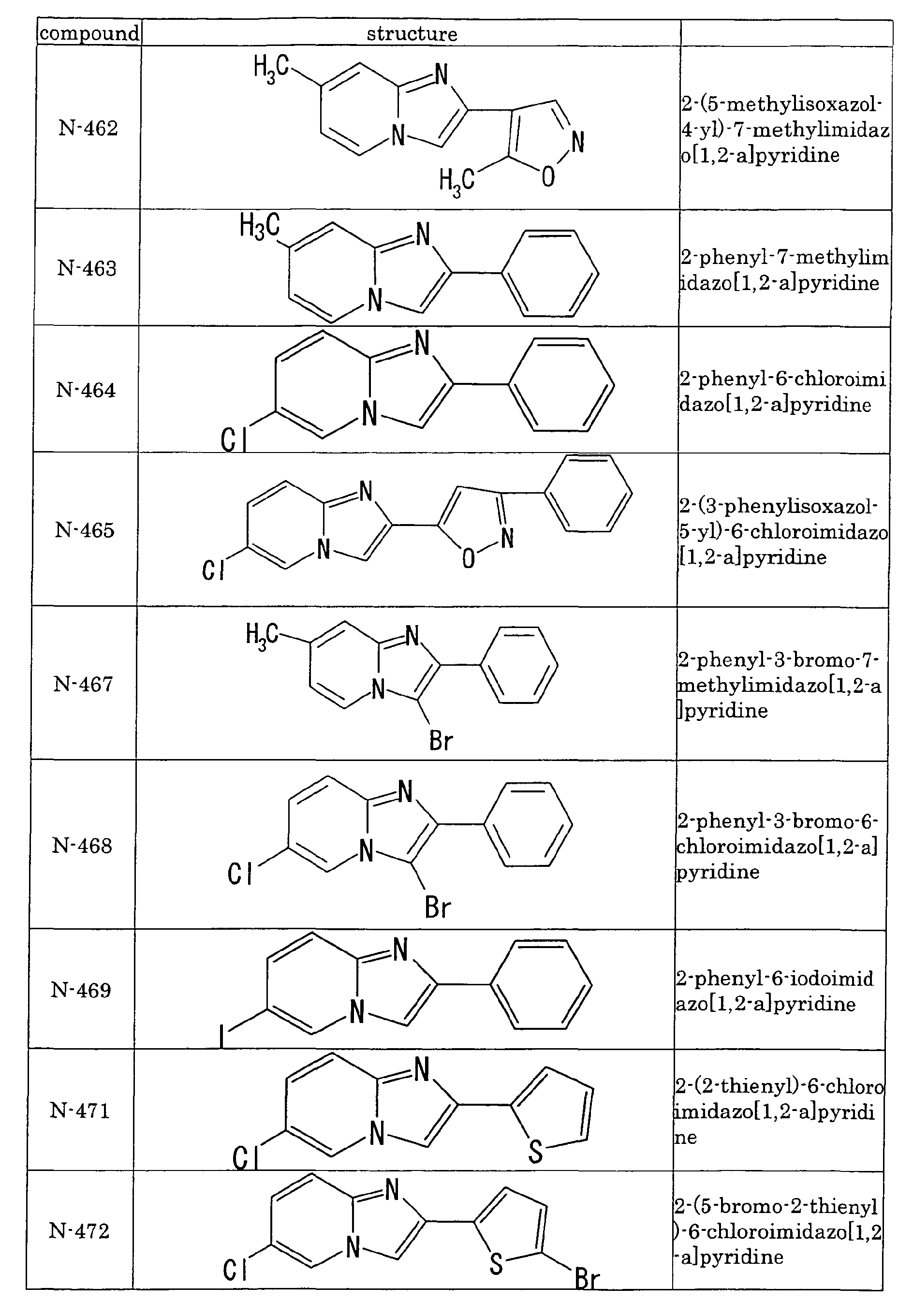

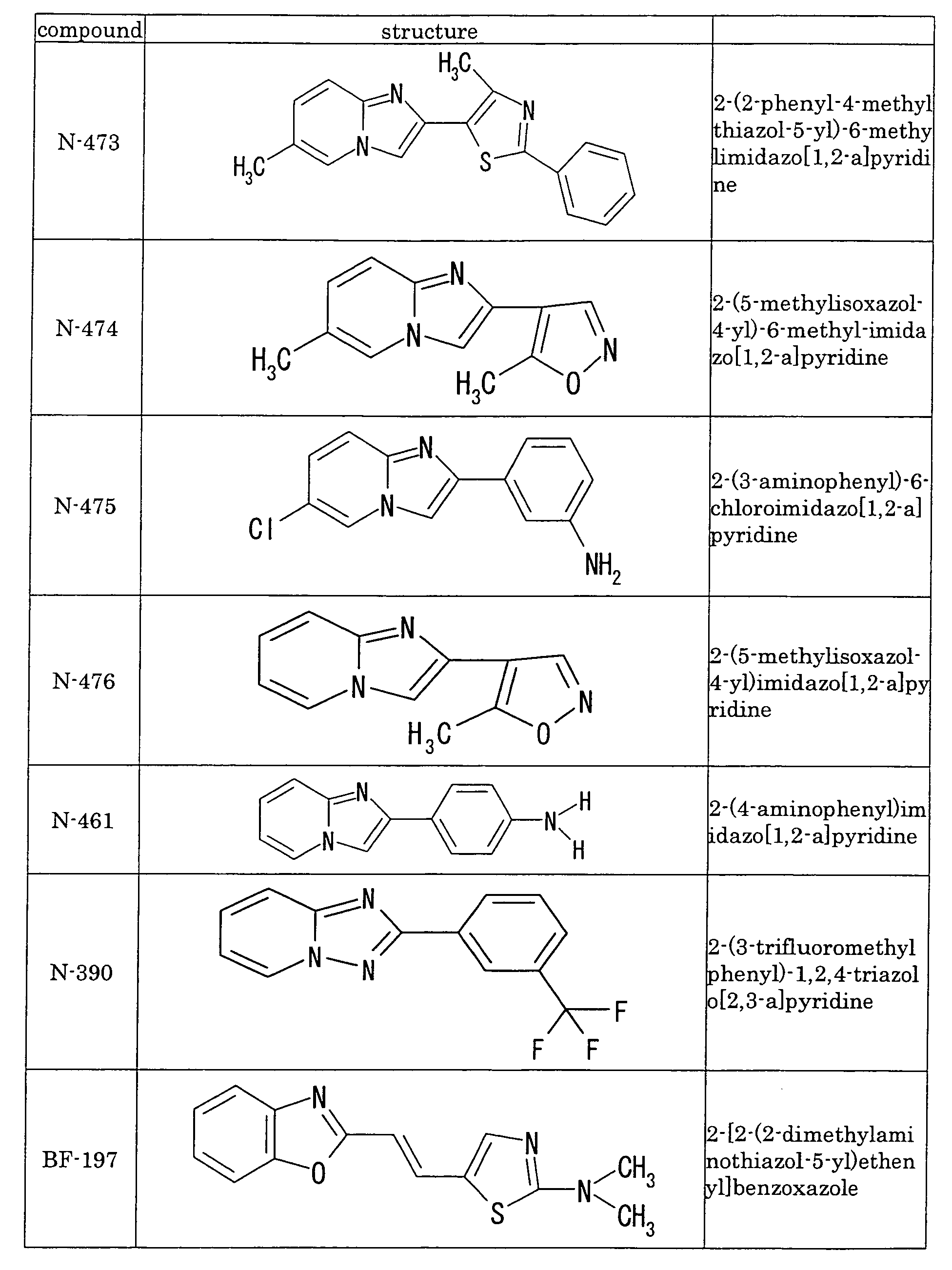

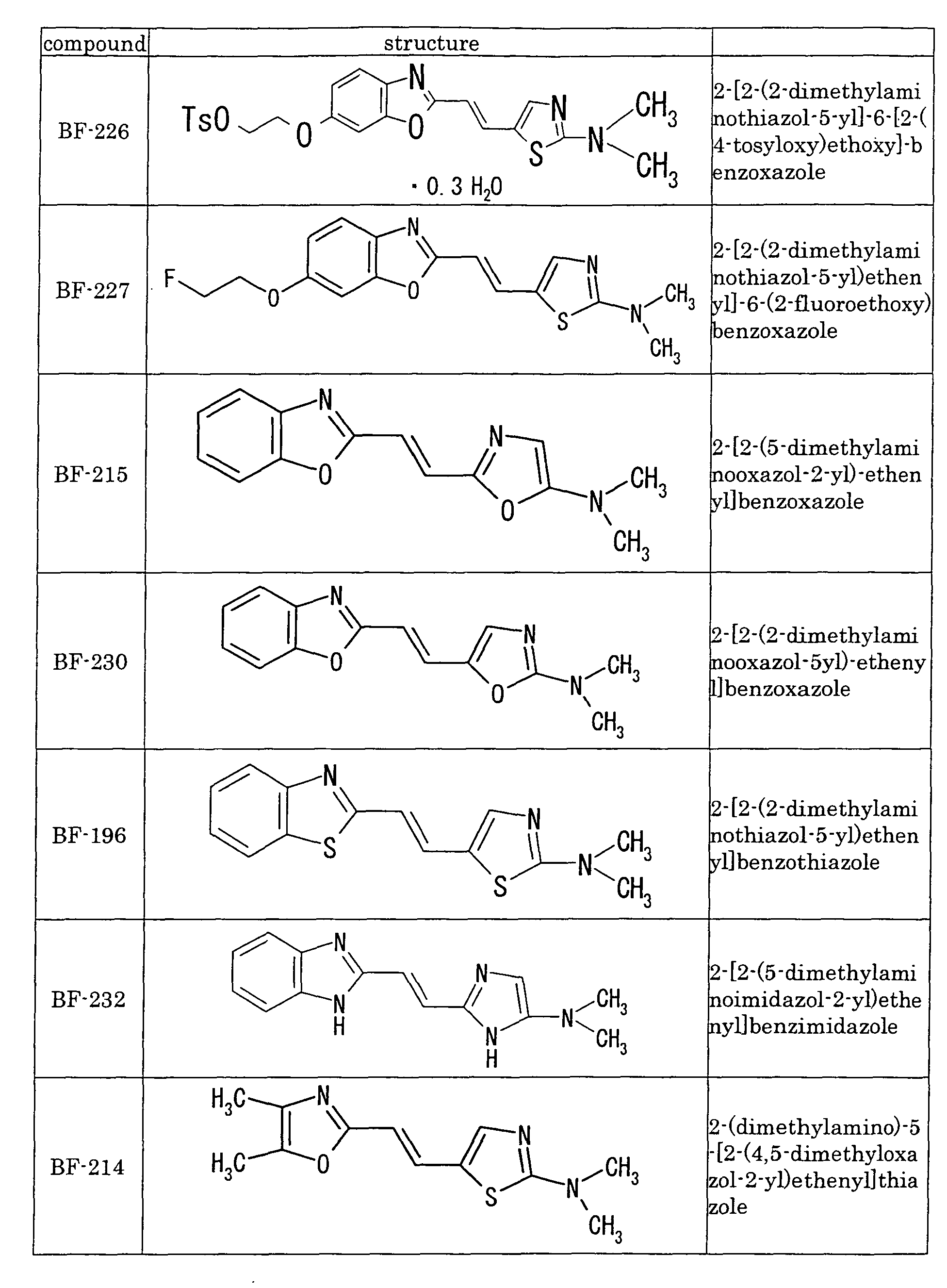

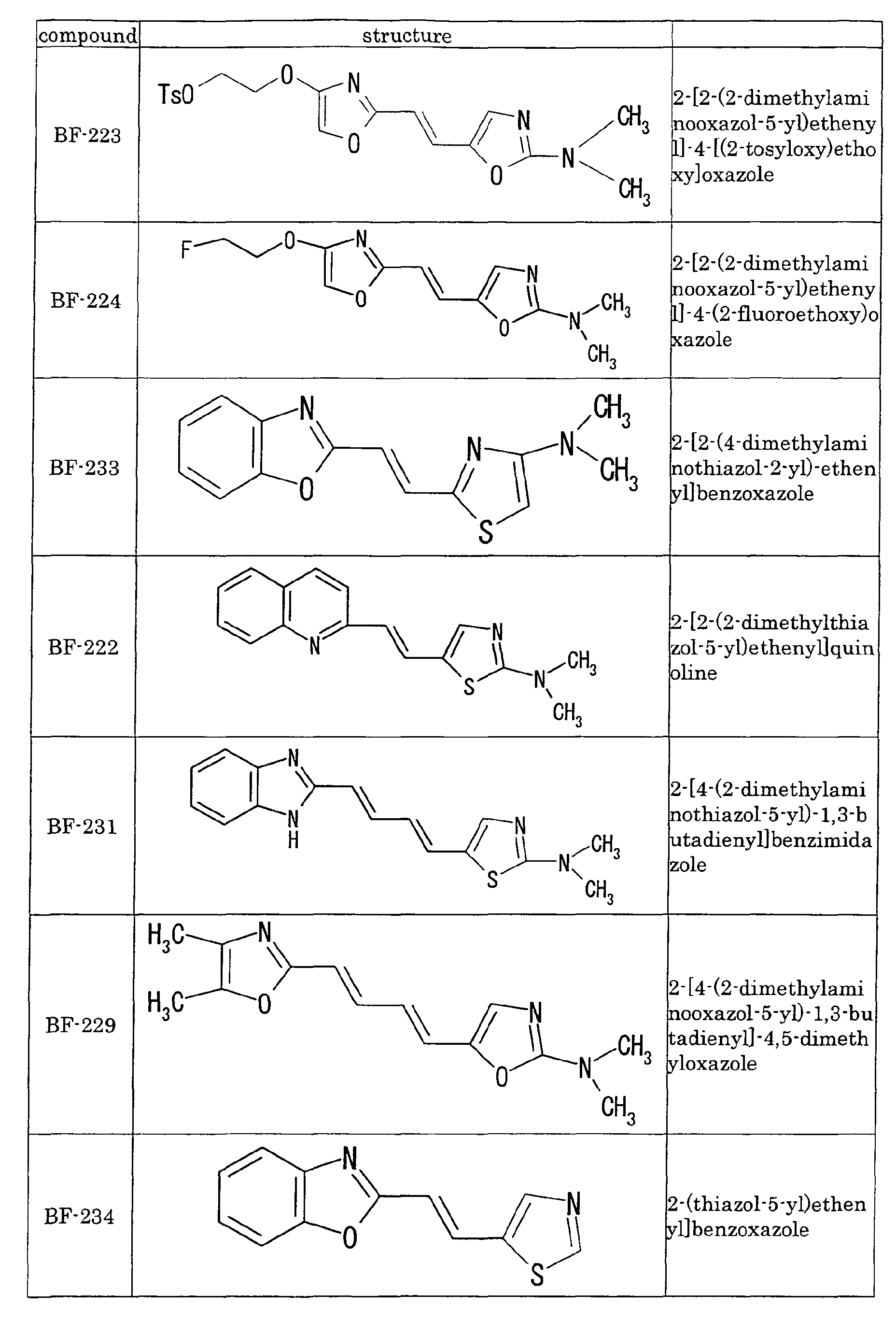

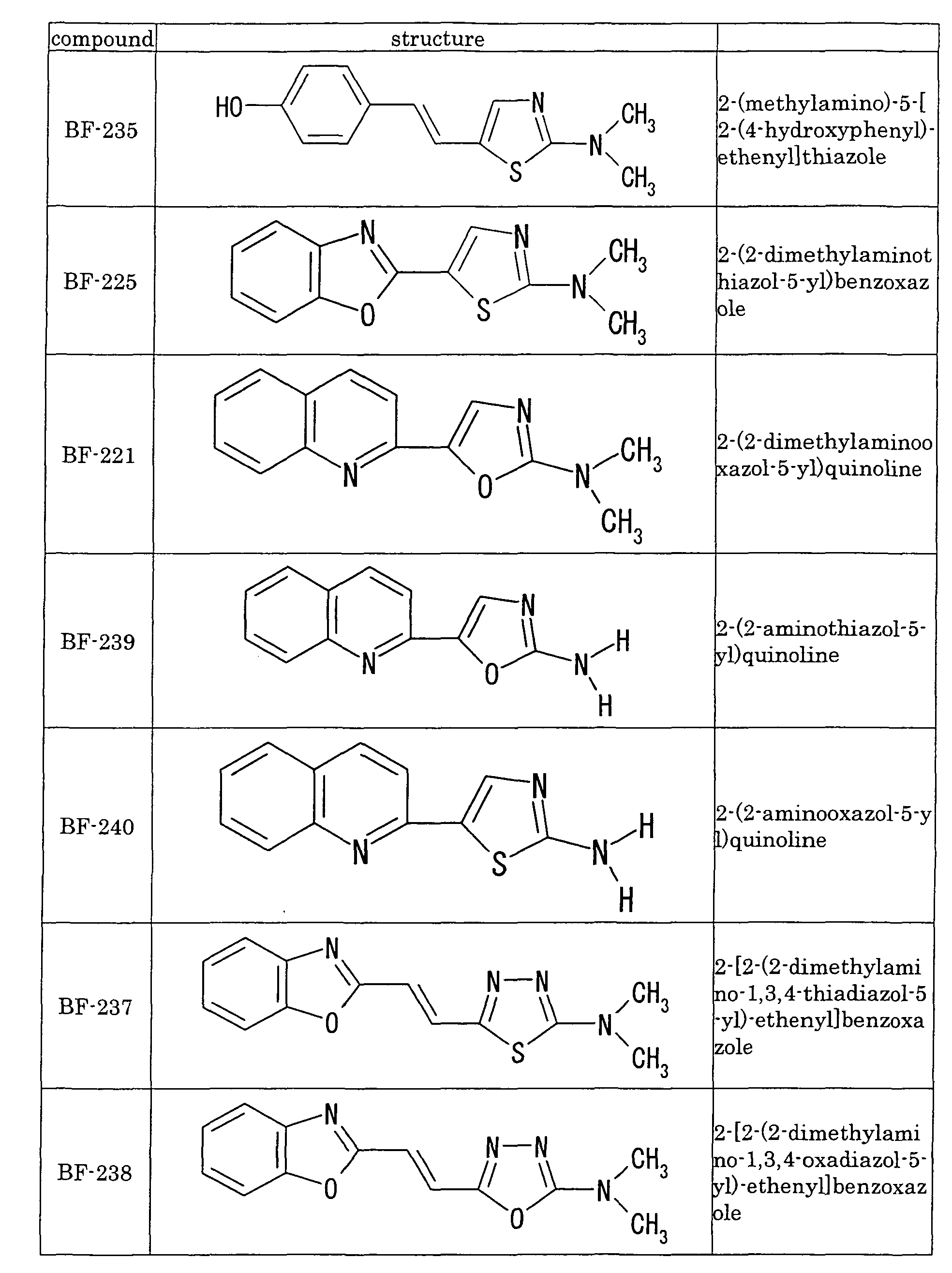

According to the present invention, as probes for the imaging diagnosis of diseases in which prion protein is accumulated, compounds of the formula (I) or (II), or salts or solvates thereof is used, which have been labeled and which specifically bind to abnormal prion protein in vivo in individuals having diseases in which prion protein is accumulated. As illustrated below in the Examples, the compounds of the present invention allow clear staining of abnormal prion proteins in the living body. As referred to herein, a "disease having accumulated prion protein" refers to an illness having the accumulation of prion protein in the brain as the main sign. Diseases which can be diagnosed using prion protein as marker include, for example, in humans, Creutzfeldt-Jacobdisease (CJD), Gerstmann-Sträussler-Scheinker disease (GSS), variant Creutzfeldt-Jacob disease (vCJD), fatal familial insomnia (FFI), kuru, and in non-human animals, sheep scrapie, bovine spongiform encephalopathy (BSE), transmissible mink encephalopathy, feline spongiform encephalopathy, and the like. These diseases may be collectively referred herein to as prion diseases.

As described above, it has turned out that neuronal degeneration characteristic to prion diseases has already taken place much earlier than when their initial clinical symptoms appear. It is believed that accumulating of prion protein takes place much earlier than the onset of the prion diseases. Therefore, early detection of accumulated prion protein will make it possible to early discover and diagnose prion diseases.

Thus, compositions of the present invention for the diagnosis of prion diseases which comprise compounds represented by the formula (I) or (II), or pharmaceutically acceptable salts or solvates thereof, are useful for early discovery and diagnosis thereof.

In addition, the present invention relates to a method for the detection of individuals having accumulated prion protein in the living body, characterized by obtaining samples from a subject animal, and contacting to the samples a compound of the present invention, or salt or solvate thereof. Subject animals include mammals, such as bovines, sheep, goats, cats, monkeys, and others, and humans are also included in the subject animals. Living-body samples which can be obtained from subject animals may be of any kind, with both biopsies and autopsies being possible. Samples generally utilize brain and spinal cord samples, and may be body fluids such as urine, blood, and others. Usually, samples obtained from a living body are contacted with a compound of the present invention, followed by detection, observation, or identification of binding of prion protein in the samples and the compounds of the present invention by an appropriate means, for example, microscopy. Those skilled in the art can readily select kinds of samples, methods for obtaining samples and for contacting the samples with compounds of the present invention, and the like, depending upon the purpose. In an alternative, in the case of labeled compounds, identification can be made by appropriate means, for example, fluorometers, measurements of enzyme reactions, scintillation counters, and the like. Labels are fluorescent substances, affinity substances, enzyme substrates, radionuclides, and others. These labels, methods for attaching to the compounds, as well as means and methods for detection are well known in the art.

Therefore, the compounds of the present invention can be used as reagents for the in vitro diagnosis of prion diseases. The compounds of the present invention bind to abnormal prion proteins, and thus are also applicable as staining agents and in vitro diagnostic reagents for prion diseases in humans and animals. The use of the compounds of the present invention allows easier diagnosis of prion diseases, whose definite diagnosis have been made by confirming abnormal prion proteins through immunostaining and Western blotting.

For example, in conventional techniques, confirmation of abnormal prion proteins of bovine spongiform encephalopathy requires identification of the prion protein by ELISA methods as primary screening and reexamination of the primary screening and by Western blotting methods as an identification test of the secondary testing and immunohistological examinations of tissue sections as a second identification test of the secondary testing, whereas staining or determining of brain sections or brain homogenates with the compounds of the present invention enable one to confirm abnormal prion proteins easier and for a shorter time to diagnose prion diseases. In addition, prion diseases can be diagnosed by confirming prion abnormal protein in lymphoid tissues, urine, and/or blood using the compounds of the present invention. Further, the compounds of the present invention can be used to confirm abnormal prion proteins in bovine-derived foods, medical preparations (for example, gelatin capsules), cosmetics (for example, collagen), and others.

Therefore, the present invention provides a method for the in vitro diagnosis of an individual with a disease having accumulated prion protein in the living body, the method characterized by contacting a compound of the present invention, or pharmaceutically acceptable salt or solvate thereof to samples obtained from subject animals or products derived from animals which are suspected to be affected with prion diseases (for example, bovine spongiform encephalopathy), as well as an in vitro diagnostic composition for the use in such an in vitro diagnostic method, the composition comprising a compound of the present invention, and an in vitro diagnostic kit for the use in such an in vitro diagnostic method, the kit comprising as the essential ingredient a compound of the present invention. In these cases, the compounds of the present invention may be unlabeled or labeled, and its salts or solvates may be pharmaceutically unacceptable, since the samples are removed from the subject and then stained. Preferred compounds to be used in such an in vitro diagnosis include BF-168, BF-191, BF-192, BF-196, BF-197, BF-198, BF-200, BF-201, BF-203, BF-206, BF-208, BF-227, BF-228, N-278, and the like.

Further, the present invention relates to a method for the diagnosis of diseases in which prion protein is accumulated, characterized by using a compound of the present invention, or pharmaceutically acceptable salt or solvate thereof. The method is carried out by obtaining samples from subjects (for example, brain samples), contacting to the samples a compound of the present invention, and detecting binding of prion protein in the samples and the compound of the present invention by an appropriate means (for example, microscopy). In addition, the present invention also relates to a method for the imaging diagnosis of diseases in which prion protein is accumulated, characterized by using a compound of the present invention, or pharmaceutically acceptable salt or solvate thereof, wherein the compound is radioactively labeled. It is also possible that the compound of the present invention is administered into the body of subjects, followed by acquiring images, at a specified time, non-invasively on an instrument such as PET or the like as described above. Those skilled in the art can appropriately select types of samples in these procedures, methods for obtaining samples, contacting the samples with a compound of the present invention, detecting binding of prion protein and the compound of the present invention, or administering the compounds of the present invention to subjects, dosages, instruments and methods for imaging diagnosis, and others, so as to carry out the present invention.

It is common that in vivo diagnosis of diseases in which prion protein is accumulated employs labeled compounds of the present invention as diagnostic probes. Usually, imaging diagnosis of diseases in which prion protein is accumulated uses probes which have been labeled with radionuclides. Compounds of the present invention can be labeled with a variety of radionuclides by methods well known in the art. For example, ³H, ¹⁴C, ³⁵S, ¹³¹I, and others are radionuclides conventionally used and have many in vivo applications. General requirements for probes for imaging diagnosis and means for detecting the probes are to permit in vivo diagnosis, to cause less damage to patients (especially, to be non-invasive), to have high sensitivity of detection, to have an appropriate half-life (to provide an appropriate period of time for preparing labeled probes and for diagnosis), and the like. Accordingly, one have recently tended to employ positron emission tomography (PET) utilizing γ-ray displaying high sensitivity and permeability of materials or computered tomography based on γ-ray emitting nuclides (SPECT). Of them, PET, which detects two γ-rays emitting in opposite directions form a positron emitting nuclide by means of simultaneous counting with a pair of detectors, provides information which is superior in resolution and quantification and thus is preferable. For SPECT, compounds of the present invention can be labeled with γ-ray emitting nuclides such as ^{99m}Tc, ¹¹¹In, ⁶⁷Ga, ²⁰¹Tl, ¹²³I, ¹³³Xe, and others. ^{99m}Tc and ¹²³I are often used for SPECT. For PET, compounds of the present invention can be labeled with positron emitting nuclides such as ¹¹C, ¹³N, ¹⁵O, ¹⁸F, ⁶²Cu, ⁶⁸Ga, ⁷⁶Br, and others. Of positron emitting nuclides, ¹¹C, ¹³N, ¹⁵O and ¹⁸F are preferable, from the viewpoint of having an appropriate half-life, the ease of labeling, and the like. ¹⁸F is particularly preferable. The position at which a compound of the present invention is labeled with a radiation emission nuclide such as a positron or γ-ray emitting nuclide, or the like can be any position in the formula (I) or (II). For example, a hydrogen atom on the benzene ring of a compound of the present invention may be substituted with a positron emitting nuclide such as ¹⁸F, or alternatively one or more of the carbon atoms constituting the structure of a compound of the present invention may be ¹¹C. Also, when a compound of the present invention is labeled with ¹⁸F, for example, ¹⁸F may be contained anywhere in the side chain, or a substituent on the ring of the compound may be ¹⁸F itself. For example, a substituent R₁ on the benzene-ring portion of an oxazoline ring may be ¹⁸F. Those skilled in the art can appropriately determine which position a label is attached at, and readily synthesize such labeled compounds. Such labeled compounds of the formula (I) or (II) are also included in the present invention.

Also included in the present invention are precursors for producing labeled materials of the compounds represented by the formula (I) or (II) (herein referred to as "labeled precursors"). Labeled precursors are varied, depending upon the structure of compounds to be labeled, labels used, and others. For example, preferred compounds of the present invention which are to be labeled with ¹⁸F include BF-168, BF-224, N-227, and others, and in those cases, the labeled precursors are preferably tosylate derivatives. Preferred tosylate derivatives of BF-168, BF-224, and N-227 are BF-167, BF-223, and N-226, respectively (see, Table 1).

In general, these nuclides are generated on an instrument termed cyclotron or generator. Those skilled in the art can select methods and instruments for production, depending upon nuclides to be produced. Nuclides thus produced can be used to label the compounds of the present invention.

Methods for producing labeled compounds, which have been labeled with these radionuclides, are well known in the art. Typical methods include chemical synthesis, isotope exchanging, and biosynthesis processes. Chemical synthesis processes have been conventionally and widely employed, and are essentially the same as usual chemical synthesis processes, except that radioactive starting materials are used. Various nuclides are introduced into compounds by chemical processes. Isotope exchanging processes are processes by which ³H, ³⁵S, ¹²⁵I, and the like contained in compounds of simple structures are transferred into ones of more complex structures, thereby obtaining compounds that have been labeled with these nuclides and possess more complex structures. Biosynthese processes are processes by which compounds labeled with ¹⁴C, ³⁵S, and the like are given to microbial cells or others to obtain their metabolites having these nuclides introduced therein.

With respect to the labeling position, similarly to usual synthesis, synthetic schemes can be designed, depending upon the purpose, so that a label can be introduced at a desired position. Such designing is well known to those skilled in the art.

When utilizing positron emitting nuclides, such as ¹¹C, ¹³N, ¹⁵O, and ¹⁸F, which have relatively short half-lives, it is also possible to generate a desired nuclide on a (super) small-sized cyclotron placed in a facility of hospitals or the like, which in turn is used to label a desired compound at its desired position by any one of the above-described methods, followed by carrying out immediately diagnosis, examination, treatment, or the like.

These methods well known to those skilled in art enable one to carry out labeling by introducing a desired nuclide into a compound of the present invention at its desired position.

Upon imaging diagnosis, labeled compounds of the present invention may be administered to subjects locally or systemically. Routes for administration include intradermal, intraperitoneal, intravenous, intra-arterial injections or infusions, injections or infusions into the spinal fluid, and others, and can be selected, depending upon factors such as types of diseases, nuclides used, compounds used, condition of a subject, sites to be examined, and others. Sites to be examined can be investigated with means such as PET, SPECT, or the like by administering a probe of the present invention, followed by the elapse of a sufficient time to allow its binding to abnormal prion protein and decay. These procedures can be selected as appropriate, depending upon factors such as types of diseases, nuclides used, compounds used, condition of a subject, sites to be examined, and others.

The dosage of compounds of the present invention labeled with radionuclides varies, depending upon types of diseases, nuclides used, compounds used, age, physical condition, and gender of a subject, degrees of diseases, sites to be examined, and others. In particular, sufficient care has to be taken of the exposure dose to subjects. For example, the radioactivity of compounds of the present invention labeled with positron emitting nuclides such as ¹¹C, ¹³N, ¹⁵O, ¹⁸F, and others ranges from 3.7 megabecquerel to 3.7 gigabecquerel, and preferably from 18 megabecquerels to 740 megabecquerels.

The present invention also provides a composition for the imaging diagnosis of diseases in which prion protein is accumulated, the composition comprising a compound of the present invention. The composition comprises a compound of the present invention and a pharmaceutically acceptable carrier. Preferably,the compounds of the present invention in the composition is labeled. Although a variety of labeling methods is possible as described above, labeling with radionuclides (in particular, positron emitting nuclides such as ¹¹C, ¹³N, ¹⁵O, ¹⁸F, and others) is desirable for in vivo image-diagnosis applications. It is preferable from their purposes that forms of the compositions of the present invention are ones allowing injection or infusion. Therefore, pharmaceutically acceptable carriers are preferably liquids and include, but not limiting to, aqueous mediums such as potassium phosphate buffer, saline, Ringer's solution, distilled water, and others, or non-aqueous mediums such as polyethylene glycols, vegetable oils, ethanol, glycerin, dimethyl sulfoxide, propylene glycols, and others. The ratio of formulation of a carrier and a compound of the present invention can be selected as appropriate, depending upon sites to be applied, means for detection, and the like, and the ratio usually ranges from 100,000:1 to 2:1, preferably from 10,000:1 to 10:1. Additionally, the compositions of the present invention may further contain well-known antimicrobials (for example, antibiotics etc.), local anesthetics (for example, procaine hydrochloride, dibucaine hydrochloride, etc.), buffers (for example, Tris-HCl buffer, HEPES buffer, etc.), osmoregulatory agents (for example, glucose, sorbitol, sodium chloride, etc.), and the like.

Further, the present invention provides a kit for the diagnosis of diseases in which prion protein is accumulated, comprising a compound of the present invention as the essential ingredient. Usually, the kit is a package in which components such as a compound of the present invention, solvent for dissolving it, buffer, osmoregulatory agent, antimicrobial, local anesthetic, and the like are each packaged separately into respective containers, or some of the components are packaged together into respective containers. The compounds of the present invention may be unlabeled or labeled. When not labeled, the compounds of the present invention can be labeled, prior to use, by usual methods as described above. In addition, the compounds of the present invention may be presented in solid, such as lyophilized powder, or in solutions in appropriate solvents. Solvents may be similar to carriers used in the above-mentioned compositions of the present invention. Components such as a buffer, an osmoregulatory agent, an antimicrobial, a local anesthetic, and the like, also may be similar to those used in the above-mentioned compositions of the present invention. While containers can be selected as appropriate, they may be of shapes suitable for carrying out the introduction of a label into a compound of the present invention, or of light-shielding materials, depending upon the nature of compounds, or take forms such as vials or syringes, so as to be convenient for administration to patients. The kit may also contains, as appropriate, tools necessary for diagnosis, for example, syringes, a set for infusion, or in the case of the compounds of the present invention labeled, for example, with positron emitting nuclides, apparatus for use in a PET instrument. An instruction is usually attached to the kit.

Preferred compounds of the present invention to be used as probes for PET and SPECT as described above include labeled materials, in general, radioactively labeled materials, such as BF-124, BF-148, BF-165, BF-168, BF-191, BF-192, BF-196, BF-197, BF-198, BF-200, BF-201, BF-203, BF-206, BF-208, BF-227, BF-228, N-276, N-277, and N-313, and preferably the above-described compounds labeled with y-ray emitting nuclides (for example, ^{99m}Tc, ¹²³I) or positron emitting nuclides (for example, ¹⁸F) (methods for labeling compounds and the labeling position are as explained above).

Further, the compounds of the present invention have properties of binding specifically to abnormal prion protein, and thus can be also used as agents for specifically staining abnormal prion protein contained in samples such as brain samples. Therefore, the present invention provides a composition for specifically staining abnormal prion protein in samples, comprising a compound of the present invention, or salt or solvate thereof. In addition, the present invention provides a kit for specifically staining abnormal prion protein in samples, comprising a compound of the present invention, or salt or solvate thereof as the essential ingredient. In these cases, the compounds of the present invention may be unlabeled or labeled, and its salts or solvates may be pharmaceutically unacceptable, since samples are removed from subjects and then stained. In addition, an instruction is usually attached to the kit. The present invention relates to a method for specifically staining abnormal prion protein in samples, characterized by using a compound of the present invention, or salt or solvate thereof. Conditions for staining abnormal prion protein in samples using these staining compositions, kits, or methods of the present invention are those which can be selected as appropriate and under which staining can be carried out with ease, by those skilled in the art. Preferable compounds of the present invention to be used as such staining agents include BF-124, BF-125, BF-126, BF-133, BF-136, BF-142, BF-143, BF-147, BF-148, BF-150, BF-151, BF-154, BF-160, BF-162, BF-165, BF-168, BF-172, BF-180, BF-191, BF-192, BF-196, BF-197, BF-198, BF-200, BF-201, BF-203, BF-206, BF-208, BF-225, BF-227, BF-228, N-227, N-228, N-276, N-282, N-283, and N-407.

Further, as mentioned above, the compounds of the present invention are specific for abnormal prion proteins, and thus believed to suppress the cytotoxicity of abnormal prion proteins or the production of abnormal prion protein by cells.

Therefore, the present invention relates to a pharmaceutical composition for the prophylaxis and/or treatment of diseases in which the accumulation of prion protein in the body constitutes or partially constitutes the etiology, for example, prion diseases such as, in humans, Creutzfeldt-Jacob disease (CJD), Gerstmann-Sträussler-Scheinker disease (GSS), vatriant Creutzfeldt-Jacob disease (vCJD), fatal familial insomnia (FFI), kuru, and in non-human animals, sheep scrapie, bovine spongiform encephalopathy (BSE), transmissible mink encephalopathy, feline spongiform encephalopathy, and the like, the composition comprising a compound of the present invention, or pharmaceutically acceptable salt or solvate thereof, and a pharmaceutically acceptable carrier.

Formulated forms of such compositions are varied, and liquid formulations, in particular, formulations for injection, are preferable. Such formulations for injection may be also injected directly into the brain. Alternatively, pharmaceutical compositions described above may be formulated for intravenous injection or infusion and subjected to administration, since the compounds of the present invention have enhanced blood-brain barrier permeability, as illustrated below in Examples. Such liquid formulations can be prepared in methods well known in the art. Solutions can be prepared by dissolving a compound of the present invention in an appropriate carrier, water for injection, saline, Ringer's solution, or the like, sterilizing the solution through a filter or the like, and filling the sterilized solution into appropriate containers, for example, vials or ampules. Solutions also can be lyophilized and when used, re-constituted with an appropriate carrier. Suspensions can be prepared by sterilizing a compound of the present invention, for example, through exposure to ethylene oxide, and then suspending it in a sterilized suspending liquid carrier. Methods for preparing such formulations and other methods are well known in the art.

Doses of the compounds of the present invention depend on condition, sex, age, weight of a patients, and the like, and in general the dosage ranges from 0.1 mg to 1 g, preferably from 1 mg to 100 mg, more preferably from 5 mg to 50 mg, per day for adult humans weighing 70 kg. It is possible to conduct treatment with such a dosage for a specified period of time, followed by increasing or reducing the dosage according to the outcome.

Further, the present invention relates to a method for the prophylaxis and/or treatment of diseases in which the accumulation of prion protein in the body constitutes or partially constitutes the etiology, characterizing by administering to a subject a compound of the present invention, or pharmaceutically acceptable salt or solvate thereof, as well as to use of a compound of the present invention for the treatment and/or prophylaxis of such diseases. Such diseases include diseases described above, and preferable diseases for the treatment and/or prophylaxis by the method of the present invention include transmissible spongiform encephalopathy or prion diseases.

Doses and methods for administering the compounds of the present invention in such treatment and/or prophylaxis methods, and others are as described above for the pharmaceutical composition for the treatment and/or prophylaxis of diseases in which prion protein is accumulated. Compounds of the present invention to be used for such treatment and/or prophylaxis may be unlabeled, but radioactively labeled, for example, in order to facilitate the confirming of delivery to sites to be treated. Subjects for such treatment and/or prophylaxis are animals which may be contaminated or affected with prion protein and include, in particular, bovines and humans.

Further, the present invention relates to use of a compound of the present invention, or pharmaceutically acceptable salt or solvate thereof for the prophylaxis and/or treatment of diseases in which the accumulation of prion protein in the body constitutes or partially constitutes the etiology, in particular, transmissible spongiform encephalopathy or prion diseases, as well as to use of a compound of the present invention for manufacturing an medicament for the prophylaxis and/or treatment of diseases in which the accumulation of prion protein in the body constitutes or partially constitutes the etiology, in particular, transmissible spongiform encephalopathy or prion diseases.

Preferred compounds of the present invention for the treatment and/or prophylaxis of diseases in which the accumulation of prion protein in the body constitutes or partially constitutes the etiology, as described above, include BF-130, BF-135, BF-136, BF-141, BF-146, BF-148, BF-150, BF-153, BF-168, N-220, N-221, N-223, N-224, N-232, N-243, N-246, N-407, N-437, N-441, N-453, N-457, BF-192, BF-193, BF-198, BF-199, BF-201, BF-203, BF-204, BF-206, BF-208, BF-211, BF-213, BF-227, and BF-231. Taking into account anti-prion effects, TC and the safety concentration margin, mutagenesis, or the like, more preferable compounds of the present invention for the treatment and/or prophylaxis of diseases in which the accumulation of prion protein in the body constitutes or partially constitutes the etiology, as described above, include BF-130, BF-135, BF-146, N-407, N-437, N-441, N-453, N-457, BF-208, BF-227, BF-231, BF-192, BF-193, BF-198, BF-199, BF-201, BF-203, BF-204, BF-206, BF-208, BF-211, BF-213, N-220, N-221, N-223, and N-224.

### EXAMPLES

The following examples further illustrate the present invention in detail, but should not be construed as limiting the present invention thereto.

### Example 1: Properties of permeability of compounds of the present invention into the brain

Compounds of the present invention were intravenously administered to mice to determine their in vivo permeability into the brain. Testing was in accordance with the following procedures:
(1) as mice were employed S1c:ICR weighing 30-40 g (7 weeks old, n=3) (Nippon SLC),
(2) compounds to be tested were dissolved in a mixture of 1N HCl, polyethylene glycol 400, and DMSO, or in DMSO or ethyl alcohol, and then diluted with purified water, and injected via tail vein. Two minutes after administration, the mice, under ether anesthesia, were subjected to collecting the blood from the abdominal aorta with a heparin-treated syringe and removing the brain,
(3) after drawing the blood, the blood was centrifuged at 14,000 rpm at 4°C for 10 minutes, and the supernatant was kept as plasma sample at -80°C. The brain (including cerebellum) was kept at -80°C after the removal,
(4) the plasma sample, when used, was thawed, diluted with purified water, and then applied to a conditioned C18 solid-phase extraction cartridge (bond elute C18, 200 mg, Varian), followed by elution with methyl alcohol. Alternatively, after thawing the plasma sample, a diethyl ether/cyclohexane mixture was added, and the mixture was shaken, and then centrifuged to separate an oil layer,
(5) the brain, when used, were subjected to measuring its wet weight, as was frozen, and saline was added for homogenization. The homogenate was centrifuged for 10 minutes, and the supernatant was applied to a conditioned C18 solid-phase extraction cartridge and eluted with methyl alcohol. Alternatively, after measuring the wet weight of the brain, a diethyl ether/cyclohexane mixture was added, and the mixture was homogenized, shaken, and then centrifuged to separate an oil layer,
(6) the absorbance and fluorescence were detected employing high performance liquid chromatography,
(7) for each of the plasma and brain, the contents in the plasma or brain of the compounds to be tested (%ID (injected)/ml or g) were determined, relative to the amount of dose,
(8) for the compounds to be tested, the absorbance and fluorescence were detected employing high performance liquid chromatography, and
(9) for each of the plasma and brain, the contents in the plasma or brain of the compounds to be tested (%ID (injected) /ml or g) were determined, relative to the amount of dose.

Table 2 shows the permeability into the brain in mice two minutes after intravenous administration of compounds to be tested.

**Table 2.**

| Permeability of compounds of the present invention into the brain two minutes after intravenous administration (mice) | | |
|---|---|---|
| Compound | %ID/g or ml | |
| | Brain | Plasma |
| BF-124 | 2.4 | 1.6 |
| BF-125 | 3.0 | 2.5 |
| BF-126 | 7.2 | 2.7 |
| BF-130 | 7.3 | 2.4 |
| BF-133 | 5.5 | 1.3 |
| BF-137 | 6.5 | 2.9 |
| BF-140 | 5.5 | 1.1 |
| BF-145 | 4.4 | 1.2 |
| BF-150 | 11.2 | 1.5 |
| BF-154 | 1.1 | 1.4 |
| BF-155 | 1.6 | 2.2 |
| BF-158 | 9.7 | 1.8 |
| BF-165 | 7.2 | 1.9 |
| BF-170 | 9.1 | 1.4 |
| BF-172 | 4.9 | 2.9 |
| BF-177 | 8.2 | 1.6 |
| BF-178 | 6.3 | 4.5 |
| BF-179 | 4.3 | 1.7 |
| BF-180 | 2.4 | 1.2 |
| BF-183 | 3.9 | 1.4 |
| BF-185 | 3.9 | 1.0 |
| BF-187 | 3.6 | 1.3 |
| BF-188 | 4.8 | 1.7 |
| BF-191 | 12.0 | 2.1 |
| BF-192 | 5.5 | 2.6 |
| BF-193 | 5.2 | 2.7 |
| BF-195 | 4.8 | 1.2 |
| BF-196 | 19 | 1.8 |
| BF-197 | 15 | 1.9 |
| BF-198 | 9.9 | 4.7 |
| BF-208 | 11.0 | 0.53 |
| BF-214 | 9.0 | 2.1 |
| BF-215 | 8.8 | 2.5 |
| BF-222 | 13.0 | 2.0 |
| BF-227 | 7.9 | 2.1 |
| N-282 | 4.0 | 0.7 |
| N-310 | 15 | 1.0 |
| N-313 | 4.6 | 1.1 |
| N-407 | 16.0 | 1.3 |
| N-438 | 11 | 1.9 |
| N-441 | 5.6 | 2.7 |
| N-453 | 5.0 | 1.4 |
| N-457 | 7.1 | 2.8 |
| N-491 | 7.4 | 2.3 |

Contents of the tested compounds of the present invention in the brain two minutes after administration were 1% ID/g or higher for all the compounds. With regard to the permeability into the brain of compounds for PET or SPECT whose target is the central nervous system, it is believed that values of 0.5 %ID/g or higher would be sufficient. In that sense, these compounds of the present invention are compounds having extremely high degrees of the permeability into the brain.

### Example 2: Acute toxicity of compounds of the present invention

Acute toxicity of compounds of the present invention was determined employing mice by intravenous administration. Male Crj:CD1 mice were used and divided into groups of 4 mice, with an average weight of each group of 31-32 g. Each compound was dissolved in a mixture of HCl, polyethylene glycol 400, and distilled water, or in DMSO, and then diluted with purified water, and administered via tail vein. Up to 7 days after administration, observations were made. Table 3 shows the results of the acute toxicity test on compounds of the present invention performed by the above-described procedures.

**Table 3.**

| Results of testing the acute toxicity of compounds of the present invention | |
|---|---|
| Compound | Maximum Tolerated Dose (mg/kg, intravenous administration) |
| BF-124 | ≧10 |
| BF-125 | ≧10 |
| BF-126 | ≧10 |
| BF-137 | ≧10 |
| BF-140 | ≧10 |
| BF-141 | 3 or higher, and less than 10 |
| BF-145 | ≧10 |
| BF-153 | 3 or higher, and less than 10 |
| BF-158 | ≧10 |
| BF-159 | ≧10 |
| BF-165 | ≧10 |
| BF-166 | <10 |
| BF-168 | ≧10 |
| BF-169 | ≧10 |
| BF-170 | ≧10 |
| BF-171 | ≧10 |
| BF-172 | ≧10 |
| BF-173 | ≧10 |
| BF-177 | ≧10 |
| BF-178 | ≧10 |
| BF-179 | ≧10 |
| BF-180 | ≧10 |
| BF-181 | 3 or higher, and less than 10 |
| BF-185 | ≧10 |
| BF-187 | ≧10 |
| BF-188 | ≧10 |
| BF-189 | ≧10 |
| BF-192 | ≧10 |
| BF-193 | ≧10 |
| BF-195 | ≧10 |
| BF-197 | ≧10 |
| BF-198 | ≧10 |
| BF-199 | ≧10 |
| BF-200 | ≧10 |
| BF-201 | ≧10 |
| BF-203 | ≧10 |
| BF-206 | ≧10 |
| BF-207 | ≧10 |
| BF-208 | ≧10 |
| BF-210 | ≧10 |
| BF-211 | ≧10 |
| BF-213 | ≧10 |
| BF-214 | ≧10 |
| BF-215 | ≧10 |
| BF-222 | ≧10 |
| BF-225 | ≧10 |
| BF-227 | ≧10 |
| BF-228 | ≧10 |
| BF-230 | ≧10 |
| BF-231 | ≧10 |
| N-313 | ≧10 |
| N-407 | ≧10 |
| N-437 | ≧10 |
| N-438 | ≧10 |
| N-441 | ≧10 |
| N-453 | ≧10 |
| N-457 | ≧10 |
| N-491 | ≧10 |

Most of the compounds of the present invention examined had a maximum tolerated dose of 10 mg/kg or higher upon intravenous administration. In general, the total dose of administration of a positron label and unlabeled compound for PET imaging in humans utilizes intravenous administrations ranging from 1 x 10⁻¹² to 1 x 10⁻⁵ mg/kg, and often from 1 x 10⁻¹⁰ to 1 x 10⁻⁷ mg/kg. Compared the values obtained with the compounds of the present invention to the total amount of compounds required for PET imaging, it is likely that these compounds of the present invention are extremely safe compounds as probes for PET imaging, since there are differences by a factor of at least 100, 000 or more between both compounds.

### Example 3: Imaging of abnormal prion proteins in autopsy brain sections

Formalin-fixed sections (7 µm thick) of autopsy brains of patients who were pathologically and definitely diagnosed as Gerstmann-Sträussler-Scheinker disease (GSS) or sporadic Creutzfeldt-Jacob disease (sCJD) were deparaffined, and stained for 30 minutes with solutions of compounds to be tested (10-200 µM), dissolved in 50% ethanol. After differentiation with 50% ethanol, the sections were washed with water, and fluorescent signals on the sections were observed under a confocal laser microscope (Leica, DMRXA) with an FITC or UV filter. The detection of abnormal prion proteins in the tissue sections was performed according to the method of Doh-ura et al. , Journal of Neuropathology and Experimental Neurology, vol. 59, pp. 774-785, 2000: the deparaffined tissue sections were treated by autoclaving them in diluted HCl (1-2 mM) for 10 minutes, and subj ected to immunoreaction using an anti-human prion protein antibody 3F4 (Seneteck, diluted 1:500) as a primary antibody and an horseradish peroxidase-labeled anti-mouse IgG antibody as a secondary antibody, and employing a color reaction with diaminobenzidine.

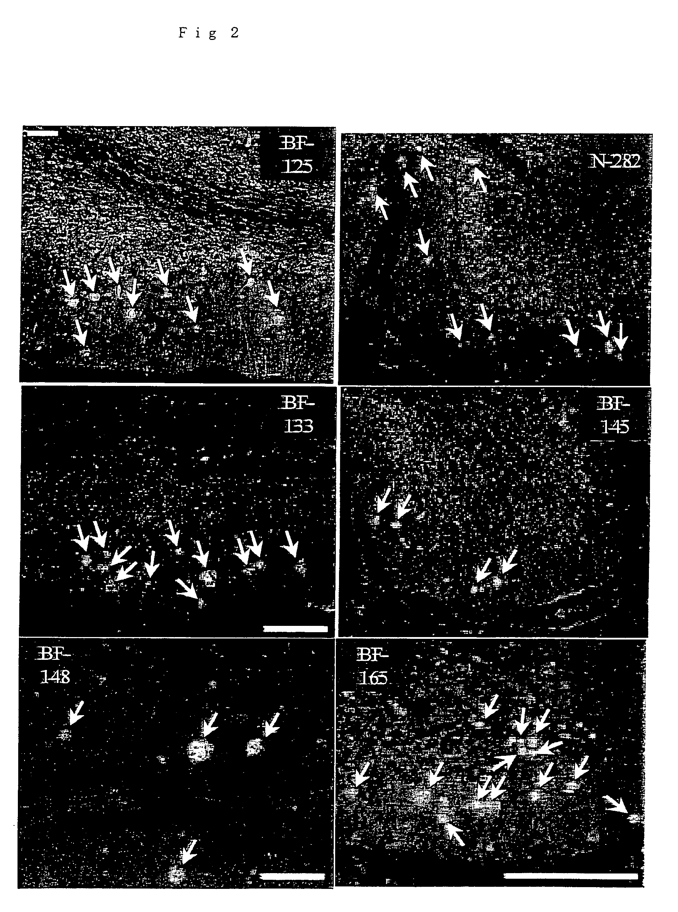

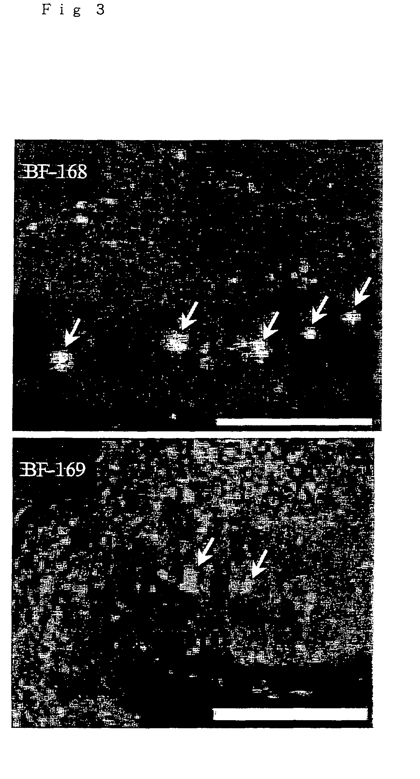

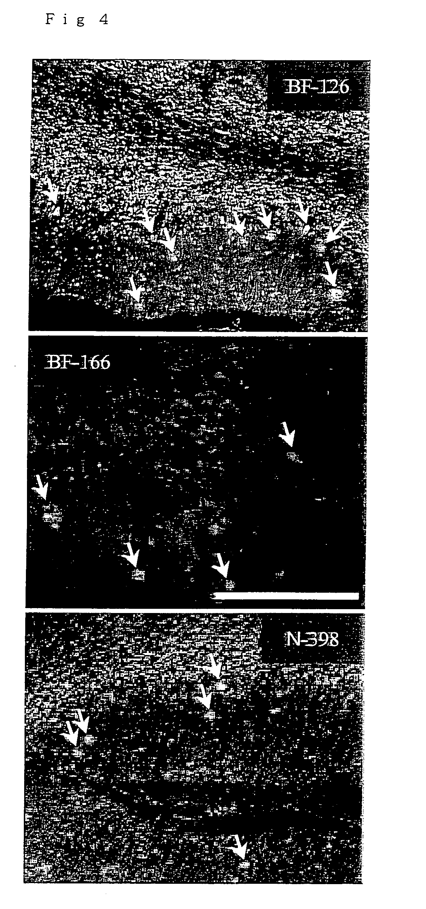

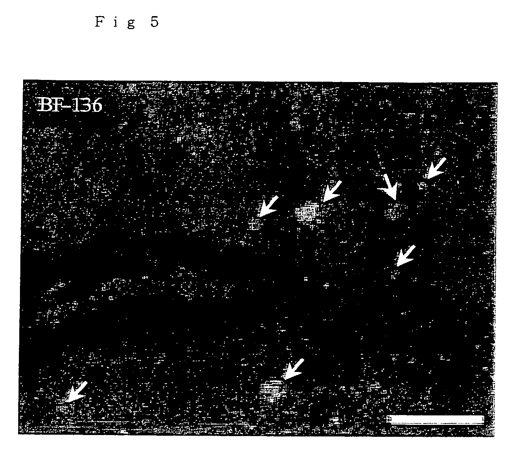

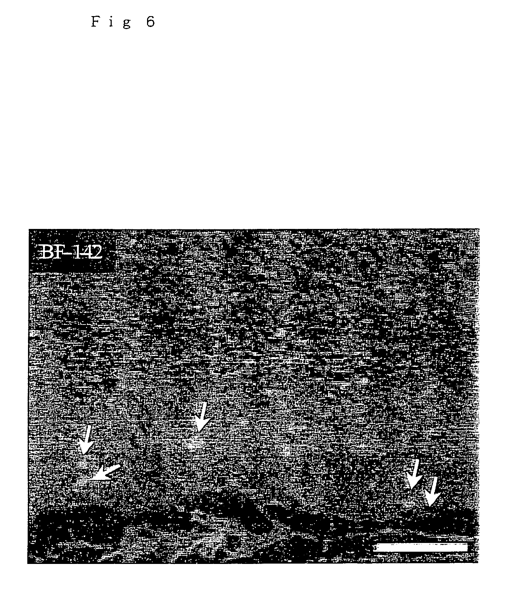

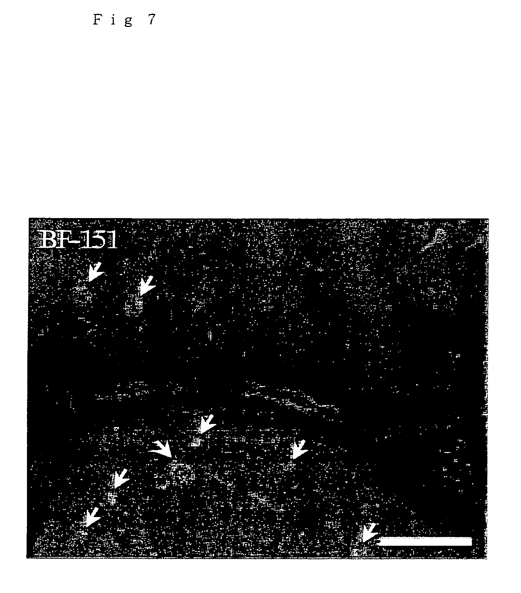

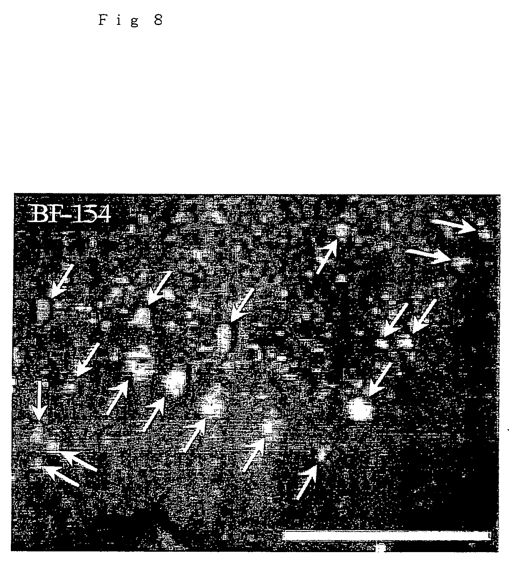

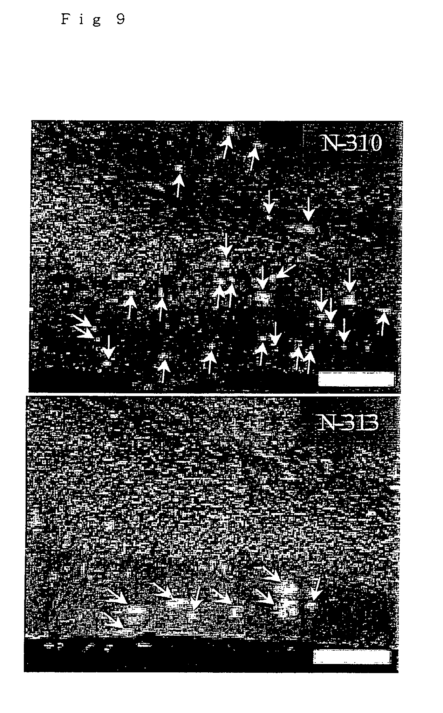

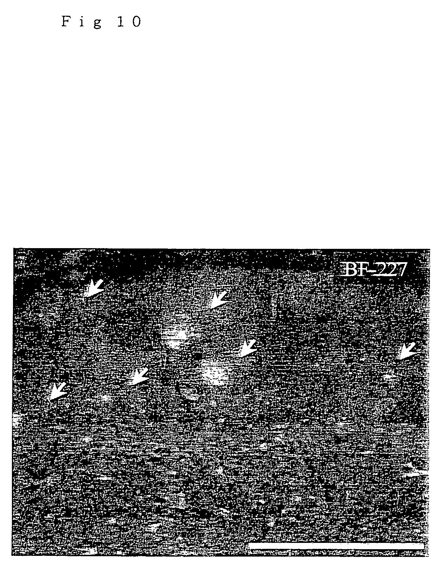

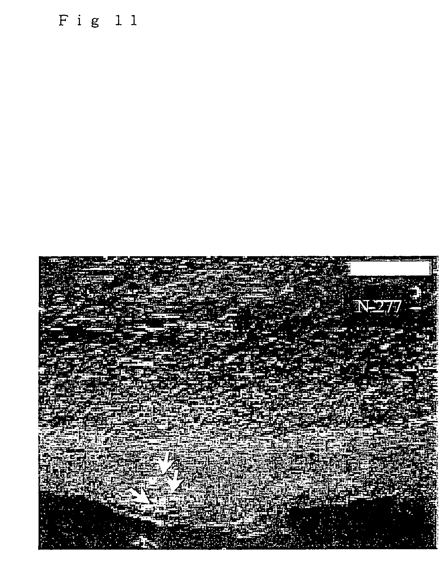

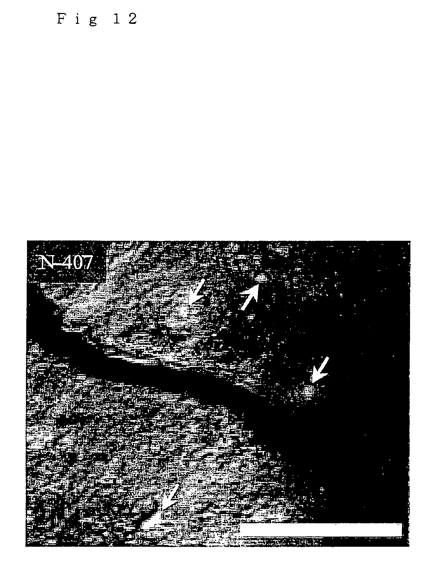

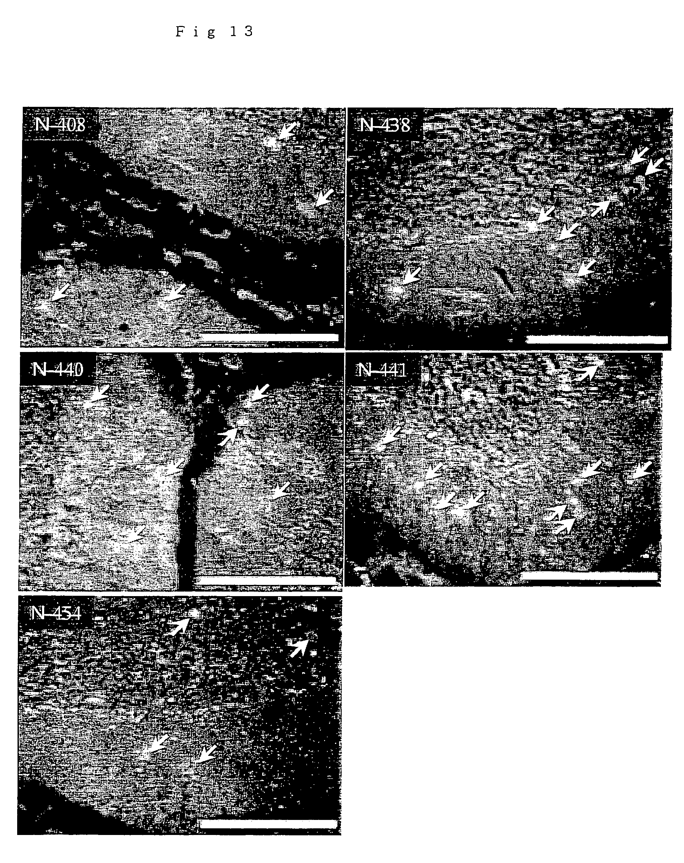

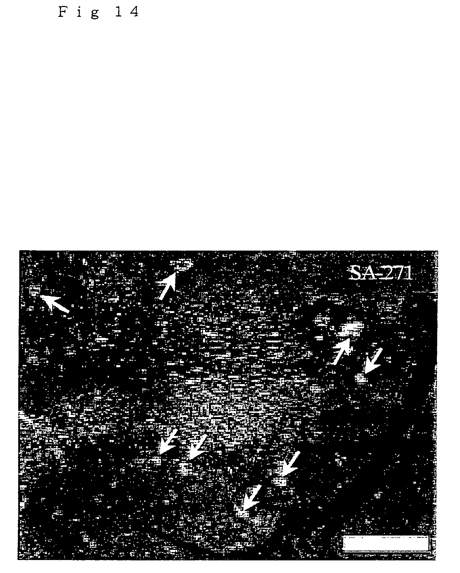

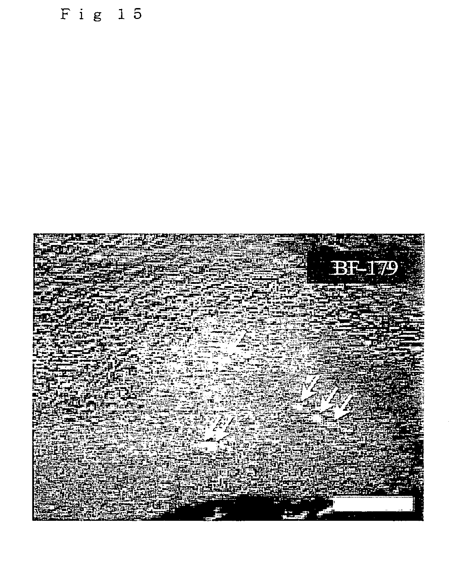

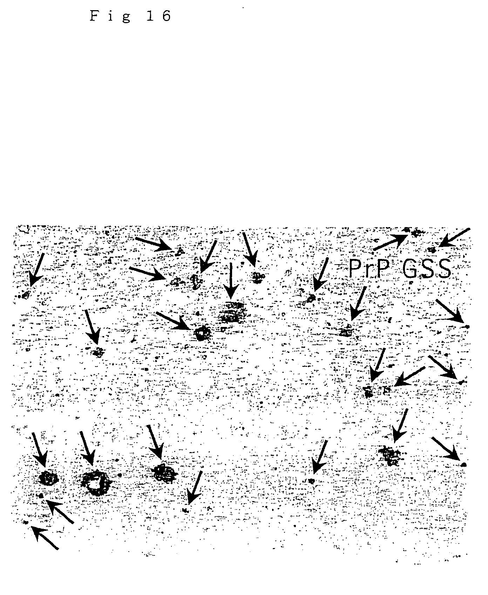

Testing was carried out using, as examples of compounds of the present invention, the following compounds: BF-124, N-276, N-227, BF-283, and BF-162 (Fig. 1), BF-125, N-282, BF-133, BF-145, BF-148, and BF-165 (Fig. 2), BF-168 and BF-169 (Fig. 3), BF-126, BF-166, and N-398 (Fig. 4), BF-136 (Fig. 5), BF-142 (Fig. 6), BF-151 (Fig. 7), BF-154 (Fig. 8), N-310 and N-313 (Fig. 9), BF-227 (Fig. 10), N-227 (Fig. 11), N-407 (Fig. 12), N-408, N-438, N-440, N-441, and N-454 (Fig. 13), SA-271 (Fig. 14), and BF-179 (Fig. 15). With any one of these compounds, spotted fluorescent signals were observed in the brain of the GSS patients, mainly in the cerebellar cortex. These spotted structures were identical to spotted depositions (kuru plaques) of abnormal prion proteins identified by immunostaining prion protein in serial sections. Fig. 16 shows immunostaining of abnormal prion proteins in brain sections of a GSS patient.

All of these compounds resulted in specific imaging of abnormal prion protein plaques and did not give non-specifically stained images (for example, blood and connective tissues) (Figs. 1-15). Thus, it has been revealed that the compounds of the present invention have superior properties of detecting abnormal prion protein in samples and can be used as agents for specifically staining abnormal prion protein.

### Example 4: Examination of anti-prion effects in a prion-infected cultured cell model

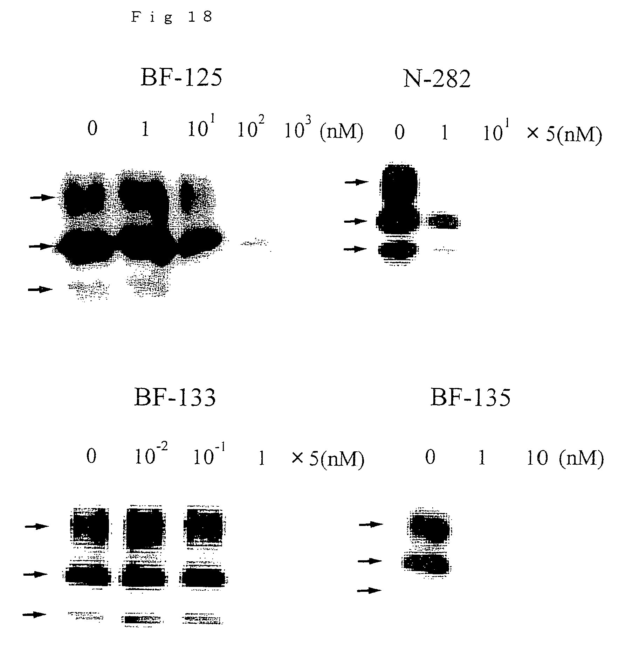

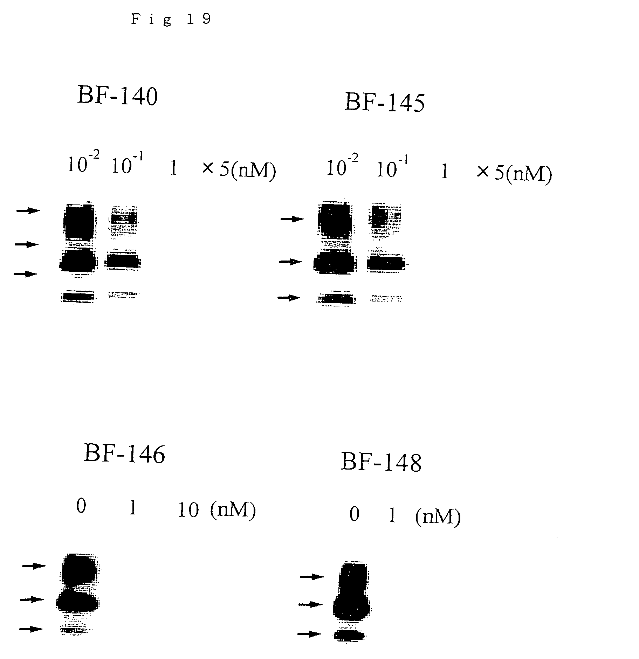

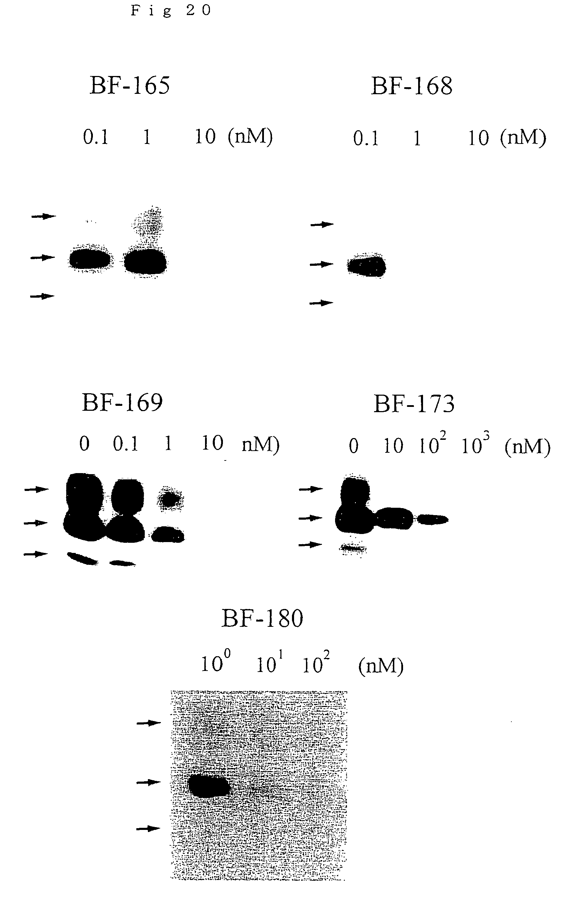

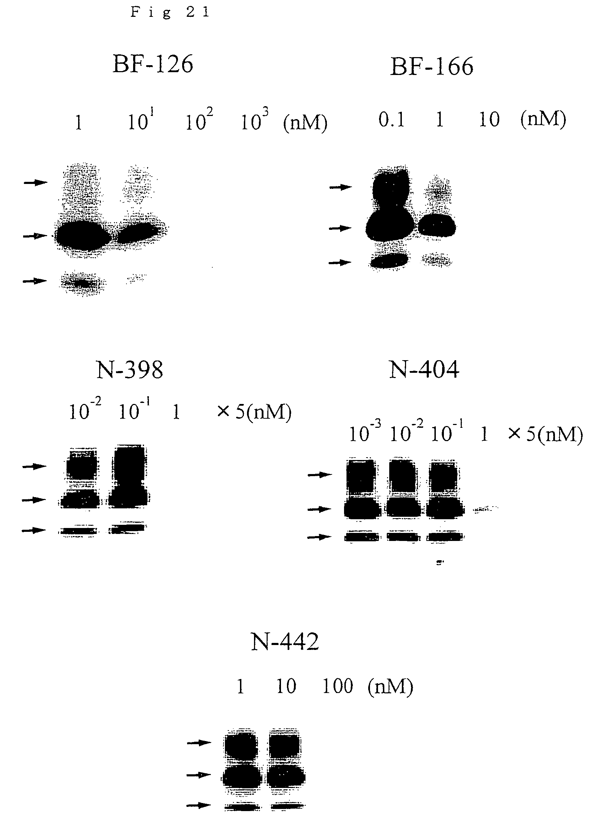

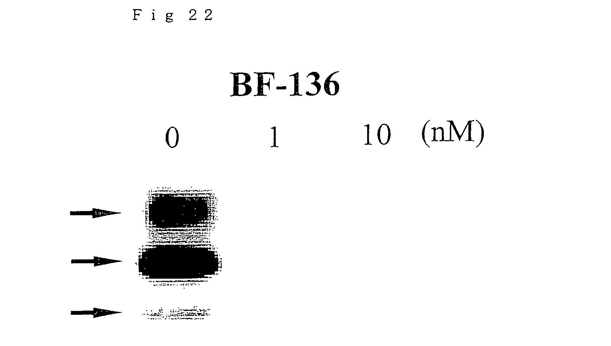

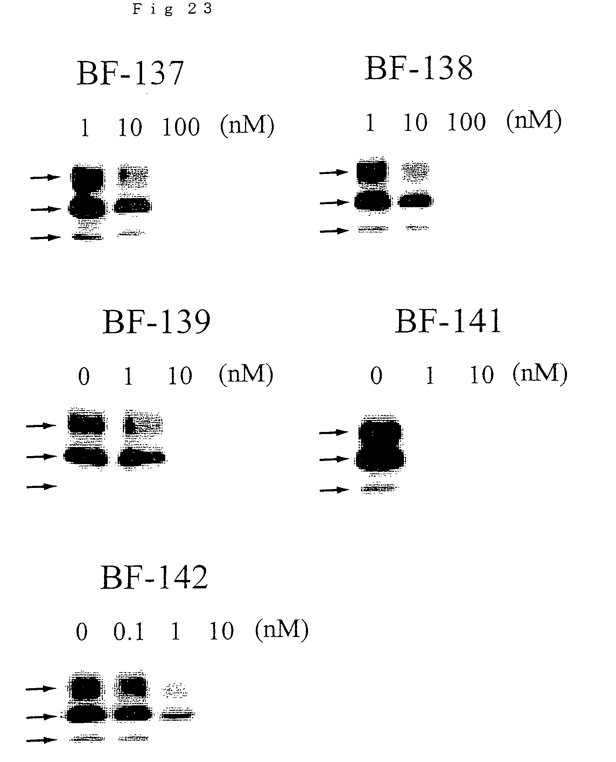

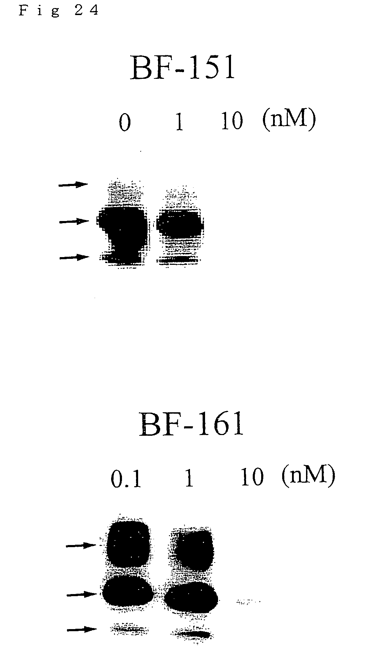

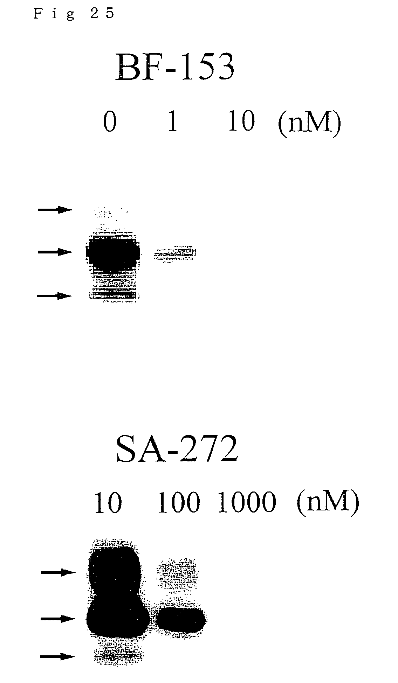

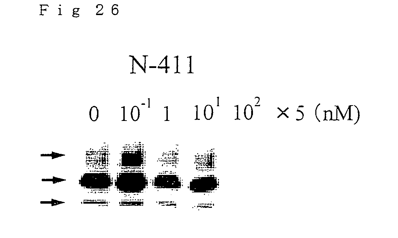

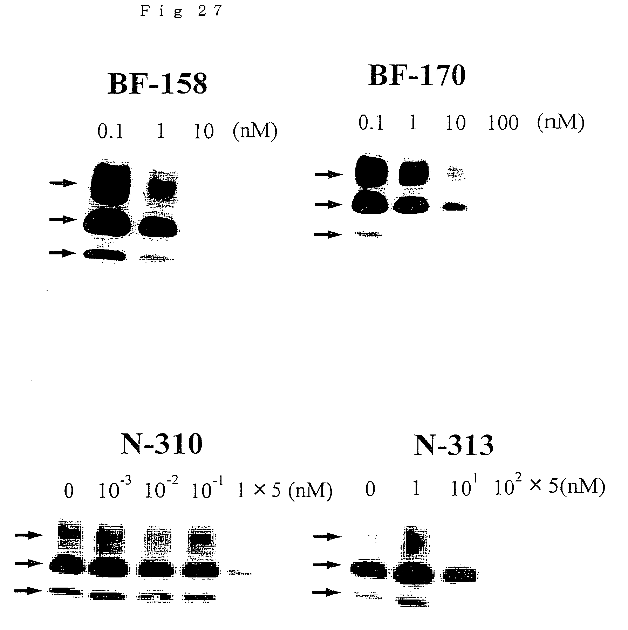

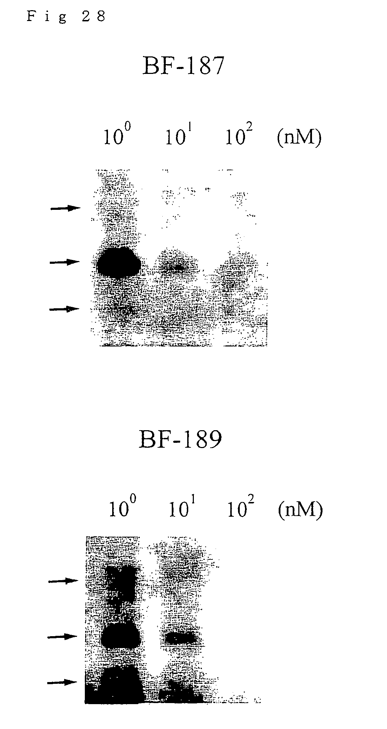

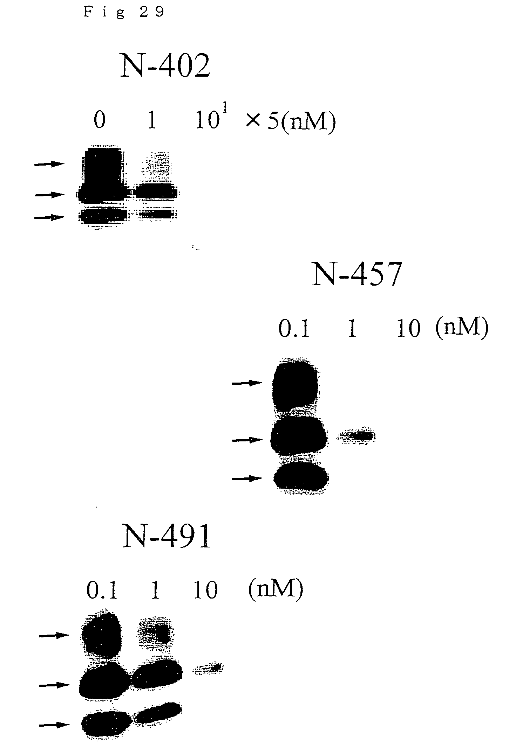

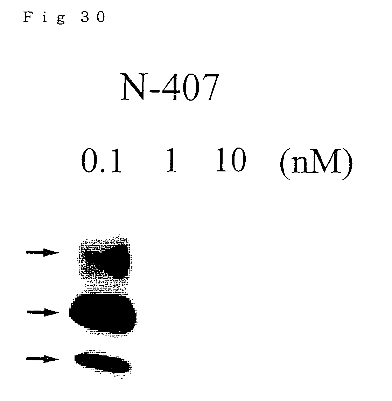

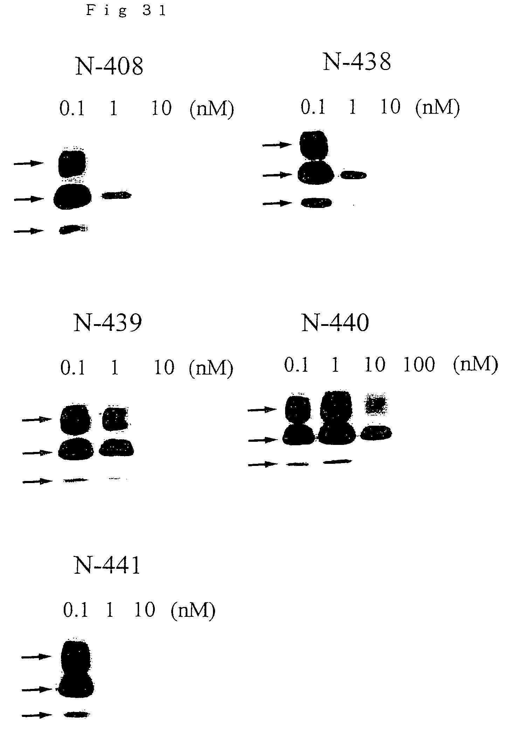

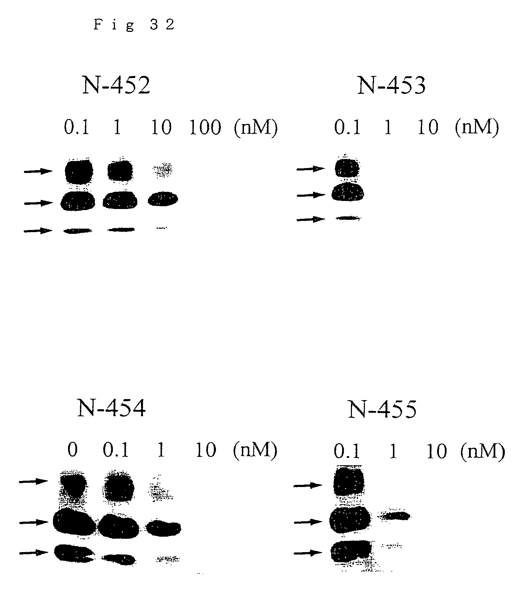

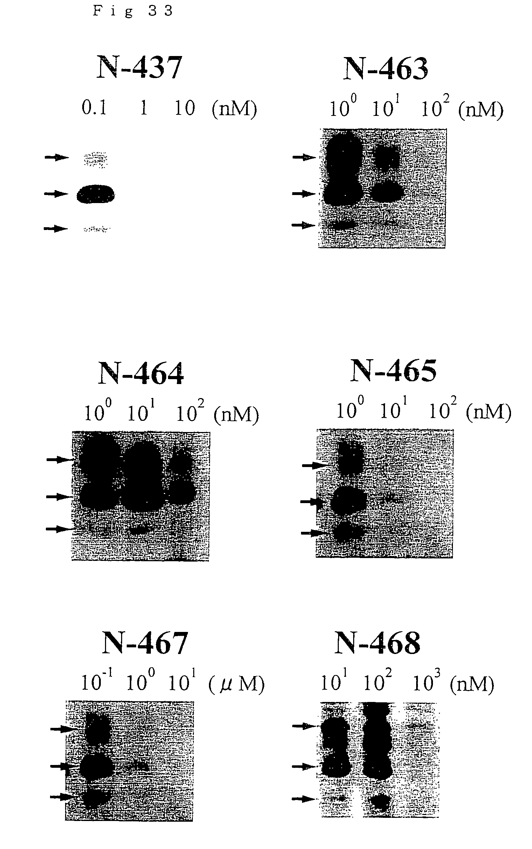

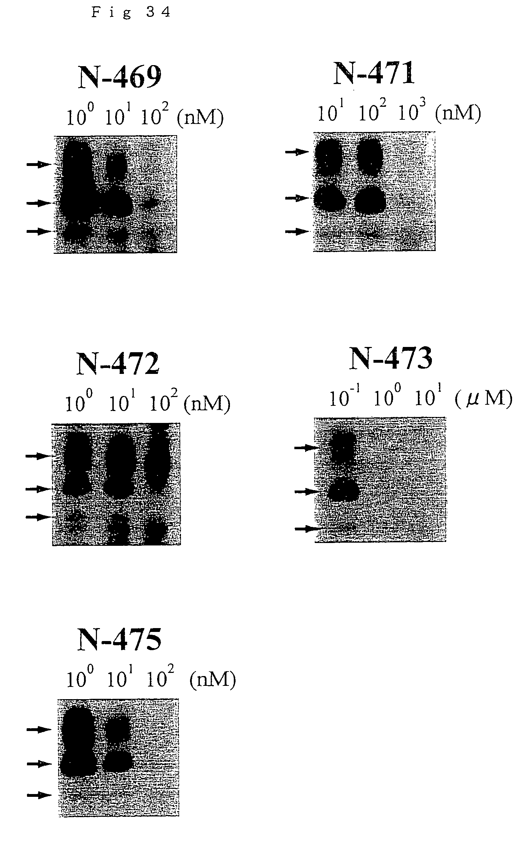

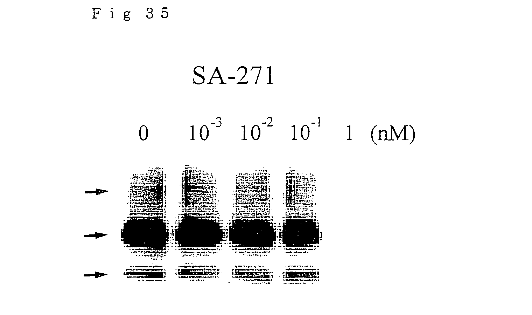

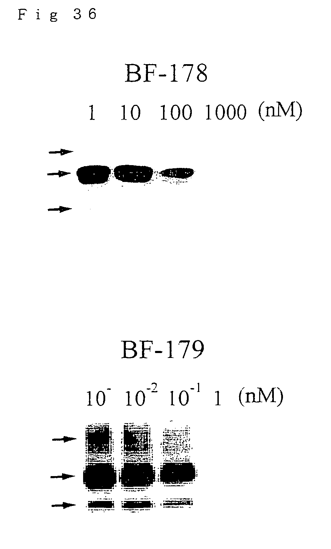

Mouse neuroblastoma cells (ScN2a) with persistent infection of the infectious agent of scrapie, a sheep prion disease, (Race et al. , Journal of Virology, vol. 62, 2845-2849, 1998), were used to examine inhibitory effects of compounds to be tested (listed in Table 4) on producing abnormal prion proteins (with resistance against proteolytic enzymes), according to the method of Doh-ura etal., Journal of Virology, vol. 74, 4894-4897, 2000. Inpassaging cells (cells of 1/10 of the cell number at the confluent state into culture flasks), compounds to be tested, which were dissolved in 100% dimethyl sulfoxide (DMSO), were added at various concentrations (1 nM to 1 µM) to culture medium (an OPTI-MEM medium (GIBCO BRL) supplemented with 10% bovine fetal serum). Medium to which DMSO alone was added was utilized as control. Four days later, when control cells reached confluence, the presence or absence of cell proliferation impairment in the groups in which the compounds were administered was evaluated by counting the number of cells, and then the cells were washed with phosphate buffered saline and subjected to lysis in a lysing solution (0.5% deoxycholate, 0.5% Nonidate P40 in phosphate buffered saline). The cell lysate was centrifuged to remove nucleic acid components, and to the supernatant, proteinase K (at a final concentration of 10 µg/ml) was added, followed by reaction at 37°C for 30 minutes. Then, PMSF, a serine-protease inhibitor (at a final concentration of 10 µg/ml, 4°C) was added to stop the reaction, and the reaction solution was ultracentrifuged at 371,000 g at room temperature for 30 minutes. The residue was suspended in 1X SDS sample buffer, and after heat denaturing, subjected to electrophoresis on 15% Tris-glycine-SDS polyacrylamide gel. Separated proteins were transferred onto a PVDF membrane, which in turn was subjected to Western blotting procedures using an anti-prion protein antibody (PrP2B, a rabbit polyclonal antibody directed to a peptide of the amino acid residues 89-103 of prion protein, diluted 1:5000) as a primary antibody and an alkaline phosphatase-labeled anti-rabbit IgG antibody as a secondary antibody. Signals were visualized with a CDP-Star reaction solution (Amersham). For testing by such blotting were employed the following compounds of the present invention: BF-124, N-276, N-277, BF-283, and BF-162 (Fig. 17), BF-125, N-282, and BF-135 (Fig. 18), BF-140, BF-145, BF-146, and BF-148 (Fig. 19), BF-165, BF-168, BF-169, BF-173, andBF-180 (Fig. 20), BF-126, BF-166, N-398, N-404, and N-442 (Fig. 21), BF-136 (Fig. 22), BF-137, BF-138, BF-139, BF-141, and BF-142 (Fig. 23), BF-151 and BF-161 (Fig. 24), BF-153 and SA-272 (Fig. 25), N-411 (Fig. 26), BF-158, BF-170, N-310, and N-313 (Fig. 27), BF-187 and BF-189 (Fig. 28), N-402, N-457, and N-491 (Fig. 29), N-407 (Fig. 30), N-408, N-438, N-439, N-440, andN-441 (Fig. 31), N-452, N-453, N-454, and N-455 (Fig. 32), N-437, N-463, N-464, N-465, N-467, and N-468 (Fig. 33), N-469, N-471, N-472, N-473, and N-475 (Fig. 34), AS-271 (Fig. 35), and BF-178 and BF-179 (Fig. 36).

All of these compounds have been found to have inhibitory effects on producing abnormal prion proteins in ScN2a (Table 4, Figs. 5-8). Concentrations of compounds at which the producing of abnormal prion proteins was suppressed by 50% (IC₅₀, 50% inhibition concentration) ranged from 0.8 to 1000 nM, and concentrations of impairing cell proliferation (TC, toxic concentration) were in the range of 10 to 100 µM for most of the compounds. It is believed that the safety concentration margin (TC/IC₅₀) is between 100 and 100,000 (Table 4).

Of these compounds, the following compounds have been found to possess small IC₅₀ values and exert highly inhibitory effects on producing abnormal prion proteins: BF-130, BF-135, BF-136, BF-141, BF-146, BF-148, BF-150, BF-153, BF-168, N-220, N-221, N-224, N-232, N-243, N-246, N-407, N-441, N-453, andN-457. It has turned out that of these compounds, BF-130, BF-135, BF-146, N-407, N-441, N-453, and N-457 are compounds which have higher TCs, therefore higher safety concentration margins, and superior effects.

On the one hand, quinacrine had a safety concentration margin of 5 (cited from Doh-ura et al., Journal of Virology, vol. 74, 4894-4897, 2000).

**Table 4.**

| Inhibitory effects on producing abnormal prion proteins, concentrations of inhibiting cell proliferation, and safety concentration margins of compounds of the present invention | | | |
|---|---|---|---|
| Compound No. | Inhibitory effects on producing abnormal prion protein IC₅₀ (nM) | Concentration of inhibiting cell proliferation TC (µM) | Safety concentration margin (TC/IC₅₀) |
| BF-124 | 100 | 100 | 1000 |
| BF-125 | 100 | 50 | 500 |
| BF-126 | 100 | 25 | 250 |
| BF-130 | 1 | 100 | 100000 |
| BF-133 | 5 | 50 | 10000 |
| BF-135 | 1 | 100 | 100000 |
| BF-136 | 1 | 10 | 10000 |
| BF-137 | 100 | 100 | 1000 |
| BF-138 | 100 | 100 | 1000 |
| BF-139 | 10 | 100 | 10000 |
| BF-140 | 5 | 50 | 10000 |
| BF-141 | 1 | 10 | 10000 |
| BF-142 | 10 | 100 | 10000 |
| BF-143 | 10 | 100 | 10000 |
| BF-145 | 5 | 50 | 10000 |
| BF-146 | 1 | 100 | 100000 |
| BF-147 | 100 | 100 | 1000 |
| BF-148 | 1 | 10 | 10000 |
| BF-150 | 1 | 10 | 10000 |
| BF-151 | 10 | 100 | 10000 |
| BF-153 | 1 | 10 | 10000 |
| BF-158 | 10 | 100 | 10000 |
| BF-160 | 10 | 10 | 1000 |
| BF-161 | 10 | 100 | 10000 |
| BF-162 | 10 | 100 | 10000 |
| BF-165 | 10 | 100 | 10000 |
| BF-166 | 10 | 10 | 1000 |
| BF-168 | 1 | 10 | 10000 |
| BF-169 | 10 | 100 | 10000 |
| BF-170 | 100 | 100 | 1000 |
| BF-172 | 1.6 | > 10 | > 6250 |
| BF-173 | 100 | 100 | 1000 |
| BF-178 | 1000 | 100 | 100 |
| BF-179 | 5 | 5 | 1000 |
| BF-180 | 10 | 100 | 10000 |
| BF-187 | 10 | 10 | 1000 |
| BF-189 | 100 | 100 | 1000 |
| BF-191 | 4 | > 10 | > 2500 |
| BF-192 | 1.6 | > 10 | > 6250 |
| BF-193 | 1.6 | 10 | 6250 |
| BF-195 | 32 | 10 | 3125 |
| BF-196 | 16 | > 10 | > 625 |
| BF-197 | 16 | 10 | 625 |
| BF-198 | 0.8 | 10 | 12500 |
| BF-199 | 0.8 | > 10 | > 12500 |
| BF-201 | 0.8 | 1 | 1250 |
| BF-203 | 0.8 | 10 | 12500 |
| BF-204 | 1.6 | > 10 | > 6250 |
| BF-206 | 2 | > 10 | > 5000 |
| BF-208 | 0.8 | > 10 | > 12500 |
| BF-211 | 1.6 | > 10 | > 6250 |
| BF-213 | 2 | 10 | > 5000 |
| BF-221 | 80 | > 10 | > 125 |
| BF-222 | 8 | > 10 | > 1250 |
| BF-227 | 4 | 10 | 2500 |
| BF-228 | 16 | > 10 | > 625 |
| BF-231 | 1.6 | > 10 | > 6250 |
| BF-234 | 40 | > 10 | > 250 |
| N-220 | 1 | 10 | 10000 |
| N-221 | 1 | > 10 | > 10000 |
| N-223 | 10 | > 10 | > 1000 |
| N-224 | 1 | > 10 | > 10000 |
| N-225 | 100 | > 10 | > 100 |
| N-226 | 100 | > 10 | > 100 |
| N-227 | 10 | 10 | 1000 |
| N-231 | 10 | 10 | 1000 |
| N-232 | 1 | 10 | 10000 |
| N-233 | 100 | 10 | 100 |
| N-234 | 100 | > 10 | > 100 |
| N-236 | 100 | > 10 | > 100 |
| N-240 | 100 | 10 | 100 |
| N-241 | 10 | 10 | 1000 |
| N-242 | 100 | 10 | 100 |
| N-243 | 1 | > 10 | > 10000 |
| N-244 | 10 | > 10 | > 1000 |
| N-245 | 100 | 10 | 100 |
| N-246 | 1 | 10 | 10000 |
| N-276 | 5 | 50 | 10000 |
| N-277 | 50 | 50 | 1000 |
| N-282 | 50 | 50 | 1000 |
| N-283 | 500 | 50 | 100 |
| N-310 | 5 | 50 | 10000 |
| N-313 | 500 | 50 | 100 |
| N-398 | 5 | 50 | 10000 |
| N-402 | 50 | 50 | 1000 |
| N-404 | 5 | 50 | 10000 |
| N-407 | 1 | 100 | 100,000 |
| N-408 | 10 | 100 | 10,000 |
| N-437 | 1 | 100 | 100000 |
| N-438 | 10 | 100 | 10,000 |
| N-439 | 10 | 100 | 10,000 |
| N-440 | 100 | 100 | 1,000 |
| N-441 | 1 | 100 | 100,000 |
| N-442 | 100 | 100 | 1000 |
| N-452 | 100 | 100 | 1,000 |
| N-453 | 1 | 100 | 100,000 |
| N-454 | 10 | 100 | 10,000 |
| N-455 | 10 | 100 | 10,000 |
| N-457 | 1 | 100 | 100,000 |
| N-461 | 100 | > 10 | > 100 |
| N-463 | 100 | 100 | 1000 |
| N-464 | 100 | 100 | 1000 |
| N-465 | 10 | 100 | 10000 |
| N-467 | 1000 | 100 | 100 |
| N-468 | 1000 | 100 | 100 |
| N-469 | 100 | ≧ 100 | ≧ 1000 |
| N-471 | 1000 | ≧ 100 | ≧ 100 |
| N-472 | 10 | 100 | 10000 |
| N-473 | 1000 | 100 | 100 |
| N-475 | 100 | 100 | 1000 |
| N-491 | 10 | 100 | 10,000 |
| SA-271 | 5 | 50 | 10000 |
| SA-272 | 1000 | 100 | 100 |
| HT-040 | 10 | > 10 | > 1000 |
| HT-041 | 10 | > 10 | > 1000 |
| HT-042 | 10 | > 100 | > 1000 |
| HT-043 | 100 | > 10 | > 100 |
| TK-005 | 100 | 10 | 100 |
| quinacrine | 400 | 2 | 5 |

As described in the above Examples, it has turned out that the compounds of the present invention result in easy detection of spotted depositions of abnormal prion proteins and inhibit the production of abnormal prion proteins in infected cells which are the etiology. These results suggest that these compounds have possibilities not only of applications to prion bio-imaging probes for detecting abnormal prion proteins in patients with prion diseases, but also of applications as therapeutic or prophylactic drugs against prion diseases.

Target organs of the prion diseases are the central nervous system, and the infectious agent, prion, that is, abnormal prion proteins is accumulated in the central nervous system, leading to neuroral degeneration. The definite diagnosis of prion diseases is to identify abnormal prion proteins accumulated in the brain, but it is impossible to make a definite diagnosis during one's life, nless biopsy of brain tissues is carried out by neurosurgery. It has also turned out that in animal experiments, the accumulation of abnormal prion proteins in the brain already takes place at stages much earlier than the onset of disease signs, and the amount of their depositions increases as the disease progresses (Doh-ura et al., Journal of General Virology, vol. 80, 1551-1556, 1999). While Congo red, Thioflavin, and others are known as reagents capable of imaging abnormal prion protein amyloid in tissue sections, all the compounds examined in the present study showed clear detection of spotted depositions of abnormal prion proteins with more sensitivity and specificity than those of the above-mentioned reagents. Therefore,these compounds having high degree of the permeability into the brain can be labeled with radioisotopes, followed by peripheral administration to patients suspected to be affected with prion diseases, such that the compounds can be utilized for the representation of abnormal prion proteins accumulated in the brain by means of nuclear medical methods such as PET and SPECT, thereby to carry out early diagnosis of prion diseases and to understand progress of the illness.

On the other hand, compounds for inhibiting the proliferation of prion, which is the infectious agent of prion diseases, would be expected as drugs for prophylaxis and/or treatment. The proliferation ofprionmeans production of abnormal prion proteins, and thus compounds inhibiting and/or suppressing this production are therapeutic drugs against prion diseases. The compounds of the present invention inhibit the production of abnormal prion proteins and are estimated for their safety concentration margins to be between 1,000 and 100,000.

Compounds reported until now to be effective as inhibitors of producing abnormal prion proteins include Congo red, polysaccharide sulfates (Caughey and Roymond, Journal of Neurochemistry, vol. 59, 768-771, 1992, Caughey and Roymond, Journal of Virology, vol. 67, 643-650, 1993), cyclic tetrapyrroles (porphyrins and phthalocyanines) (Caughey et al., Proceedings of the National Academy of Sciences USA, vol. 95, 12117-12122, 1998), branched polyamines (polyamidoamine and polypropyleneimine dendrimers) (Supattapone et al., Journal of Virology, vol. 75, 3453-3461, 2001), lysosome-accumulating drugs such as quinacrine and chloroquine, E-64d cysteine-protease inhibitor (Doh-ura et al., Journal of Virology, vol. 74, 4894-4897, 2000), and others. These compounds pose problems in the permeability into the brain, or even if they have good properties of the permeability into the brain, most of the compounds are not suitable for practical application, due to their extremely narrow safety concentration margins. For example, the above-mentioned compounds except quinacrine have extremely low degree of the permeability into the brain. It has been reported that quinacrine, which draws recent interest, has an extremely narrow safety concentration margin (less than 10) (Doh-ura et al. , Journal of Virology, vol. 74, 4894-4897, 2000). Unlike these conventional drugs, the compounds of the present invention are effective as inhibitors of abnormal prion protein biosynthesis, and moreover have extremely high degree of the permeability into the brain and safety.

The following will give an explanation of applicability as in vitro diagnostics of the compounds of the present invention. The compounds of the present invention bind to abnormal prion proteins, and therefore are applicable also as staining agents and in vitro diagnostics of prion diseases in humans and animals. The use of the compounds of the present invention allows an easier diagnosis of prion diseases which have been definitely diagnosed by identifying abnormal prion proteins via ELISA methods, Western blotting, immunostaining.

In conventional techniques, for example, confirmation of abnormal prion proteins of bovine spongiform encephalopathy has been done by identifying the prion protein via ELISA methods, Western blotting, or immunostaining. However, the compounds of the present invention can be used to stain or determine brain sections or brain homogenates, thereby identifying abnormal prion proteins easier and for a shorter time to make a diagnosis of prion diseases. It is also possible to diagnose prion disease by using the compounds of the present invention to identify abnormal prion proteins in lymphoid tissues, urine, blood. Further, it is possible to use the compounds of the present invention to identify abnormal prion proteins in bovine-derived foods, medical preparations (for example, gelatin capsules), cosmetics (for example, collagen), and others.

As explained above, the compounds of the present invention have high specificity for abnormal prion proteins, enhanced blood-brain barrier permeability, and extremely high degree of safety. Therefore, the compounds of the present invention are exceedingly useful for early diagnosis and discovery of prion diseases. According to the present invention, there are provided a composition and a kit for the diagnosis of diseases in which prion protein is accumulated, the composition and the kit comprising a compound of the present invention. There is also provided a method for the diagnosis of diseases in which prion protein is accumulated, the method using a compound of the present invention. Further, according to the present invention, there is also provided a composition for the prophylaxis and/or treatment of diseases in which the accumulation of prion protein in the body constitutes or partially constitutes the etiology, the composition having a compound of the present invention contained therein. There is also provided a method for the treatment and/or prophylaxis of diseases in which the accumulation of prion protein in the body constitutes or partially constitutes the etiology, the method
characterized by administering a compound of the present invention to a subject. The present invention will make it possible to carry out early and effective treatment of prion diseases in combination with early diagnosis and discovery of diseases in which prion protein is accumulated. Further, according to the present invention, there are also provided a composition and a kit for staining abnormal prion protein in samples, the composition and the kit comprising a compound of the present invention, as well as a method for staining abnormal prion protein in samples, the method using a compound of the present invention.

## Claims

1. A compound, which is used as a probe for diagnosing diseases in which prion protein is accumulated, represented by the formula (I) or (II): 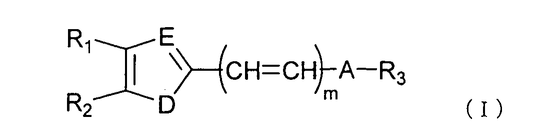 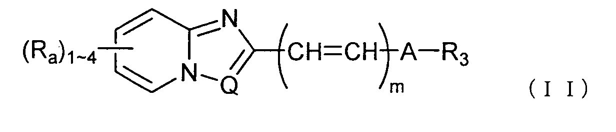 wherein D is NR', S, O, CH=CH, or CH₂,
R' is H, alkyl having 1 to 4 carbons (hereinafter, referred to as C₁₋₄ alkyl), or phenyl, wherein the C₁₋₄ alkyl is optionally substituted with halogen(s),
E is N or CH,
Rₐ is, each independently, selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O- C₁₋₄ alkyl, COOH, and SO₃H, wherein the C₁₋₄ alkyl is optionally substituted with halogen(s),
Q is N or CR_{b},
R_{b} is selected from the group consisting of H, C₁₋₄ alkyl, halogen, OH, C₁₋₄ alkyl-OH, C₁₋₄ alkyl-O-C₁₋₄ alkyl, NH(C₁₋₄ alkyl), NH₂, N(C₁₋₄ alkyl)₂, NO₂, O-C₁₋₄ alkyl, COOH, and SO₃H, wherein the C₁₋₄ alkyl is optionally substituted with halogen(s),
m is an integer of 0 to 4,
R₁ and R₂ are independently selected from the group consisting of H, C₁₋₄ alkyl, halogen, OH, C₁₋₄ alkyl-OH, C₁₋₄ alkyl-O-C₁₋₄ alkyl, NH(C₁₋₄ alkyl), NH₂, N(C₁₋₄ alkyl)₂, NO₂, O-C₁₋₄ alkyl, COOH, and SO₃H, wherein the C₁₋₄ alkyl is optionally substituted with halogen (s),
or alternatively, R₁ and R₂, together, form a benzene or naphthalene ring which is optionally substituted with one to four R₄,
R₃ is selected from the group consisting of H, C₁₋₄ alkyl,
halogen, OH, C₁₋₄alkyl-OH, C₁₋₄alkyl-O-C₁₋₄alkyl, NH₂, NH(C₁₋₄alkyl), N(C₁₋₄ alkyl)₂, NO₂, O-C₁₋₄ alkyl, COOH, SO₃H, wherein the C₁₋₄ alkyl is optionally substituted with halogen(s), and any one of the moieties represented by (a) to (e): 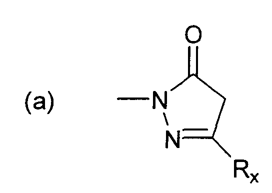 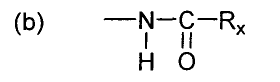 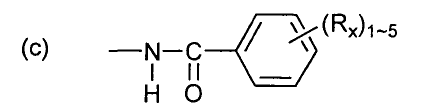 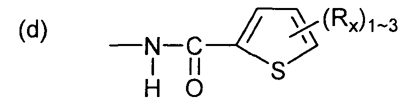 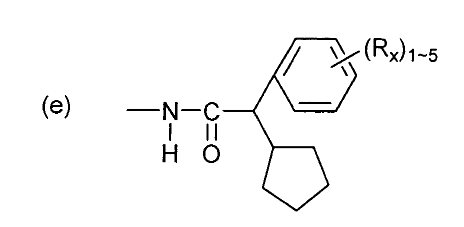 wherein each Rₓ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, N=CH-allyl, NO₂, O-C₁₋₄ alkyl, COOH, and SO₃H, wherein the C₁₋₄ alkyl is optionally substituted with halogen(s),
each R₄ is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, SO₃H,
wherein the C₁₋₄ alkyl is optionally substituted with halogen(s), and any one of the moieties represented by (f) to (l): 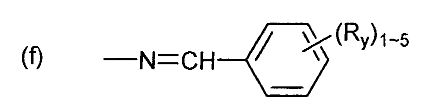 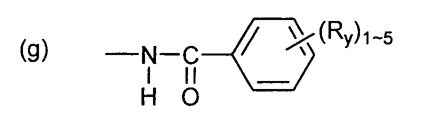 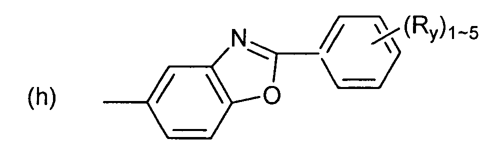 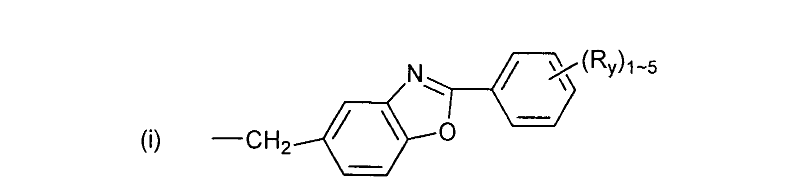 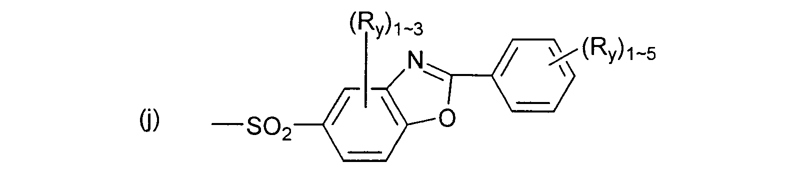 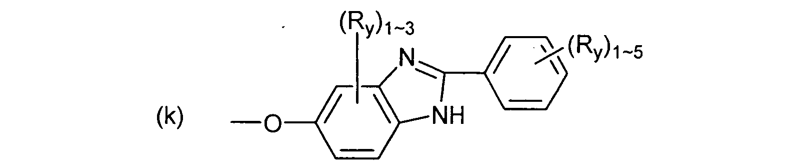 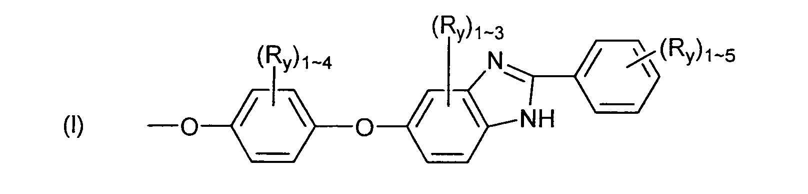 wherein two R₄s attached on adjacent carbons may form a methylenedioxy group, and wherein the C₁₋₄ alkyl is optionally substituted with halogen(s),
each R_{y} is independently selected from the group consisting of H, C₁₋₄ alkyl, halogen, OH, C₁₋₄ alkyl-OH, C₁₋₄ alkyl-O-C₁₋₄ alkyl, NH₂, NH(C₁₋₄ alkyl), N(C₁₋₄ alkyl)₂, NO₂, O-C₁₋₄ alkyl, COOH, and SO₃H,
wherein the C₁₋₄ alkyl is optionally substituted with halogen (s),
A is any one of the rings represented by (i) to (ix): 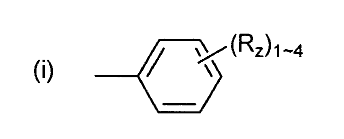 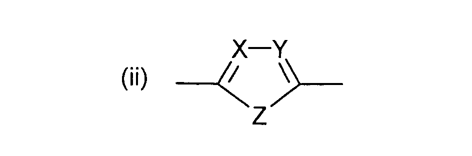 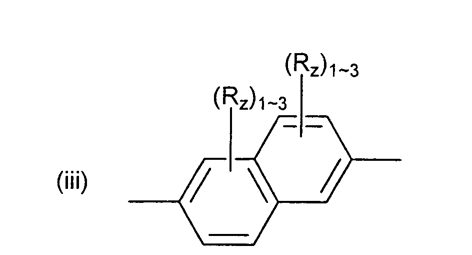 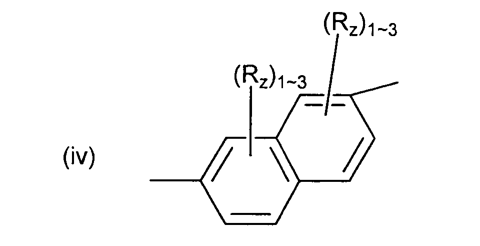 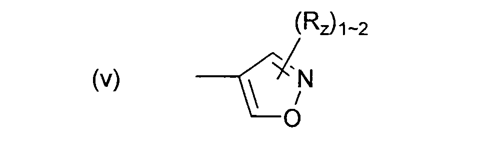 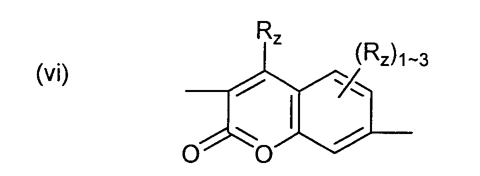 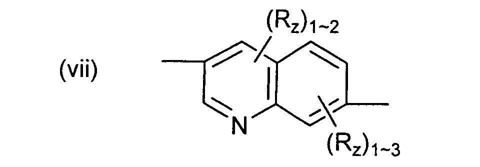 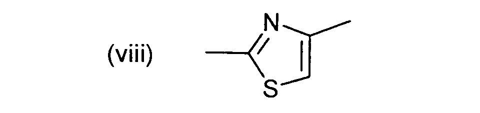 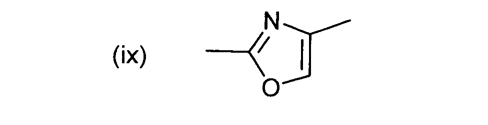 wherein each R_{z} is independently selected from the group consisting of H, C₁₋₄ alkyl, halogen, OH, C₁₋₄ alkyl-OH, C₁₋₄ alkyl -O-C₁₋₄ alkyl, NH₂, NH(C₁₋₄ alkyl), N(C₁₋₄ alkyl)₂, NO₂, O-C₁₋₄ alkyl, phenyl, COOH, and SO₃H, wherein the C₁₋₄ alkyl is optionally substituted with halogen(s),
X is N or CH,
Y is N or CH,
Z is O, S, CH₂, N-CₚH₂ₚ₊₁, and
p is an integer of 0 to 4,
or a salt or solvate thereof.

2. The compound according to claim 1, wherein the compound is selected from the group consisting of BF-124, BF-125, BF-126, BF-133, BF-136, BF-142, BF-143, BF-147, BF-148, BF-150, BF-151, BF-154, BF-160, BF-162, BF-165, BF-168, BF-172, BF-180, BF-191, BF-192, BF-196, BF-197, BF-198, BF-200, BF-201, BF-203, BF-206, BF-208, BF-225, BF-227, BF-228, N-227, N-228, N-276, N-282, N-283, and N-407.

3. The compound according to claim 1, wherein the compound is selected from the group consisting of BF-124, BF-148, BF-165, BF-168, BF-191, BF-192, BF-196, BF-197, BF-198, BF-200, BF-201, BF-203, BF-206, BF-208, BF-227, BF-228, N-276, N-277, and N-313.

4. The compound according to any one of claims 1 to 3, wherein the compound is labeled, or a salt or solvate thereof.

5. The compound according to any one of claims 1 to 3, wherein the compound is labeled with a radionuclide, or a salt or solvate thereof.

6. The compound according to any one of claims 1 to 3, wherein the compound is labeled with a γ-ray emitting nuclide, or a salt or solvate thereof.

7. The compound according to any one of claims 1 to 3, wherein the compound is labeled with a γ-ray emitting nuclide selected from the group consisting of ^{99m}Tc, ¹¹¹In, ⁶⁷Ga, ²⁰¹Tl, ¹²³I, and ¹³³Xe, or a salt or solvate thereof.

8. The compound according to any one of claims 1 to 3, wherein the compound is labeled with a γ-ray emitting nuclide selected from the group consisting of ^{99m}Tc and ¹²³I, or a salt or solvate thereof.

9. The compound according to any one of claims 1 to 3, wherein the compound is labeled with a positron emitting nuclide, or a salt or solvate thereof.

10. The compound according to any one of claims 1 to 3, wherein the compound is labeled with a positron emitting nuclide selected from the group consisting of ¹¹C, ¹³N, ¹⁵O, and ¹⁸F, or a salt or solvate thereof.

11. The compound according to any one of claims 1 to 3, wherein the compound is labeled with ¹⁸F, or a salt or solvate thereof.

12. A composition for the diagnosis of diseases in which prion protein is accumulated, comprising a compound according to any one of claims 1 to 11, or a salt or solvate thereof and a pharmaceutically acceptable carrier.

13. A kit for the diagnosis of diseases in which prion protein is accumulated, comprising a compound according to anyone of claims 1 to 11, or a salt or solvate thereof as the essential ingredient.

14. A method for the diagnosis of diseases in which prion protein is accumulated, which comprises employing a compound according to any one of claims 1 to 11, or a salt or solvate thereof.

15. The composition according to claim 12, the kit according to claim 13, or the method according to claim 14, wherein the compound is a compound according to claim 2.

16. A composition for the imaging diagnosis of diseases in which prion protein is accumulated, comprising a compound according to any one of claims 5 to 11, or a pharmaceutically acceptable salt or solvate thereof, and a pharmaceutically acceptable carrier.

17. The composition according to claim 16, comprising a compound according to claim 8, or a pharmaceutically acceptable salt or solvate thereof.

18. The composition according to claim 16, comprising a compound according to claim 11, or a pharmaceutically acceptable salt or solvate thereof.

19. A kit for the imaging diagnosis of diseases in which prion protein is accumulated, comprising a compound according to any one of claims 5 to 11, or a pharmaceutically acceptable salt or solvate thereof as the essential ingredient.

20. The kit according to claim 19, comprising a compound according to claim 8, or a pharmaceutically acceptable salt or solvate thereof as the essential ingredient.

21. The kit according to claim 19, comprising a compound according to claim 11, or a pharmaceutically acceptable salt or solvate thereof as the essential ingredient.

22. A method for the imaging diagnosis of diseases in which prion protein is accumulated, which comprises employing a compound according to any one of claims 5 to 11, or a pharmaceutically acceptable salt or solvate thereof.

23. The composition according to any one of claims 16 to 18, the kit according to any one of claims 19 to 21, or the method according to claim 22, wherein the compound is a compound according to claim 3 labeled with a γ-ray or positron emitting nuclide, and the imaging diagnosis is carried out by PET or SPECT.

24. A composition for staining abnormal prion protein in samples, comprising a compound according to any one of claims 1 to 11, or a salt or solvate thereof.

25. A kit for staining abnormal prion protein in samples, comprising a compound according to any one of claims 1 to 11, or a salt or solvate thereof as the essential ingredient.

26. A method for staining abnormal prion protein in samples, which comprises employing a compound according to any one of claims 1 to 11, or a salt or solvate thereof.

27. The composition according to claim 24, the kit according toclaim25, or the method according to claim 2 6, wherein the compound is a compound according to claim 2.

28. A composition for the in vitro diagnosis of an individual with a disease having accumulated prion protein in the living body, comprising a compound according to any one of claims 1 to 11, or a salt or solvate thereof.

29. A kit for the in vitro diagnosis of an individual with a disease having accumulated prion protein in the living body, comprising a compound according to any one of claims 1 to 11, or a salt or solvate thereof as the essential ingredient.

30. A method for the in vitro diagnosis of an individual with a disease having accumulated prion protein in the living body, which comprises obtaining samples from a subject animal, and contacting to said samples a compound according to any one of claims 1 to 11, or a salt or solvate thereof.

31. The composition according to claim 28, the kit according to claim 29, or the method according to claim 30, wherein the compound is selected from the group consisting of BF-168, BF-191, BF-192, BF-196, BF-197, BF-198, BF-200, BF-201, BF-203, BF-206, BF-208, BF-227, BF-228, and N-278.

32. A pharmaceutical composition for the prophylaxis and/or treatment of a disease in which the accumulation of prion protein in the body constitutes or partially constitutes the etiology, comprising a compound according to claim 1, or a pharmaceutically acceptable salt or solvate thereof and a pharmaceutically acceptable carrier.

33. The pharmaceutical composition according to claim 32, wherein the disease is selected from the group consisting of transmissible spongiform encephalopathy and prion diseases.

34. A method for the treatment of a disease in which the accumulation of prion protein in the body constitutes or partially constitutes the etiology, which comprises administrating a compound according to claim 1, or a pharmaceutically acceptable salt or solvate thereof.

35. The method according to claim 34, wherein the disease is selected from the group consisting of transmissible spongiform encephalopathy and prion diseases.

36. Use of a compound according to claim 1, or a pharmaceutically acceptable salt or solvate thereof for prophylaxis and/or treatment of a disease in which the accumulation of prion protein in the body constitutes or partially constitutes the etiology.

37. The use according to claim 36, wherein the disease is selected from the group consisting of transmissible spongiform encephalopathy and prion diseases.

38. Use of a compound of the present invention for manufacturing a medicament for the prophylaxis and/or treatment of a disease in which the accumulation of prion protein in the body constitutes or partially constitutes the etiology.

39. The use according to claim 38, wherein the disease is selected from the group consisting of transmissible spongiform encephalopathy and prion diseases.

40. The composition according to claim 32 or 33, the kit according to claim 34 or 35, the method according to claim 36 or 37, or the method according to claim 38 or 39, wherein the compound is selected from the group consisting of BF-130, F-135, BF-136, BF-141, BF-146, BF-148, BF-150, BF-153, BF-168, N-220, N-221, N-223, N-224, N-232, N-243, N-246, N-407, N-437, N-441, N-453, N-457, BF-192, BF-193, BF-198, BF-199, BF-201, BF-203, BF-204, BF-206, BF-208, BF-211, BF-213, BF-227, and BF-231.

41. The composition according to claim 32 or 33, the kit according to claim 34 or 35, the method according to claim 36 or 37, or the method according to claim 38 or 39, wherein the compound is selected from the group consisting of BF-130, BF-135, BF-146, N-407, N-437, N-441, N-453, N-457, BF-208, BF-227, BF-231, BF-192, BF-193, BF-198, BF-199, BF-201, BF-203, BF-204, BF-206, BF-208, BF-211, BF-213, N-220, N-221, N-223, and N-224.

42. A labeled precursor of a compound according to claim 1.

43. A labeled precursor of BF-168, BF-224, or N-227, wherein the precursor is a tosylate derivative.
